# EUROPEAN PATENT APPLICATION

(11) **EP 2 311 491 A1**
(43) Date of publication of application: **20.04.2011**
(21) Application number: 10185318.2
(22) Date of filing: 02.03.2006
(51) Int. Cl.: A61K 39/395, A61K 47/18, A61P 35/00, A61P 37/00, C07K 16/24

(54) **Composition comprising an antibody against macrophage colony-stimulating factor (M-CSF) and a chelating agent**

(30) Priority: 08.03.2005 US 659766 P; 19.10.2005 US 728165 P; 20.12.2005 US 752712 P; 26.01.2006 US 762456 P
(62) Divisional of application: 06748276.0
(71) Applicant: Pharmacia & Upjohn Company LLC, Kalamazoo, MI 49001-0199 (US)
(72) Inventor: Carroll, James, Alan, Chesterfield, MI 63017 (US); Das, Tapan Kanti, Chesterfield, MI 63017 (US); Allan, Corey M., Chesterfield, MI 63017 (US); Nema, Sandeep, Chesterfield, MI 63017 (US); Zeng, David, Li, Chesterfield, MI 63017 (US)
(74) Representative: Courgeon, Antoine

(57) **Abstract**

The present invention provides for compositions of anti-M-CSF antibodies comprising a chelating agent and/or histidine. Also provided are methods of treating M-CSF-mediated disorders with pharmaceutical formulations of anti-M-CSF antibodies, including inflammatory diseases and neoplasia disorders.

SEQUENCE LISTING

<110>  Pharmacia and Upjohn Company, LLC
       Das, Tapan
       Carroll, James A
       Allan, Corey
       Nema, Sandeep
       Zeng, David

<120>  ANTI-M-CSF ANTIBODY COMPOSITIONS

<130>  PC 32802

<150>  60/659,766
<151>  2005-03-08

<150>  60/728,165
<151>  2005-10-19

<150>  60/752,712
<151>  2005-12-20

<150>  60/762,456
<151>  2005-01-26

<160>  4

<170>  PatentIn version 3.3

<210>  1
<211>  1398
<212>  DNA
<213>  Homo sapiens

<400>  1
atggagttgg ggctgtgctg ggttttcctt gttgctattt tagaaggtgt ccagtgtgag      60

gtgcagctgg tggagtctgg gggaggcttg gtacagcctg gggggtccct gagactctcc     120

tgtgcagcct ctggattcac cttcagtagt tttagtatga cctgggtccg ccaggctcca     180

ggaaaggggc tggagtgggt ttcatacatt agtagtagaa gtagtaccat atcctacgca     240

gactctgtga agggccgatt caccatctcc agagacaatg ccaagaactc actgtatctg     300

caaatgaaca gcctgagaga cgaggacacg gctgtgtatt actgtgcgag agatcctctt     360

ctagcgggag ctaccttctt tgactactgg ggccagggaa ccctggtcac cgtctcctca     420

gcctccacca agggcccatc ggtcttcccc ctggcgccct gctccaggag cacctccgag     480

agcacagcgg ccctgggctg cctggtcaag gactacttcc ccgaaccggt gacggtgtcg     540

tggaactcag gcgctctgac cagcggcgtg cacaccttcc cagctgtcct acagtcctca     600

ggactctact ccctcagcag cgtggtgacc gtgccctcca gcaacttcgg cacccagacc     660

tacacctgca acgtagatca caagcccagc aacaccaagg tggacaagac agttgagcgc     720

aaatgttgtg tcgagtgccc accgtgccca gcaccacctg tggcaggacc gtcagtcttc     780

ctcttccccc caaaacccaa ggacaccctc atgatctccc ggacccctga ggtcacgtgc     840

gtggtggtgg acgtgagcca cgaagacccc gaggtccagt tcaactggta cgtggacggc 900
gtggaggtgc ataatgccaa gacaaagcca cgggaggagc agttcaacag cacgttccgt 960
gtggtcagcg tcctcaccgt tgtgcaccag gactggctga acggcaagga gtacaagtgc 1020
aaggtctcca acaaaggcct cccagccccc atcgagaaaa ccatctccaa aaccaaaggg 1080
cagccccgag aaccacaggt gtacaccctg cccccatccc gggaggagat gaccaagaac 1140
caggtcagcc tgacctgcct ggtcaaaggc ttctacccca gcgacatcgc cgtggagtgg 1200
gagagcaatg ggcagccgga gaacaactac aagaccacac ctcccatgct ggactccgac 1260
ggctccttct tcctctacag caagctcacc gtggacaaga gcaggtggca gcaggggaac 1320
gtcttctcat gctccgtgat gcatgaggct ctgcacaacc actacacgca gaagagcctc 1380
tccctgtctc cgggtaaa 1398

<210> 2
<211> 466
<212> PRT
<213> Homo sapiens

<400> 2

Met Glu Leu Gly Leu Cys Trp Val Phe Leu Val Ala Ile Leu Glu Gly
1               5                   10                  15

Val Gln Cys Glu Val Gln Leu Val Glu Ser Gly Gly Gly Leu Val Gln
            20                  25                  30

Pro Gly Gly Ser Leu Arg Leu Ser Cys Ala Ala Ser Gly Phe Thr Phe
        35                  40                  45

Ser Ser Phe Ser Met Thr Trp Val Arg Gln Ala Pro Gly Lys Gly Leu
    50                  55                  60

Glu Trp Val Ser Tyr Ile Ser Ser Arg Ser Ser Thr Ile Ser Tyr Ala
65                  70                  75                  80

Asp Ser Val Lys Gly Arg Phe Thr Ile Ser Arg Asp Asn Ala Lys Asn
                85                  90                  95

Ser Leu Tyr Leu Gln Met Asn Ser Leu Arg Asp Glu Asp Thr Ala Val
            100                 105                 110

Tyr Tyr Cys Ala Arg Asp Pro Leu Leu Ala Gly Ala Thr Phe Phe Asp
        115                 120                 125

Tyr Trp Gly Gln Gly Thr Leu Val Thr Val Ser Ser Ala Ser Thr Lys
    130                 135                 140

Gly Pro Ser Val Phe Pro Leu Ala Pro Cys Ser Arg Ser Thr Ser Glu
145                 150                 155                 160

Ser Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Asn Phe Gly Thr Gln Thr Tyr Thr Cys Asn
    210                 215                 220

Val Asp His Lys Pro Ser Asn Thr Lys Val Asp Lys Thr Val Glu Arg
225                 230                 235                 240

Lys Cys Cys Val Glu Cys Pro Pro Cys Pro Ala Pro Pro Val Ala Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Phe Arg
305                 310                 315                 320

Val Val Ser Val Leu Thr Val Val His Gln Asp Trp Leu Asn Gly Lys
                325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210> 3
<211> 705
<212> DNA
<213> Homo sapiens

<400> 3
atggaaaccc cagcgcagct tctcttcctc ctgctactct ggctcccaga taccaccgga 60

gaaattgtgt tgacgcagtc tccaggcacc ctgtctttgt ctccagggga aagagccacc 120

ctctcctgca gggccagtca gagtgttagc agcagttact tagcctggta ccagcagaaa 180

cctggccagg ctcccaggct cctcatctat ggtgcatcca gcagggccac tggcatccca 240

gacaggttca gtggcagtgg gtctgggaca gacttcactc tcaccatcag cagactggag 300

cctgaagatt ttgcagtgta ttactgtcag cagtatggta gctcacctct cactttcggc 360

ggagggacca aggtggagat caaacgaact gtggctgcac catctgtctt catcttcccg 420

ccatctgatg agcagttgaa atctggaact gcctctgttg tgtgcctgct gaataacttc 480

tatcccagag aggccaaagt acagtggaag gtggataacg ccctccaatc gggtaactcc 540

caggagagtg tcacagagca ggacagcaag gacagcacct acagcctcag cagcaccctg 600

acgctgagca aagcagacta cgagaaacac aaagtctacg cctgcgaagt cacccatcag 660

ggcctgagct cgcccgtcac aaagagcttc aacaggggag agtgt 705

<210> 4
<211> 235
<212> PRT
<213> Homo sapiens

<400> 4

Met Glu Thr Pro Ala Gln Leu Leu Phe Leu Leu Leu Leu Trp Leu Pro
1               5                   10                  15

Asp Thr Thr Gly Glu Phe Val Leu Thr Gln Ser Pro Gly Thr Leu Ser
            20                  25                  30

Leu Ser Pro Gly Glu Arg Ala Thr Leu Ser Cys Arg Ala Ser Gln Ser
        35                  40                  45

Val Ser Ser Ser Tyr Leu Ala Trp Tyr Gln Gln Lys Pro Gly Gln Ala
    50                  55                  60

Pro Arg Leu Leu Ile Tyr Gly Ala Ser Ser Arg Ala Thr Gly Ile Pro
65                  70                  75                  80

Asp Arg Phe Ser Gly Ser Gly Ser Gly Thr Asp Phe Thr Leu Thr Ile
                85                  90                  95

Ser Arg Leu Glu Pro Glu Asp Phe Ala Val Tyr Tyr Cys Gln Gln Tyr
            100                 105                 110

Gly Ser Ser Pro Leu Thr Phe Gly Gly Gly Thr Lys Val Glu Ile Lys
        115                 120                 125

Arg Thr Val Ala Ala Pro Ser Val Phe Ile Phe Pro Pro Ser Asp Glu
    130                 135                 140

Gln Leu Lys Ser Gly Thr Ala Ser Val Val Cys Leu Leu Asn Asn Phe
145                 150                 155                 160

Tyr Pro Arg Glu Ala Lys Val Gln Trp Lys Val Asp Asn Ala Leu Gln
                165                 170                 175

Ser Gly Asn Ser Gln Glu Ser Val Thr Glu Gln Asp Ser Lys Asp Ser
            180                 185                 190

Thr Tyr Ser Leu Ser Ser Thr Leu Thr Leu Ser Lys Ala Asp Tyr Glu
        195                 200                 205

Lys His Lys Val Tyr Ala Cys Glu Val Thr His Gln Gly Leu Ser Ser
    210                 215                 220

Pro Val Thr Lys Ser Phe Asn Arg Gly Glu Cys
225                 230                 235

## Description

### CROSS-REFERENCE TO RELATED PATENTS AND PATENT APPLICATIONS

This application claims the benefit of U.S. Provisional Patent Application Serial No. 60/659,766 filed March 8, 2005; U.S. Provisional Patent Application Serial No. 60/728,165 filed October 19, 2005; U.S. Provisional Patent Application Serial No. 60/752,712 filed December 20, 2005; and U.S. Provisional Patent Application Serial No. 60/762,456 filed January 26, 2006, all of which are incorporated by reference herein in their entireties.

### BACKGROUND OF THE INVENTION

Macrophage colony stimulating factor (M-CSF) is a secreted or a cell surface glycoprotein comprised of two subunits that are joined by a disulfide bond with a total molecular mass varying from 40 to 90 kDa ((Stanley, et al, Mol. Reprod. Dev., 46:4-10 (1997)). Similar to other colony stimulation factors, M-CSF is produced by macrophages, monocytes, and human joint tissue cells, such as chondrocytes and synovial fibroblasts, in response to proteins such as interleukin-1 or tumor necrosis factor-alpha. M-CSF stimulates the formation of macrophage colonies from pluripotent hematopoietic progenitor stem cells (Stanley, et al., Mol. Reprod. Dev., 46:4-10 (1997)).

M-CSF is an important regulator of the function, activation, and survival of monocytes/macrophages. A number of animal models have confirmed the role of M-CSF in various diseases, including rheumatoid arthritis and cancer. Macrophages comprise key effector cells in rheumatoid arthritis. The degree of synovial macrophage infiltration in rheumatoid arthritis has been shown to closely correlate with the extent of underlying joint destruction. M-CSF, endogenously produced in the rheumatoid joint by monocytes/macrophages, fibroblasts, and endothelial cells, acts on cells of the monocyte/macrophage lineage to promote their survival and differentiation into bone destroying osteoclasts, and enhance pro-inflammatory cellular functions such as cytotoxicity, superoxide production, phagocytosis, chemotaxis and secondary cytokine production. Antibodies to M-CSF have been used to treat animal models of rheumatoid arthritis (Cambell, et al., J. Leukocyte Biol. 68:144-150 (2000)). For example, U.S. Published Application No. 20050059113 to Bedian, et al. describes anti-M-CSF antibodies. Thus, there is a need in the art for formulations of M-CSF antibodies that can be used to treat diseases, and in particular such diseases as rheumatoid arthritis, cancer, and other M-CSF-mediated diseases.

### SUMMARY

In one aspect, the present invention provides a composition comprising at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF; and further comprising a chelating agent.

The present invention also provides a liquid pharmaceutical composition comprising at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF; and further comprising a chelating agent.

The present invention also provides a composition comprising at least one antibody wherein the antibody comprises a heavy chain amino acid sequence comprising SEQ ID NO: 2 and a light chain amino acid sequence comprising SEQ ID NO: 4, wherein the antibody binds to human M-CSF; and a chelating agent.

The present invention also provides a composition comprising at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the composition contains antibody in amount of at least about 10 mg/ml and the antibody binds to human M-CSF; and a chelating agent.

The present invention also provides a composition comprising at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the composition contains antibody in amount of equal to or less than about 15 mg/ml and the antibody binds to human M-CSF; and a chelating agent.

The present invention also provides a composition comprising at least one antibody wherein the antibody comprises a heavy chain amino acid sequence comprising SEQ ID NO: 2 and a light chain amino acid sequence comprising SEQ ID NO: 4, wherein the antibody binds to human M-CSF and the C-terminal lysine of the heavy chain of the antibody is not present; and a chelating agent.

The present invention also provides a composition comprising at least one antibody wherein the antibody comprises a heavy chain amino acid sequence comprising SEQ ID NO: 2 and a light chain amino acid sequence comprising SEQ ID NO: 4, wherein the antibody binds to human M-CSF; and a chelating agent, wherein the composition further comprises at least one or two excipients selected from the group consisting of tonicity agents, surfactants and buffers.

The present invention also provides a composition comprising at least one antibody wherein the antibody comprises a heavy chain amino acid sequence comprising SEQ ID NO: 2 and a light chain amino acid sequence comprising SEQ ID NO: 4, wherein the antibody binds to human M-CSF; and a pharmaceutically acceptable chelating agent, wherein the composition further comprises a tonicity agent, a surfactant and a buffer.

The present invention also provides a composition comprising at least one antibody wherein the antibody comprises a heavy chain amino acid sequence comprising SEQ ID NO: 2 and a light chain amino acid sequence comprising SEQ ID NO: 4, wherein the antibody binds to human M-CSF; and a chelating agent, wherein the composition further comprises a tonicity agent selected from the group of mannitol, trehalose and sucrose.

The present invention also provides a composition comprising at least one antibody wherein the antibody comprises a heavy chain amino acid sequence comprising SEQ ID NO: 2 and a light chain amino acid sequence comprising SEQ ID NO: 4, wherein the antibody binds to human M-CSF; and EDTA.

The present invention also provides a composition comprising at least one antibody wherein the antibody comprises a heavy chain amino acid sequence comprising SEQ ID NO: 2 and a light chain amino acid sequence comprising SEQ ID NO: 4, wherein the antibody binds to human M-CSF and the concentration of the antibody is from about 0.1 mg/ml to about 100 mg/ml; and EDTA.

The present invention also provides a composition comprising at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF; and further comprising a chelating agent, wherein the composition is administered to the subject parenterally.

The present invention also provides a composition comprising at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF; and further comprising a chelating agent, wherein the composition is administered to the subject intravenously.

The present invention also provides a composition comprising at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF; and further comprising a chelating agent, wherein the composition comprises from about 0.1 mg/ml to about 200 mg/ml of antibody; from about 1 mM to about 100 mM of histidine; from about from about 0.01 mg/ml to about 10 mg/ml of polysorbate 80; from about 1 micromolar to about 1.0 millimolar of EDTA; and from about 10 millimolar to about 600 millimolar of a tonicity agent.

The present invention also provides a composition comprising at least one antibody wherein the antibody comprises a heavy chain amino acid sequence comprising SEQ ID NO: 2 and a light chain amino acid sequence comprising SEQ ID NO: 4, wherein the antibody binds to human M-CSF; and a chelating agent, and the antibody is stable at a temperature of about 5°C for at least about 26 weeks.

The present invention also provides a composition comprising at least one antibody wherein the antibody comprises a heavy chain amino acid sequence comprising SEQ ID NO: 2 and a light chain amino acid sequence comprising SEQ ID NO: 4, wherein the antibody binds to human M-CSF; and a chelating agent, and the antibody is stable at a temperature of about 25°C for at least about 26 weeks.

The present invention also provides a composition comprising at least one antibody wherein the antibody comprises a heavy chain amino acid sequence comprising SEQ ID NO: 2 and a light chain amino acid sequence comprising SEQ ID NO: 4, wherein the antibody binds to human M-CSF; and a chelating agent, and the antibody is stable at a temperature of about 40°C for at least about 26 weeks.

The present invention also provides a stable composition comprising at least one monoclonal anti-M-CSF antibody and a stabilizing amount of a chelating agent, wherein after the composition is stored for a period of about 26 weeks at a temperature of about 40°C, one or both of the following conditions are satisfied: the decrease between a fragment chromatogram peak area for the stable composition comprising at least one monoclonal anti-M-CSF antibody and the chelating agent, and a fragment chromatogram peak area for an isotonic composition lacking the chelating agent, is at least about 1%; or the decrease between a fragment chromatogram peak area for the stable composition comprising at least one monoclonal anti-M-CSF antibody and the chelating agent, and a fragment chromatogram peak area for an isotonic composition lacking the chelating agent, is at least about 0.5%.

The present invention also provides a method for stabilizing at least one monoclonal anti-M-CSF antibody comprising the method of forming a composition comprising the antibodies and a stabilizing amount of a chelating agent, wherein after the composition is stored for a period of about 26 weeks at a temperature of about 40°C, one or both of the following conditions are satisfied: the decrease between an aggregate chromatogram peak area for the stable composition comprising monoclonal anti-M-CSF antibodies and the chelating agent, and an aggregate chromatogram peak area for an isotonic composition lacking the chelating agent, is at least about 1 %; or the decrease between an aggregate chromatogram peak area for the stable composition comprising monoclonal anti-M-CSF antibodies and the chelating agent, and an aggregate chromatogram peak area for an isotonic composition lacking the chelating agent, is at least about 0.5%.

The present invention also provides methods for stabilizing at least one monoclonal anti-M-CSF antibody in a composition comprising the method: forming a composition comprising the antibody and at least one stabilizing chelating agent compound, wherein the composition comprises a sufficient amount of the stabilizing chelating agent to stabilize the composition during storage for a period of about 26 weeks at a temperature of about 40°C; and wherein at the end of the storage period at least one of the following conditions are satisfied: the amount of aggregated antibodies is less than or equal to about 3.5% by peak area of a chromatogram of the antibodies after chromatographic separation; or the amount of antibody fragment formed by antibody hydrolysis having a molecular weight ranging from about 10.5 kD to about 11.5 kD is less than or equal to about 1.7% by peak area of a chromatogram of the antibodies after chromatographic separation.

The present invention also provides a method for analyzing the stability of at least one anti-M-CSF antibody comprising identifying an antibody fragment formed by antibody hydrolysis having a molecular weight ranging from about 10.5 kD to about 11.5 kD polypeptide fragment in a composition comprising the anti-M-CSF antibody by separating species by organic SE-HPLC, and identifying the presence of the polypeptide fragment in the composition by using ultraviolet detection at 214 nanometers.

The present invention also provides a method for detecting the presence of a polypeptide fragment having a molecular weight of between about 10 kD and about 12 kD in a composition comprising at least one anti-M-CSF antibody antibody, the method comprising separating species by organic SE-HPLC, and identifying the presence of the polypeptide fragment in the composition by using ultraviolet detection at 214 nanometers or at other suitable wavelengths, *e.g.,* 280 nm.

The present invention also provides a method for stabilizing at least one anti-M-CSF antibody by combining the antibody in a liquid composition with a chelating agent in an amount, which reduces chemical or physical instability of the antibody.

The invention also provides a liquid pharmaceutical composition comprising at least one anti-M-CSF antibody and a chelating agent, wherein the molar concentration of the antibody ranges from about 0.01 millimolar to about 2 millimolar and the molar concentration of the chelating agent ranges from about 0.001 millimolar to about 5 millimolar, and wherein the molar ratio of antibody to chelating agent ranges from about 0.002 to about 2000; from about 0.01 to about 500; from about 0.05 to about 100; from about 0.1 to about 50; from about 0.5 to about 10; from about 1 to about 5; from about 1 to about 3; or about 1.6.

The present invention also provides a composition comprising at least one anti-M-CSF antibody and a chelating agent, wherein the molar concentration of the antibody ranges from about 0.01 millimolar to about 2 millimolar and the molar concentration of the chelating agent ranges from about 0.001 millimolar to about 5 millimolar, and wherein the molar ratio of antibody to chelating agent ranges from about 0.002 to about 2000; from about 0.01 to about 500; from about 0.05 to about 100; from about 0.1 to about 50; from about 0.5 to about 10; from about 1 to about 5; or about 3.8.

The present invention also provides methods for the treatment of an M-CSF-mediated disorder in a subject, comprising administering to the subject a therapeutically effective amount of a liquid pharmaceutical composition comprising: at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF; and a chelating agent.

The present invention also provides a method for the treatment of an M-CSF-mediated disorder in a subject, comprising administering to the subject a therapeutically effective amount of a liquid pharmaceutical composition comprising: at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF; and a chelating agent, wherein the subject is in need of the treatment of a M-CSF-mediated disorder.

The present invention also provides methods for the treatment of an M-CSF-mediated disorder in a subject, comprising administering to the subject a therapeutically effective amount of a liquid pharmaceutical composition comprising: at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF; and a chelating agent, wherein the M-CSF-mediated disorder is a neoplasia disorder.

The present invention also provides methods for the treatment of an M-CSF-mediated disorder in a subject, comprising administering to the subject a therapeutically effective amount of a liquid pharmaceutical composition comprising: at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF; and a chelating agent, wherein the M-CSF-mediated disorder is osteoarthritis.

The present invention also provides methods for the treatment of an M-CSF-mediated disorder in a subject, comprising administering to the subject a therapeutically effective amount of a liquid pharmaceutical composition comprising: at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF; and a chelating agent, wherein the M-CSF-mediated disorder is rheumatoid arthritis.

The present invention provides a liquid pharmaceutical composition comprising at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF and the composition contains a concentration of antibody that is at least about 10 mg/ml; and further comprising a chelating agent.

The present invention provides compositions comprising at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF and the composition contains a concentration of antibody that ranges from about 50 mg/ml to about 100 mg/ml; and further comprising a chelating agent.

The present invention also provides a process for preparing a composition comprising mixing at least one anti-M-CSF antibody having the heavy and light chain amino acid sequences of antibody 8.10.3F in solution, with a chelating agent.

The present invention also provides methods for preparing a stable composition comprising mixing at least one monoclonal anti-M-CSF antibody 8.10.3F with at least one chelating agents, in an amount, which reduces chemical or physical instability of the antibody.

The present invention also provides an article of manufacture comprising a container which holds a mixture of at least one anti-M-CSF antibody having the heavy and light chain amino acid sequences of antibody 8.10.3F, and a chelating agent.

The present invention also provides kits for preparing compositions of at least one anti-M-CSF antibody comprising: a first container comprising at least one anti-M-CSF antibody having the heavy and light chain amino acid sequences of antibody 8.10.3F, and a second container comprising a chelating agent.

The present invention also provides kits for preparing a liquid pharmaceutical composition of at least one anti-M-CSF antibody comprising: a first container comprising at least one anti-M-CSF antibody having the heavy and light chain amino acid sequences of antibody 8.10.3F in solution, and a second container comprising a pharmaceutically acceptable chelating agent.

### BRIEF DESCRIPTION OF THE DRAWINGS

Figure 1 shows a line graph that shows the percent fragmentation in SDS-PAGE reduced gels with anti-M-CSF antibody 8.10.3F compositions stored for six weeks at 40°C;
Figure 2 shows a line graph that shows the percent major IEF band in IEF gels with anti-M-CSF antibody 8.10.3F compositions stored for six weeks at 40°C;
Figure 3 shows a line graph that shows the percent aggregation determined from SE-HPLC data with anti-M-CSF antibody 8.10.3F compositions stored for six weeks at 40°C;
Figure 4 shows a line graph that shows the percent aggregation determined from SE-HPLC data with anti-M-CSF antibody 8.10.3F compositions stored for 26 weeks at 40°C;
Figure 5 shows a line graph that shows the percent fragmentation (approximately 11kD) determined from organic SE-HPLC data with anti-M-CSF antibody 8.10.3F compositions stored for up to 26 weeks at 40°C;
Figure 6 shows a line graph that shows the percent fragmentation from SDS-PAGE reduced gels with anti-M-CSF antibody 8.10.3F compositions stored for 26 weeks at 40°C;
Figure 7 shows a line graph that shows the percentage of remaining antibody monomer from SDS-PAGE non-reduced gels with anti-M-CSF antibody 8.10.3F compositions stored for 26 weeks at 40°C;
Figure 8 shows an organic SE-HPLC chromatogram (shown expanded in y-axis) of anti-M-CSF antibody 8.10.3F compositions 11 and 9 stored for 26 weeks at 40°C;
Figure 9 shows a photograph of a reduced SDS-PAGE gel of anti-M-CSF antibody 8.10.3F compositions stored for six weeks at 40°C;
Figure 10 shows an organic SE-HPLC chromatogram of anti-M-CSF antibody 8.10.3F in formulation 1 (top) stored at 40°C for 6 weeks compared to a control sample (bottom).
Figure 11 shows an SE-HPLC chromatogram for anti-M-CSF antibody 8.10.3F stored in formulation 11 for 26 weeks at 40°C.

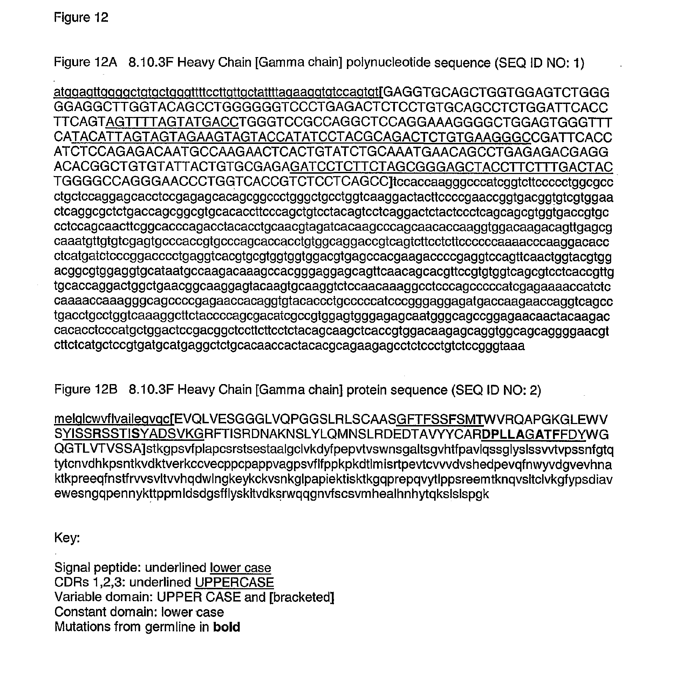

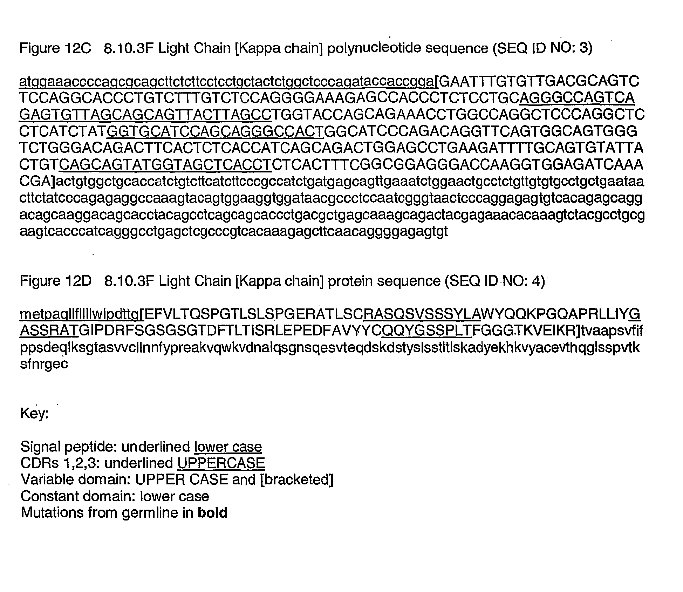

Figure 12, comprising Figures 12A-12D, shows the nucleic acid and amino acid sequence for anti-M-CSF antibody 8.11.3F. Figure 12A shows the full-length nucleic acid sequence for the 8.11.3F heavy chain (SEQ ID NO: 1). Figure 12B shows the full-length amino acid sequence for the 8.11.3F heavy chain (SEQ ID NO: 2), and the amino acid sequence for the 8.11.3F heavy chain amino acid variable region is in upper case and designated between brackets "[ ]" (SEQ ID NO: 5). The amino acid sequence of each 8.11.3F heavy chain CDR is underlined and in lowercase. The heavy chain CDR amino acid sequences are as follows: CDR1: GFTFSSFSMT (SEQ ID NO: 7); CDR2: YISSRSSTISYADSVKG (SEQ ID NO: 8); and CDR3: DPLLAGATFFDY (SEQ ID NO: 9). Figure 12C shows the nucleic acid sequence for the full-length 8.11.3F light chain (SEQ ID NO: 3). Figure 12D shows the amino acid sequence of the full-length 8.11.3F light chain (SEQ ID NO: 4), and the 8.11.3F light chain amino acid variable region is in upper case and designated between brackets "[ ]" (SEQ ID NO: 6). The amino acid sequence of each light chain CDR is indicated as follows: CDR1: RASQSVSSSYLA (SEQ ID NO: 10); CDR2: GASSRAT (SEQ ID NO: 11); and CDR3: QQYGSSPLT (SEQ ID NO: 12).

### DETAILED DESCRIPTION OF THE INVENTION

The methods and techniques of the present invention are generally performed according to conventional methods well known in the art and as described in various general and more specific references that are cited and discussed throughout the present specification unless otherwise indicated. See, *e.g.,* Sambrook et al., Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990). Enzymatic reactions and purification techniques are performed according to manufacturer's specifications, as commonly accomplished in the art or as described herein. The nomenclatures used in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of subjects.

### Definitions:

In order to aid the reader in understanding the following detailed description, the following definitions are provided:

As used herein, the term "antibody" refers to an intact antibody or an antigen-binding portion that competes with the intact antibody for specific binding. See generally, Fundamental Immunology, Ch. 7 (Paul, W., ed., 2nd ed. Raven Press, N.Y. (1989). Antigen-binding portions may be produced by recombinant DNA techniques or by enzymatic or chemical cleavage of intact antibodies. In some embodiments, antigen-binding portions include Fab, Fab', F(ab')₂, Fd, Fv, dAb, and complementarity determining region (CDR) fragments, single-chain antibodies (scFv), chimeric antibodies, diabodies and polypeptides that contain at least a portion of an antibody that is sufficient to confer specific antigen binding to the polypeptide. From N-terminus to C-terminus, both the mature light and heavy chain variable domains comprise the regions FR1, CDR1, FR2, CDR2, FR3, CDR3 and FR4. The assignment of amino acids to each domain is in accordance with the definitions of Kabat, Sequences of Proteins of Immunological Interest (National Institutes of Health, Bethesda, Md. (1987 and 1991)), Chothia & Lesk, J. Mol. Biol. 196:901-917 (1987), or Chothia et al., Nature 342:878-883 (1989).

In some embodiments, the antibody is a single-chain antibody (scFv) in which a V_{L} and V_{H} domains are paired to form a monovalent molecules via a synthetic linker that enables them to be made as a single protein chain. (Bird et al., Science 242:423-426 (1988) and Huston et al., Proc. Natl. Acad. Sci. USA 85:5879-5883 (1988).) In some embodiments, the antibodies are diabodies, *i.e.,* are bivalent antibodies in which V_{H} and V_{L} domains are expressed on a single polypeptide chain, but using a linker that is too short to allow for pairing between the two domains on the same chain, thereby forcing the domains to pair with complementary domains of another chain and creating two antigen binding sites. (See *e.g.,* Holliger P. et al., Proc. Natl. Acad. Sci. USA 90:6444-6448 (1993), and Poljak R. J. et al., Structure 2:1121-1123 (1994)). In some embodiments, one or more CDRs from an antibody of the invention may be incorporated into a molecule either covalently or noncovalently to make it an immunoadhesin that specifically binds to M-CSF. In such embodiments, the CDR(s) may be incorporated as part of a larger polypeptide chain, may be covalently linked to another polypeptide chain, or may be incorporated noncovalently.

As used herein, an antibody that is referred to by number is the same as a monoclonal antibody that is obtained from the hybridoma of the same number. For example, monoclonal antibody 8.10.3F is the same antibody as one obtained from hybridoma 8.10.3F. For example, monoclonal antibody 8.10.3F has the same heavy and light chain amino acid sequences as one obtained from hybridoma 8.10.3F. Thus, reference to antibody 8.10.3F includes an antibody, which has the heavy and light chain amino acid sequences shown in SEQ ID NOS. 30 and 32, respectively. It also includes an antibody lacking a terminal lysine on the heavy chain, as this is normally lost in a proportion of antibodies during manufacture.

As used herein, an Fd fragment means an antibody fragment that consists of the V_{H} and C_{H}1 domains; an Fv fragment consists of the V_{L} and V_{H} domains of a single arm of an antibody; and a dAb fragment (Ward et al., Nature 341:544-546 (1989)) consists of a V_{H} domain.

As used herein, the term "polypeptide" encompasses native or artificial proteins, protein fragments and polypeptide analogs of a protein sequence. A polypeptide may be monomeric or polymeric.

The terms "or an antigen-binding portion thereof" when used with the term "antibody" refers to a polypeptide that has an amino-terminal and/or carboxy-terminal deletion, but where the remaining amino acid sequence is identical to the corresponding positions in the naturally-occurring sequence. In some embodiments, the antigen-binding portion thereof may be at least 14, at least 20, at least 50, or at least 70, 80, 90, 100, 150 or at least 200 amino acids long.

As used herein, the terms "is capable of specifically binding" refers to when an antibody binds to an antigen with a dissociation constant that is ≤ 1 µM, preferably ≤ 1 nM and most preferably ≤ 10 pM. In certain embodiments, the K_{D} is 1 pM to 500 pM. In other embodiments, the K_{D} is between 500 pM to 1 µM. In other embodiments, the K_{D} is between 1 µM to 100 nM. In other embodiments, the K_{D} is between 100 mM to 10 nM.

As used herein, the term "monoclonal antibody" refers to an antibody obtained from a population of substantially homogeneous antibodies, *i.e.,* the individual antibodies comprising the population are identical except for possible naturally occurring mutations that may be present in minor amounts or lacking a C-terminal lysine. Monoclonal antibodies are highly specific, being directed against a single antigenic site. Furthermore, in contrast to conventional (polyclonal) antibody preparations, which typically include different antibodies, directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler, et al., Nature 256:495 (1975), or may be made by recombinant DNA methods (see, *e.g.,* U.S. Pat. No. 4,816,567). The "monoclonal antibodies" may also be isolated from phage antibody libraries using the techniques described in Clackson, et al., Nature 352:624-628 (1991) and Marks, et al., J. Mol. Biol. 222:581-597 (1991), for example.

The term "isolated protein", "isolated polypeptide" or "isolated antibodies" is a protein, polypeptide or antibody that by virtue of its origin or source of derivation has one to four of the following: (1) is not associated with naturally associated components that accompany it in its native state, (2) is free of other proteins from the same species, (3) is expressed by a cell from a different species, or (4) does not occur in nature. Thus, a polypeptide that is chemically synthesized or synthesized in a cellular system different from the cell from which it naturally originates will be "isolated" from its naturally associated components. An isolated protein/antibody may also be rendered substantially free of naturally associated cellular components by isolation, using protein purification techniques well known in the art.

Examples of isolated/purified antibodies include an anti-M-CSF antibody that has been affinity purified using M-CSF, an anti-M-CSF antibody that has been synthesized by a hybridoma or other cell line *in vitro,* and a human anti-M-CSF antibody derived from a transgenic mouse. Thus, in preferred embodiments, the anti-M-CSF antibodies have a purity of at least about 95% (w/w - weight anti-M-CSF antibodies/weight of components other than pharmaceutically acceptable excipients), and in further embodiments, the anti-M-CSF antibodies have a purity from about 95% w/w to about 99.5% w/w.

An antibody is "substantially pure," "substantially homogeneous," or "substantially purified" when at least about 60 to 75% of a sample exhibits a single species of antibody. The antibody may be monomeric or multimeric. A substantially pure antibody can typically comprise about 50%, 60%, 70%, 80% or 90% w/w of an antibody sample, more usually about 95%, and preferably will be over 99% pure. Antibody purity or homogeneity may be indicated by a number of means well known in the art, such as polyacrylamide gel electrophoresis of an antibody sample, followed by visualizing a single polypeptide band upon staining the gel with a stain well known in the art. For certain purposes, higher resolution may be achieved by using HPLC or other means well known in the art for purification.

As used herein, the term "human antibody" is intended to include antibodies having variable and constant regions derived from human germline immunoglobulin sequences. The human antibodies of the invention may include amino acid residues not encoded by human germline immunoglobulin sequences (*e.g.,* mutations introduced by random or site-specific mutagenesis in vitro or by somatic mutation *in vivo*), for example in the CDRs and in particular CDR3. However, the term "human antibody", as used herein, is not intended to include antibodies in which CDR sequences derived from the germline of another mammalian species, such as a mouse, have been grafted onto human framework sequences.

As used herein, the term "recombinant human antibody" is intended to include all human antibodies that are prepared, expressed, created or isolated by recombinant means, such as antibodies expressed using a recombinant expression vector transfected into a host cell, antibodies isolated from a recombinant, combinatorial human antibody library, antibodies isolated from an animal (*e.g.,* a mouse) that is transgenic for human immunoglobulin genes (see *e.g.,* Taylor, L. D., et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

The term "epitope" includes any protein determinant capable of specific binding to an immunoglobulin or T-cell receptor or otherwise interacting with a molecule. Epitopic determinants generally consist of chemically active surface groupings of molecules such as amino acids or sugar side chains and generally have specific three-dimensional structural characteristics, as well as specific charge characteristics. An epitope may be "linear" or "conformational." In a linear epitope, all of the points of interaction between the protein and the interacting molecule (such as an antibody) occur linearly along the primary amino acid sequence of the protein. In a conformational epitope, the points of interaction occur across amino acid residues on the protein that are separated from one another.

As used herein, the term "polynucleotide" or "nucleic acid", used interchangeably herein, means a polymeric form of nucleotides of at least 10 bases in length, either ribonucleotides or deoxynucleotides or a modified form of either type of nucleotide. The term includes single and double stranded forms. A "polynucleotide" or a "nucleic acid" sequence encompasses its complement unless otherwise specified. Thus, a reference to a nucleic acid having a particular sequence should be understood to encompass its complementary strand, with its complementary sequence.

As used herein, the term "isolated polynucleotide" or "isolated nucleic acid" means a polynucleotide of genomic, cDNA, or synthetic origin or some combination thereof, which by virtue of its origin or source of derivation, the isolated polynucleotide has one to three of the following: (1) is not associated with all or a portion of a polynucleotide with which the "isolated polynucleotide" is found in nature, (2) is operably linked to a polynucleotide to which it is not linked in nature, or (3) does not occur in nature as part of a larger sequence.

The term "oligonucleotide" as used herein includes naturally occurring, and modified nucleotides linked together by naturally occurring and non-naturally occurring oligonucleotide linkages. Oligonucleotides are a polynucleotide subset generally comprising a length of 200 bases or fewer. Preferably oligonucleotides are 10 to 60 bases in length and most preferably 12, 13, 14, 15, 16, 17, 18, 19, or 20 to 40 bases in length. Oligonucleotides are usually single stranded, e.g. for primers and probes; although oligonucleotides may be double stranded, e.g. for use in the construction of a gene mutant. Oligonucleotides of the invention can be either sense or antisense oligonucleotides.

As used herein, the term "naturally occurring nucleotides" includes deoxyribonucleotides and ribonucleotides. The term "modified nucleotides" as used herein includes nucleotides with modified or substituted sugar groups and the like. The term "oligonucleotide linkages" referred to herein includes oligonucleotides linkages such as phosphorothioate, phosphorodithioate, phosphoroselenoate, phosphorodiselenoate, phosphoroanilothioate, phoshoraniladate, phosphoroamidate, and the like. See e.g., LaPlanche et al., Nucl. Acids Res. 14:9081 (1986); Stec et al., J. Am. Chem. Soc. 106:6077 (1984); Stein ef al., Nucl. Acids Res. 16:3209 (1988); Zon et al., Anti-Cancer Drug Design 6:539 (1991); Zon et al., Oligonucleotides and Analogues: A Practical Approach, pp. 87-108 (F. Eckstein, Ed., Oxford University Press, Oxford England (1991)); U.S. Patent No. 5,151,510; Uhlmann and Peyman, Chemical Reviews 90:543 (1990), the disclosures of which are hereby incorporated by reference. An oligonucleotide can include a label for detection, if desired.

As used herein, the terms "selectively hybridize" mean to detectably and specifically bind. Polynucleotides, oligonucleotides and fragments thereof in accordance with the invention selectively hybridize to nucleic acid strands under hybridization and wash conditions that minimize appreciable amounts of detectable binding to nonspecific nucleic acids. "High stringency" or "highly stringent" conditions can be used to achieve selective hybridization conditions as known in the art and discussed herein. One example of "high stringency" or "highly stringent" conditions is the incubation of a polynucleotide with another polynucleotide, wherein one polynucleotide may be affixed to a solid surface such as a membrane, in a hybridization buffer of 6X SSPE or SSC, 50% formamide, 5X Denhardt's reagent, 0.5% SDS, 100 µg/ml denatured, fragmented salmon sperm DNA at a hybridization temperature of 42°C for 12-16 hours, followed by twice washing at 55ºC using a wash buffer of 1X SSC, 0.5% SDS. See also Sambrook *et al, supra,* pp. 9.50-9.55.

As applied to polynucleotides, the terms "substantial identity", "percent identity" or "% identical" mean the percent of residues when a first contiguous sequence is compared and aligned for maximum correspondence to a second contiguous sequence. The length of sequence identity comparison may be over a stretch of at least about nine nucleotides, usually at least about 18 nucleotides, more usually at least about 24 nucleotides, typically at least about 28 nucleotides, more typically at least about 32 nucleotides, and preferably at least about 36, 48 or more nucleotides. The terms "substantial identity", "percent identity" or "% identical"' mean that when a polynucleotide molecule is optimally aligned with appropriate nucleotide insertions or deletions with another polynucleotide molecule (or its complementary strand), there is polynucleotide sequence identity of at least about 85%, preferably at least about 90%, and more preferably at least about 95%, 96%, 97%, 98% or 99% of the nucleotide bases, as measured by any well-known algorithm of sequence identity, such as FASTA, BLAST or Gap. There are a number of different algorithms known in the art that can be used to measure polynucleotide sequence identity. For instance, polynucleotide sequences can be compared using FASTA, Gap or Bestfit, which are programs in Wisconsin Package Version 10.0, Genetics Computer Group (GCG), Madison, Wisconsin. FASTA, which includes, *e.g.,* the programs FASTA2 and FASTA3, provides alignments and percent sequence identity of the regions of the best overlap between the query and search sequences (Pearson, Methods Enzymol. 183:63-98 (1990); Pearson, Methods Mol. Biol. 132:185-219 (2000); Pearson, Methods Enzymol. 266:227-258 (1996); Pearson, J. Mol. Biol. 276:71-84 (1998)). Unless otherwise specified, default parameters for a particular program or algorithm are used. For instance, percent sequence identity between nucleic acid sequences can be determined using FASTA with its default parameters (a word size of 6 and the NOPAM factor for the scoring matrix) or using Gap with its default parameters as provided in GCG Version 6.1, herein incorporated by reference.

As applied to polypeptides, the terms "substantial identity", "percent identity" or "% identical" mean that two peptide sequences, when optimally aligned, such as by the programs GAP or BESTFIT using default gap weights, as supplied with the programs, share at least 70%, 75% or 80% sequence identity, preferably at least 90% or 95% sequence identity, and more preferably at least 97%, 98% or 99% sequence identity. In certain embodiments, residue positions that are not identical differ by conservative amino acid substitutions. A "conservative amino acid substitution" is one in which an amino acid residue is substituted by another amino acid residue having a side chain R group with similar chemical properties (*e.g.,* charge or hydrophobicity). In general, a conservative amino acid substitution will not substantially change the functional properties of a protein. In cases where two or more amino acid sequences differ from each other by conservative substitutions, the percent sequence identity may be adjusted upwards to correct for the conservative nature of the substitution. Means for making this adjustment are well-known to those of skill in the art. See, *e.g.,* Pearson, Methods Mol. Biol. 243:307-31 (1994). Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Conservative amino acids substitution groups are: valine-leucine-isoleucine, phenylalanine-tyrosine, lysine-arginine, alanine-valine, glutamate-aspartate, and asparagine-glutamine. Sequence identity for polypeptides, is typically measured using sequence analysis software. Protein analysis software matches sequences using measures of similarity assigned to various substitutions, deletions and other modifications, including conservative amino acid substitutions. For instance, GCG contains programs such as "Gap" and "Bestfit" which can be used with default parameters, as specified with the programs, to determine sequence homology or sequence identity between closely related polypeptides, such as homologous polypeptides from different species of organisms or between a wild type protein and a mutant thereof. See, *e.g.,* GCG Version 6.1. Polypeptide sequences also can be compared using FASTA using default or recommended parameters, see GCG Version 6.1. (University of Wisconsin WI) FASTA (*e.g.,* FASTA2 and FASTA3) provides alignments and percent sequence identity of the regions of the best overlap between the query and search sequences (Pearson, Methods Enzymol. 183:63-98 (1990); Pearson, Methods Mol. Biol. 132:185-219 (2000)). Another preferred algorithm when comparing a sequence of the invention to a database containing a large number of sequences from different organisms is the computer program BLAST, especially blastp or tblastn, using default parameters, as supplied with the programs. See, *e.g.,* Altschul et al., J. Mol. Biol. 215:403-410 (1990); Altschul et al., Nucleic Acids Res. 25:3389-402 (1997). The length of polypeptide sequences compared for homology will generally be at least about 16 amino acid residues, usually at least about 20 residues, more usually at least about 24 residues, typically at least about 28 residues, and preferably more than about 35 residues. When searching a database containing sequences from a large number of different organisms, it is preferable to compare amino acid sequences.

"Operably linked" sequences include both expression control sequences that are contiguous with the gene of interest and expression control sequences that act in *trans* or at a distance to control the gene of interest. The term "expression control sequence" as used herein means polynucleotide sequences that are necessary to effect the expression and processing of coding sequences to which they are ligated. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (*i.e.,* Kozak consensus sequence); sequences that enhance protein stability; and when desired, sequences that enhance protein secretion. The nature of such control sequences differs depending upon the host organism; in prokaryotes, such control sequences generally include promoter, ribosomal binding site, and transcription termination sequence; in eukaryotes, generally, such control sequences include promoters and transcription termination sequence. The term "control sequences" is intended to include, at a minimum, all components whose presence is essential for expression and processing, and can also include additional components whose presence is advantageous, for example, leader sequences and fusion partner sequences.

As used herein, the term "vector" means a nucleic acid molecule capable of transporting another nucleic acid to which it has been linked. In some embodiments, the vector is a plasmid, *i.e.,* a circular double stranded DNA loop into which additional DNA segments may be ligated. In some embodiments, the vector is a viral vector, wherein additional DNA segments may be ligated into the viral genome. In some embodiments, the vectors are capable of autonomous replication in a host cell into which they are introduced (*e.g.,* bacterial vectors having a bacterial origin of replication and episomal mammalian vectors). In other embodiments, the vectors (*e.g.,* non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors").

As used herein, the terms "recombinant host cell" (or simply "host cell") means a cell into which a recombinant expression vector has been introduced. It should be understood that "recombinant host cell" and "host cell" mean not only the particular subject cell but also the progeny of such a cell. Because certain modifications may occur in succeeding generations due to either mutation or environmental influences, such progeny may not, in fact, be identical to the parent cell, but are still included within the scope of the term "host cell" as used herein.

A "therapeutically effective amount" refers to an amount effective, at dosages and for periods of time necessary, to achieve the desired therapeutic result, which includes treatment or prophylactic prevention of any M-CSF meditated condition, including inflammatory diseases and neoplasia disorders. It is to be noted that dosage values may vary with the severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition. Likewise, a therapeutically effective amount of the antibody or antibody portion may vary according to factors such as the disease state, age, sex, and weight of the individual, the ability of the antibody or antibody portion to elicit a desired response in the individual, and the desired route of administration of the antibody formulation. A therapeutically effective amount is also one in which any toxic or detrimental effects of the antibody or antibody portion are outweighed by the therapeutically beneficial effects.

As used herein, the term "subject" for purposes of treatment includes any subject, and preferably is a subject who is in need of the treatment of an M-CSF-mediated disorder. For purposes of prevention, the subject is any subject, and preferably is a subject that is at risk for, or is predisposed to, developing an M-CSF-mediated disorder. The term "subject" is intended to include living organisms, *e.g.,* prokaryotes and eukaryotes. Examples of subjects include mammals, *e.g.,* humans, dogs, cows, horses, pigs, sheep, goats, cats, mice, rabbits, rats, and transgenic non-human animals. In specific embodiments of the invention, the subject is a human.

As used herein, the term "M-CSF-mediated disorder" is intended to include diseases and other disorders in which the presence of M-CSF in a subject suffering from the disorder is elevated in comparison to a normal healthy subject, whether the elevated M-CSF levels are now known or later evidenced or suspected of being either responsible for the pathophysiology of the disorder or a factor that contributes to a worsening of the disorder. Such disorders may be evidenced, for example, by an increase in the levels of M-CSF secreted and/or on the cell surface or increased tyrosine autophosphorylation of *c-fms* in the affected cells or tissues of a subject suffering from the disorder. The increase in M-CSF levels may be detected, for example, using an anti-M-CSF antibody as would be understood by one of skill in the art. Examples of M-CSF-mediated disorders that are encompassed by the present invention include inflammatory diseases, cardiovascular disorders, and neoplasia disorders.

As used herein, the terms "neoplasia" and "neoplasia disorders", used interchangeably herein, refer to new cell growth that results from a loss of responsiveness to normal growth controls, e.g. to "neoplastic" cell growth. Neoplasia is also used interchangeably herein with the term "cancer" and for purposes of the present invention; cancer is one subtype of neoplasia. As used herein, the term "neoplasia disorder" also encompasses other cellular abnormalities, such as hyperplasia, metaplasia and dysplasia. The terms neoplasia, metaplasia, dysplasia and hyperplasia can be used interchangeably herein and refer generally to cells experiencing abnormal cell growth.

As used herein, the term "treatment" refers to both therapeutic treatment and prophylactic or preventative measures, wherein the object is to prevent or slow down (lessen) the targeted pathologic condition or condition. Those in need of treatment include those already with the condition as well as those prone to have the condition or those in whom the condition is to be prevented.

When introducing elements of the present invention or the preferred embodiment(s) thereof, the articles "a", "an", "the" and "said" are intended to mean that there are one or more of the elements. Throughout this specification and claims, the terms "comprising", "comprise", "comprises", "including" and "having" are intended to be inclusive and mean that there may be additional elements other than the listed elements.

### Anti-M-CSF antibodies:

In accordance with the present invention, it has been discovered that the stability of certain monoclonal anti-M-CSF antibodies that are described herein can be improved by mixing the anti-M-CSF antibodies with a chelating agent, such as ethylenediaminetetraacetic acid ("EDTA").

While not wishing to be bound by theory, it is believed that the presence of a chelating agent in the compositions of the present invention help to improve stability of the antibody polypeptide by reducing the incidence of one or more of the following: anti-M-CSF antibody aggregation, fragmentation, oxidation, freeze/thaw instability, discoloration, and/or deamidation. The present invention comprises anti-M-CSF antibody compositions having improved chemical and/or physical stability as compared to previously disclosed antibody compositions.

Therefore, in certain aspects, the present invention provides at least one composition comprising a chelating agent, such as EDTA and at least one monoclonal anti-M-CSF antibody or an antigen-binding portion thereof. In still other aspects, the aforementioned anti-M-CSF antibody compositions comprising a chelating agent can include additional excipients, including, but not limited to, one or more excipients that are chosen from buffers, tonicity agents, surfactants, antioxidants, and mixtures thereof.

The present invention provides novel formulations for anti-M-CSF antibodies. As used herein, the phrase "anti-M-CSF antibody" refers to any antibody, or any portion thereof, that is capable of binding to any portion of a macrophage colony-stimulating factor ("M-CSF") polypeptide that may be present within or isolated from any animal. In certain embodiments, the M-CSF polypeptide is a human M-CSF polypeptide.

Suitable anti-M-CSF antibodies for use with the present invention may be chosen from polyclonal or monoclonal antibodies. In certain aspects, the monoclonal anti-M-CSF antibody can be a murine, chimeric, humanized or human antibody. In further embodiments, the monoclonal anti-M-CSF antibody is a human monoclonal anti-M-CSF antibody. In further embodiments, the monoclonal anti-M-CSF antibody is an isolated monoclonal anti-M-CSF antibody. In further embodiments, the monoclonal anti-M-CSF antibody is a recombinant monoclonal anti-M-CSF antibody.

In certain embodiments, the anti-M-CSF antibodies which are suitable for use with the present invention include those anti-M-CSF antibodies and methods to prepare them that are described in U.S. Published Application No. 20050059113 to Bedian, et al. In other embodiments, the anti-M-CSF antibodies which are suitable for use with the present invention include any one or more of those anti-M-CSF monoclonal antibodies having the heavy and light chain amino acid sequences of the antibodies designated 252, 88, 100, 3.8.3, 2.7.3, 1.120.1, 9.14.41, 9.7.21F, 9.14.4, 8.10.3, 9.7.2, 9.7.2C-Ser, 9.14.4C-Ser, 8.10.3C-Ser, 8.10.3-CG2, 9.7.2-CG2, 9.7.2-CG4, 9.14.4-CG2, 9.14.4-CG4, 9.14.4-Ser, 9.7.2-Ser, 8.10.3-Ser, 8.10.3-CG4, 8.10.3FG1 or 9.14.4G1 in U.S. Published Application No. 20050059113 to Bedian, et al. In still other embodiments, the anti-M-CSF antibodies which are suitable for use with the present invention include those anti-M-CSF monoclonal antibodies having the heavy and light chain amino acid sequences of the antibody designated 8.10.3F in U.S. Published Application No. 20050059113 to Bedian, et al.

In addition, such anti-M-CSF antibodies may be chosen based on differences in the amino acid sequences in the constant region of their heavy chains. For example, the anti-M-CSF antibodies may be chosen from the IgG class, which have "gamma" type heavy chains. The class and subclass of anti-M-CSF antibodies may be determined by any method known in the art. In general, the class and subclass of an antibody may be determined using antibodies that are specific for a particular class and subclass of antibody. Such antibodies are commercially available. The class and subclass can be determined by ELISA, or Western Blot as well as other techniques. Alternatively, the class and subclass may be determined by sequencing all or a portion of the constant domains of the heavy and/or light chains of the antibodies, comparing their amino acid sequences to the known amino acid sequences of various class and subclasses of immunoglobulins, and determining.the class and subclass of the antibodies.

The anti-M-CSF antibody can be an IgG, an IgM, an IgE, an IgA, or an IgD molecule. In further embodiments, the anti-M-CSF antibody is an IgG and is an IgG1, tgG2, IgG3 or IgG4 subclass. One of the major mechanisms through which antibodies kill cells is through fixation of complement and participation in CDC. The constant region of an antibody plays an important role in connection with an antibody's ability to fix complement and participate in CDC. Thus, generally one selects the isotype of an antibody to either provide the ability of complement fixation, or not. In the case of the present invention, generally, as mentioned above, it is generally not preferred to utilize an antibody that kills the cells. There are a number of isotypes of antibodies that are capable of complement fixation and CDC, including, without limitation, the following: murine IgM, murine IgG2a, murine IgG2b, murine IgG3, human IgM, human IgG1, and human IgG3. In contrast, preferred isotypes which are not capable of complement fixation and CDC include, without limitation, human IgG2 and human IgG4. In addition to heavy chain sequence differences, the IgG antibodies differ within their subclass based on the number of disulfide bonds and length of the hinge region. For example, the IgG2 subclass has several differences distinct from the other subclasses. The IgG2 and IgG4 subclasses are known to have 4 disulfide bonds within their hinge region, while IgG1 has 2 and IgG3 has 11 disulfide bonds. Other differences for IgG2 antibodies include their reduced ability to cross the placenta and the inability of IgG2 antibodies to bind to lymphocyte Fc receptors. Thus, in certain embodiments, the anti-M-CSF antibody is subclass IgG2 or IgG4. In another preferred embodiment, the anti-M-CSF antibody is subclass IgG2.

In other embodiments, suitable anti-M-CSF antibodies may be chosen based on differences in the amino acid sequences in their heavy chains. For example; the anti-M-CSF antibodies of the present invention may have human gamma type heavy chains that utilize any of the following human V_{H} germline genes: V_{H}1, V_{H}2, V_{H}3, V_{H}4, or V_{H}5. For purposes of the present invention, the phrase "heavy chain variable region" is often abbreviated with the term (V_{H}). In certain embodiments, the anti-M-CSF antibodies utilize the human V_{H}3 germline gene. In further embodiments, the anti-M-CSF antibodies utilize the human V_{H} 3-48 germline gene. In still further embodiments, the anti-M-CSF antibodies utilize the D1-26 human D_{H} gene. In still further embodiments, the anti-M-CSF antibodies utilize the J_{H}4 human J_{H} gene.

In further embodiments, the anti-M-CSF antibodies may be chosen based on differences in the amino acid sequences of their light chains. For example, suitable anti-M-CSF antibodies may have lambda light chains or kappa light chains. However, in certain embodiments, the anti-M-CSF antibodies of the present invention have kappa light chains. In some embodiments, where the anti-M-CSF antibody comprises a kappa light chain, the polynucleotide encoding the variable domain of the light chain comprises a human V_{κ} L5, 012, L2, B3, L15, or A27 gene and a human Jκ1, Jκ2, Jκ3, Jκ4, or Jκ5 gene. In some embodiments where the antibody comprises a kappa light chain, the light chain variable region (V_{L}) is encoded in part by a human V_{κ}A27 gene and a human J_{κ}4 gene. In particular embodiments of the invention, the light chain variable domain is encoded by human V_{κ}A27/Jκ3 genes.

Table 1 lists the sequence identifiers (SEQ ID NOS) of the nucleic acids that comprise the heavy and light chains and the corresponding predicted amino acid sequences for the anti-M-CSF monoclonal antibody 8.10.3F. While DNA sequences encoding a signal polypeptide are shown in the sequence identifiers (SEQ ID NOS), the antibody typically does not comprise a signal polypeptide because the signal polypeptide is generally eliminated during post-translational modifications. In various embodiments of the invention, one or both of the heavy and light chains of the anti-M-CSF antibodies includes a signal sequence (or a portion of the signal sequence). In other embodiments of the invention, neither the heavy nor light chain of the anti-M-CSF antibodies includes a signal sequence. Table 1 also lists the heavy chain and light chain human germline gene derivation and sequences for the anti-M-CSF monoclonal antibody 8.10.3F.

**Table 1: Heavy and Light Chain Human Gene Utilization and Sequences**

| Antibody | Heavy Chain | | | | Light Chain | | |
|---|---|---|---|---|---|---|---|
| | SEQ ID NO: | V_{H} | D_{H} | J_{H} | SEQ ID NO: | V_{κ} | J_{κ} |
| 8.10.3F | **1** (nucleic acid) | 3-48 | 1-26 | 4b | **3** (nucleic acid) | A27 | 4 |
| | **2** (amino acid) | | | | **4** (amino acid) | | |

Some anti-M-CSF antibodies in accordance with the present invention were generated with a bias towards the utilization of the human V_{H} 3-48 heavy chain variable region. In XenoMouse^{™} mice, there are more than 30 distinct functional heavy chain variable genes with which to generate antibodies. Bias, therefore, is indicative of a preferred binding motif of the antibody-antigen interaction with respect to the combined properties of binding to the antigen and functional activity.

In some embodiments, the nucleic acid molecule encodes an amino acid sequence comprising 1, 2, 3, 4, 5, 6, 7, 8, 9, 10, 11, 12, 13, 14, 15, 16, 17 or 18 mutations compared to the germline amino acid sequence of the human V, D or J genes. In some embodiments, said mutations are in the heavy chain variable region region. In some embodiments, said mutations are in the CDR regions.

In some embodiments, the nucleic acid molecule encodes one or more amino acid mutations compared to the germline sequence that are identical to amino acid mutations found in the V_{H} of monoclonal antibody 8.10.3F. In some embodiments, the nucleic acid encodes at least three amino acid mutations compared to the germline sequences that are identical to at least three amino acid mutations found in one of the above-listed monoclonal antibodies.

In some embodiments, the nucleic acid molecule encodes a V_{L} amino acid sequence comprising one or more variants compared to germline sequence that are identical to the variations found in the V_{L} of one of the antibodies 8.10.3F.

In some embodiments, the nucleic acid molecule encodes at least three amino acid mutations compared to the germline sequence found in the V_{L} of the antibody 8.10.3.

In some embodiments, the antibody is a single-chain antibody (scFv) in which a V_{L} and V_{H} domains are paired to form a monovalent molecules via a synthetic linker that enables them to be made as a single protein chain. Bird et al., Science 242:423-426 (1988) and Huston et al., Proc. Natl. Acad. Sci. USA 85:5879-5883 (1988). In some embodiments, the antibodies are diabodies, *i.e.,* are bivalent antibodies in which V_{H} and V_{L} domains are expressed on a single polypeptide chain, but using a linker that is too short to allow for pairing between the two domains on the same chain, thereby forcing the domains to pair with complementary domains of another chain and creating two antigen binding sites. See *e.g.,* Holliger P. et al., Proc. Natl. Acad. Sci. USA 90:6444-6448 (1993 and Poljak R. J. et al., Structure 2:1121-1123 (1994). In some embodiments, one or more CDRs from an antibody of the invention may be incorporated into a molecule either covalently or noncovalently to make it an immunoadhesin that specifically binds to M-CSF. In such embodiments, the CDR(s) may be incorporated as part of a larger polypeptide chain, may be covalently linked to another polypeptide chain, or may be incorporated noncovalently.

In another embodiment, the anti-M-CSF antibody has selectivity (or specificity) for M-CSF that is at least 100 times greater than its selectivity for any other polypeptide. In some embodiments, the anti-M-CSF antibody does not exhibit any appreciable specific binding to any other protein other than M-CSF. One can determine the selectivity of the anti-M-CSF antibody for M-CSF using methods well known in the art following the teachings of the specification. For instance, one can determine the selectivity using Western blot, FACS, ELISA, or RIA. Thus, in some embodiments, the monoclonal anti-M-CSF antibody is capable of specifically binding to M-CSF.

In some embodiments, the C-terminal lysine of the heavy chain of the anti-M-CSF antibody of the invention is not present.

In some embodiments, the nucleic acid molecule encodes a light chain amino acid sequence that is at least 70%, 75%, 80%, 85%, 90%, 95%, 97%, 98%, 99% or 100% identical to a light chain amino acid sequence of antibody 8.10.3F of SEQ ID NO: 4, or to a V_{L} amino acid sequence of SEQ ID NO 6. Nucleic acid molecules of the invention include nucleic acids that hybridize under highly stringent conditions, such as those described above, to a nucleic acid sequence encoding the light chain amino acid sequence of SEQ ID NO: 4, or that has the polynucleotide sequence of SEQ ID NO: 3.

In some embodiments, the nucleic acid molecule comprises a polynucleotide sequence that encodes the light chain amino acid sequence of monoclonal antibody 8.10.3F, or a portion thereof. In some embodiments, the nucleic acid molecule comprises a polynucleotide sequence that encodes the light chain polynucleotide sequence of monoclonal antibody 8.10.3F of SEQ ID NO: 3, or a portion thereof. In some embodiments, the nucleic acid molecule comprises a polynucleotide sequence that encodes the V_{L} amino acid sequence of monoclonal antibody 8.1.0.3F of SEQ ID NO: 6, or a portion thereof. In some embodiments, said portion comprises at least the CDR2 region. In some embodiments, the nucleic acid encodes the amino acid sequence of the light chain CDRs of said antibody. In some embodiments, said portion is a contiguous portion comprising CDR1-CDR3.

In some embodiments, the nucleic acid molecule encodes a heavy chain amino acid sequence that is at least 70%, 75%, 80%, 85%, 90%, 95%, 97%, 98%, 99% or 100% identical to a heavy chain amino acid sequence of antibody 8.10.3F of SEQ ID NO: 2, or to a V_{H} amino acid sequence of SEQ ID NO 5. Nucleic acid molecules of the invention include nucleic acids that hybridize under highly stringent conditions, such as those described above, to a nucleic acid sequence encoding the heavy chain amino acid sequence of SEQ ID NO: 2, or that has the polynucleotide sequence of SEQ ID NO: 1.

In some embodiments, the nucleic acid molecule comprises a polynucleotide sequence that encodes the heavy chain amino acid sequence of monoclonal antibody 8.10.3F, or a portion thereof. In some embodiments, the nucleic acid molecule comprises a polynucleotide sequence that encodes the heavy chain polynucleotide sequence of monoclonal antibody 8.10.3F of SEQ ID NO: 2, or a portion thereof. In some embodiments, the nucleic acid molecule comprises a polynucleotide sequence that encodes the V_{H} amino acid sequence of monoclonal antibody 8.10.3F of SEQ ID NO: 5, or a portion thereof. In some embodiments, said portion comprises at least the CDR2 region. In some embodiments, the nucleic acid encodes the amino acid sequence of the light chain CDRs of said antibody. In some embodiments, said portion is a contiguous portion comprising CDR1-CDR3.

In further embodiments, the nucleic acid molecule comprises a polynucleotide sequence that encodes at least a portion of the V_{H} amino acid sequence of 8.10.3F (SEQ ID NO: 5) or said sequence having conservative amino acid mutations and/or a total of three or fewer non-conservative amino acid substitutions. In various embodiments the sequence encodes one or more CDR regions, preferably a CDR3 region, all three CDR regions, a contiguous portion including CDR1-CDR3, or the entire V_{H} region.

In still further embodiments, the nucleic acid molecule comprises a polynucleotide sequence that encodes the heavy chain amino acid sequence of SEQ ID NO: 1 or a portion thereof. In still further embodiments, the nucleic acid molecule comprises a polynucleotide sequence that encodes the heavy chain variable domain amino acid sequence of SEQ ID NO: 5 or a portion thereof.

In another embodiment, the nucleic acid encodes a full-length light chain of an antibody selected from 8.10.3F, or a light chain comprising the amino acid sequence of SEQ ID NO: 4 and a constant region of a light chain, or a light chain comprising a mutation. Further, the nucleic acid may comprise the light chain polynucleotide sequence of SEQ ID NO: 3 and the polynucleotide sequence encoding a constant region of a light chain, or a nucleic acid molecule encoding a light chain comprise a mutation.

In some embodiments, the nucleic acid molecule comprises a polynucleotide sequence that encodes at least a portion of the V_{H} amino acid sequence of 8.10.3F (SEQ ID NO: 5) or said sequence having conservative amino acid mutations and/or a total of three or fewer non-conservative amino acid substitutions. In various embodiments the sequence encodes one or more CDR regions, preferably a CDR3 region, all three CDR regions, a contiguous portion including CDR1-CDR3, or the entire V_{H} region.

In another aspect of the invention, the anti-M-CSF antibodies demonstrate both species and molecule selectivity. In some embodiments, the anti-M-CSF antibody binds to human, cynomologus monkey and mouse M-CSF. Following the teachings of the specification, one may determine the species selectivity for the anti-M-CSF antibody using methods well known in the art. For instance, one may determine the species selectivity using Western blot, FACS, ELISA, RIA, a cell proliferation assay, or an M-CSF receptor-binding assay. In a preferred embodiment, one may determine the species selectivity using a cell proliferation assay or ELISA.

In another embodiment, the anti-M-CSF antibody has selectivity for M-CSF that is at least 100 times greater than its selectivity for GM-/G-CSF. In some embodiments, the anti-M-CSF antibody does not exhibit any appreciable specific binding to any other protein other than M-CSF. One can determine the selectivity of the anti-M-CSF antibody for M-CSF using methods well known in the art following the teachings of the specification. For instance one can determine the selectivity using Western blot, FACS, ELISA, or RIA.

### Preparation of the Monoclonal Anti-M-CSF Antibody Formulations:

The anti-M-CSF antibody typically is formulated as a pharmaceutical composition for parenteral administration to a subject. In certain embodiments, the pharmaceutical composition is a liquid composition.

In one embodiment, the invention is directed to compositions comprising an anti-M-CSF antibody and a chelating agent. In another embodiment, the invention is directed to a liquid pharmaceutical composition comprising an anti-M-CSF antibody and a chelating agent. In another embodiment, the invention is directed to a composition comprising an anti-M-CSF antibody and EDTA. In another embodiment, the invention is directed to a liquid pharmaceutical composition comprising an anti-M-CSF antibody and EDTA.

The term "pharmaceutical composition" refers to preparations which are in such form as to permit the biological activity of the active ingredients to be effective. In some embodiments, the pharmaceutical composition is a liquid pharmaceutical composition. "Pharmaceutically acceptable excipients" (vehicles, additives) are those, which can reasonably (*i.e.,* safely) be administered to a subject to provide an effective dose of the active ingredient employed. The term "excipient" or "carrier" as used herein refers to an inert substance, which is commonly used as a diluent, vehicle, preservative, binder or stabilizing agent for drugs. As used herein, the term "diluent" refers to a pharmaceutically acceptable (safe and non-toxic for administration to a human) solvent and is useful for the preparation of the compositions herein. Exemplary diluents include, but are not limited to, sterile water and bacteriostatic water for injection (BWFI).

The compositions of the present invention involve one or more anti-M-CSF monoclonal antibodies of the invention in combination with pharmaceutically acceptable excipients, which comprise a chelating agent. The compositions of the present invention involve one or more anti-M-CSF monoclonal antibodies of the invention in combination with pharmaceutically acceptable excipients, which comprise histidine and/or a chelating agent.

In one embodiment, the composition comprises at least one antibody comprising an amino acid sequence that is at least 90%, 95% or 99% identical to a light chain sequence shown in SEQ ID NO: 4, and further comprising an amino acid sequence that is at least 90%, 95%, or 99% identical to a heavy chain amino acid sequence shown in SEQ ID NO: 2; and a chelating agent, wherein the antibody binds to human M-CSF.

In one embodiment, the composition comprises at least one antibody comprising an amino acid sequence that is at least 90%, 95% or 99% identical to a light chain sequence shown in SEQ ID NO: 4, and further comprising an amino acid sequence that is at least 90%, 95%, or 99% identical to a heavy chain amino acid sequence shown in SEQ ID NO: 2; and histidine, wherein the antibody binds to human M-CSF.

In another embodiment, the composition comprises at least one antibody comprising an amino acid sequence that is at least 90%, 95% or 99% identical to a light chain sequence shown in SEQ ID NO: 4, and further comprising an amino acid sequence that is at least 90%, 95%, or 99% identical to a heavy chain amino acid sequence shown in SEQ ID NO: 2; and a chelating agent, wherein the antibody binds to human M-CSF and further comprising at least one or more pharmaceutically acceptable excipient that is chosen from buffers, tonicity agents, antioxidants, and surfactants.

In another embodiment, the composition comprises at least one antibody comprising an amino acid sequence that is at least 90%, 95% or 99% identical to a light chain sequence shown in SEQ ID NO: 4, and further comprising an amino acid sequence that is at least 90%, 95%, or 99% identical to a heavy chain amino acid sequence shown in SEQ ID NO: 2; and histidine, wherein the antibody binds to human M-CSF and further comprising at least one or more pharmaceutically acceptable excipient that is chosen from buffers, chelating agents, tonicity agents, antioxidants, and surfactants.

In another embodiment, the composition comprises at least one antibody comprising a heavy chain amino acid sequence that comprises the variable region of SEQ ID NO: 2 and a light chain amino acid sequence that comprises the variable region SEQ ID NO: 4; and a chelating agent, wherein the antibody binds to human M-CSF.

In another embodiment, the composition comprises at least one antibody comprising a human monoclonal IgG2 antibody having the heavy and light chain amino acid sequences of antibody 8.10.3F; and a chelating agent, wherein the antibody binds to human M-CSF.

The concentration of the anti-M-CSF antibody in the compositions of the present invention is generally at least about 0.1 milligram per milliliter (mg/ml) or higher, at least about 1.0 mg/ml or higher, at least about 10 mg/ml or higher, at least about 20 mg/ml or higher, at least about 50 mg/ml or higher, at least about 100 mg/ml or higher, or at least about 200 mg/ml or higher. In certain embodiments, the concentration of the anti-M-CSF antibody generally ranges from about 0.1 mg/ml to about 200 mg/ml, from about 0.5 mg/ml to about 100 mg/ml, from about 1 mg/ml to about 50 mg/ml, from about 2.0 mg/ml to about 35 mg/ml, from about 5.0 mg/ml to about 25 mg/ml, or from about 7 mg/ml to about 15 mg/ml. In one embodiment, the concentration of the anti-M-CSF antibody in the compositions of the present invention is generally about 5 mg/ml, about 10 mg/ml, about 20 mg/ml, about 50 mg/ml, about 65 mg/ml, about 70 mg/ml, about 75 mg/ml, about 80 mg/ml, about 85 mg/ml, or about 100 mg/ml. In another embodiment, the concentration of the anti-M-CSF antibody in the compositions range from about 1 mg/ml to about 50 mg/ml. In one embodiment, the concentration of the anti-M-CSF antibody in the composition is about 10 mg/ml. In another embodiment, the concentration of the anti-M-CSF antibody in the composition is about 75 mg/ml.

In another embodiment, the concentration of the anti-M-CSF antibody in the liquid pharmaceutical composition ranges from about 50 mg/ml to about 100 mg/ml. In some embodiments, higher antibody concentrations can be used where the composition is intended for subcutaneous delivery.

As used herein, the terms "chelating agent" generally refers to an excipient that can form at least one bond (*e.g.,* covalent, ionic, or otherwise) to a metal ion. A chelating agent is typically a multidentate ligand that can be used in selected liquid compositions as a stabilizer to complex with species, which might promote instability. Often, compounds that can act as a chelating agent will have electron-rich functional groups. Suitable electron-rich functional groups include carboxylic acid groups, hydroxy groups and amino groups. Arrangement of these groups in aminopolycarboxylic acids, hydroxypolycarboxylic acids, hydroxyaminocarboxylic acids, and the like, result in moieties that have the capacity to bind metal.

However, the present invention is not intended to be limited to chelating agents primarily by the chelating agent's ability to form bonds with a metal ion. Therefore, the present invention is not intended to be limited by any specific mechanism by which the chelating agent acts in the formulations of the present invention and the excipients termed chelating agents herein may achieve their properties through mechanisms that are altogether unrelated to the chelating agent's ability to form bonds with a metal ion.

Chelating agents that are suitable for use in the present invention, include, but are not limited to, aminopolycarboxylic acids, hydroxyaminocarboxylic acids, N-substituted glycines, 2-(2-amino-2-oxocthyl) aminoethane sulfonic acid (BES), deferoxamine (DEF), citric acid, niacinamide, and desoxycholates. Examples of suitable aminopolycarboxylic acids include ethylenediaminetetraacetic acid (EDTA), diethylenetriamine pentaacetic acid 5 (DTPA), nitrilotriacetic acid (NTA), N-2-acetamido-2-iminodiacetic acid (ADA), bis(aminoethyl)glycolether, N,N,N',N'-tetraacetic acid (EGTA), trans-diaminocyclohexane tetraacetic acid (DCTA), glutamic acid, and aspartic acid. Examples of suitable hydroxyaminocarboxylic acids include N-hydroxyethyliminodiacetic acid (HIMDA), N,N-bis-hydroxyethylglycine (bicine) and N-(trishydroxymethylmethyl) 10 glycine (tricine). An example of a suitable N-substituted glycine is glycylglycine. An example of a suitable desoxycholate is sodium desoxycholate. Mixtures of two or more chelating agents are also encompassed by the present invention.

Chelating agents used in the invention can be present, where possible, as the free acid or free base form of the compound (*e.g.,* referred to interchangeably herein as "EDTA" or "edetate") or as a corresponding salt form (*e.g.,* the corresponding acid addition salt or base addition salt, such as disodium edetate). Suitable acid addition salts, *e.g.,* include alkali metal salts (*e.g.,* sodium or potassium salts), alkaline earth metal salts (*e.g.,* calcium salts), and salts can be prepared using other weakly bound metal ions. As is known in the art, the nature of the salt and the number of charges to be neutralized will depend on the number of carboxyl groups present and the pH at which the stabilizing chelating agent is supplied. As is also known in the art, chelating agents have varying strengths with which particular target ions are bound. By way of further illustration, suitable salts of EDTA include dipotassium edetate, disodium edetate, edetate calcium disodium, sodium edetate, trisodium edetate, and potassium edetate; and a suitable salt of deferoxamine (DEF) is deferoxamine mesylate (DFM).

Chelating agents used in the invention can be present as an anhydrous, solvated or hydrated form of the compound or corresponding salt. Where the chelating agent is in a solvated or hydrated form, it can be present in varying states of solvation or hydration (including, *e.g.,* anhydrous, hydrated, dihydrated, and trihydrated forms). By way of further illustration, a suitable hydrate of EDTA is disodium EDTA dihydrate; and suitable forms of citric acid include anhydrous citric acid, citric acid monohydrate, and trisodium citrate-dihydrate.

Suitable chelating agents used in the antibody compositions of the present invention also include, for example, those that bind to metal ions in solution to render them unable to react with available O₂, thereby minimizing or preventing generation of hydroxyl radicals which are free to react with and degrade the antibody. Chelating agents can lower the formation of reduced oxygen species, reduce acidic species (*e.g.,* deamidation) formation, reduce antibody aggregation, and/or reduce antibody fragmentation in the compositions of the present invention. Such chelating agents can reduce or prevent degradation of an antibody that is formulated without the protection of a chelating agent.

When a concentration of a chelating agent is referred to, it is intended that the recited concentration represent the molar concentration of the free acid or free base form of the chelating agent. For example, the concentration of chelating agent in certain liquid pharmaceutical compositions generally ranges from about 0.01 micromolar to about 50 millimolar, from about 1 micromolar to about 10.0 millimolar, from about 15 micromolar to about 5.0 millimolar, from about 0.01 millimolar to about 1.0 millimolar, or from about 0.03 millimolar to about 0.5 millimolar. In certain embodiments, the concentration of chelating agent in the liquid pharmaceutical composition can be about 0.01 millimolar, 0.02 millimolar, 0.027 millimolar, 0.03 millimolar, about 0.04 millimolar, about 0.05 millimolar, about 0.06 millimolar, about 0.07 millimolar, about 0.10 millimolar, about 0.20 millimolar, about 0.26 millimolar, about 0.27 millimolar, about 0.30 millimolar, about 0.31 millimolar, about 0.34 millimolar, about 0.40 millimolar, about 0.50 millimolar, or about 1.0 millimolar. In certain embodiments, the concentration of chelating agent is about 0.027 millimolar, about 0.05 millimolar, about 0.13 millimolar, or about 0.27 millimolar. In one embodiment, the concentration of chelating agent is about 0.05 millimolar. In another embodiment, the concentration of chelating agent is about 0.13 millimolar.

Unless stated otherwise, the concentrations listed herein are those concentrations at ambient conditions, (*i.e.,* at 25°C and atmospheric pressure). Ranges intermediate to the above-recited chelating agent concentrations are also intended to be part of this invention. For example, ranges of values using a combination of any of the above-recited values as upper and/or lower limits are intended to be included.

In one embodiment, the chelating agent is selected from the group consisting of EDTA, DTPA, DFM, and mixtures thereof: In another embodiment, the chelating is agent is DFM. In another embodiment, the chelating agent is EDTA. In another embodiment, the chelating agent is DTPA. In another embodiment, the liquid pharmaceutical composition comprises EDTA in an amount that generally ranges from about 0.01 micromolar to about 50 millimolar, from about 1 micromolar to about 20.0 millimolar, from about 15 micromolar to about 10.0 millimolar, from about 0.01 millimolar to about 5.0 millimolar, or from about 0.03 millimolar to about 1 millimolar. In certain embodiments, the concentration of EDTA in the liquid pharmaceutical composition can be about 0.01 millimolar, 0.02 millimolar, 0.027 millimolar, 0.03 millimolar, about 0.04 millimolar, about 0.05 millimolar, about 0.06 millimolar, about 0.07 millimolar, about 0.10 millimolar, about 0.20 millimolar, about 0.26 millimolar, about 0.27 millimolar, about 0.30 millimolar, about 0.31 millimolar, about 0.34 millimolar, about 0.40 millimolar, about 0.50 millimolar, or about 1.0 millimolar. In certain embodiments, the concentration of EDTA is about 0.027 millimolar, about 0:05 millimolar, about 0.13 millimolar, or about 0.27 millimolar. In one embodiment, the concentration of EDTA is about 0.05 millimolar. In another embodiment, the concentration of EDTA is about 0.13 millimolar.

As noted above, the compositions of the present invention optionally may further comprise a buffer in addition to a chelating agent. As used herein, the term "buffer" refers to an added composition that allows a liquid antibody formulation to resist changes in pH.

In certain embodiments, the added buffer allows a liquid antibody formulation to resist changes in pH by the action of its acid-base conjugate components. For example, a buffered formulation may be prepared by adding L-histidine-HCl (L-histidine-hydrochloride) and L-histidine in the appropriate amounts to arrive at a desired pH. However, in other embodiments, the added buffer allows a liquid antibody formulation to resist changes in pH by the action of its acid-base conjugate components. By way of a second example, a buffered formulation may be prepared by adding an acid, such as hydrochloric acid, and L-histidine in the appropriate amounts to arrive at a desired pH.

Examples of suitable buffers include, but are not limited to, acetate (*e.g*., sodium acetate), succinate (*e.g*., sodium succinate), gluconate, citrate (e.g., and other organic acid buffers, including, but not limited to, buffers such as amino acids (*e.g*., histidine), acetic acid, phosphoric acid and phosphates, ascorbate, tartartic acid, maleic acid, glycine, lactate, lactic acid, ascorbic acid, imidazoles, carbonic acid and bicarbonates, succinic acid, sodium benzoic acid and benzoates, gluconate, edetate (EDTA), acetate, malate, imidazole, tris, phosphate, and mixtures thereof. In one embodiment, the buffer is acetate.

In another embodiment, the buffer is histidine. The histidine starting material used to prepare the compositions of the present invention can exist in different forms. For example, the histidine can be an enantiomeric (*e.g*., L- or D-enantiomer) or racemic form of histidine, a free acid or free base form of histidine, a salt form (e.g., a monohydrochloride, dihydrochloride, hydrobromide, sulfate, or acetate salt) of histidine, a solvated form of histidine, a hydrated form (*e.g*., monohydrate) of histidine, or an anhydrous form of histidine. The purity of histidine base and/or salt used to prepare the compositions generally can be at least about 98%, at least about 99%, or at least about 99.5%. As used herein, the term "purity" in the context of histidine refers to chemical purity of histidine as understood in the art, *e.g*., as described in The Merck Index, 13th ed., O'Neil et al. ed. (Merck & Co., 2001).

When a concentration of a buffer is referred to, it is intended that the recited concentration represent the molar concentration of the free acid or free base form of the buffer. For example, the concentration of the buffer when present in certain liquid pharmaceutical compositions can range from about 0.1 millimolar (mM) to about 100 mM. In one embodiment, the concentration of the buffer is from about 1 mM to about 50 mM. In another embodiment, the concentration of the buffer is from about 5 mM to about 30 mM. In various embodiments, the concentration of the buffer is about 1 mM, about 5 mM, about 10 mM, about 15 mM, about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM or about 100 mM. In one embodiment, the concentration of histidine in the pharmaceutical composition is about 10mM. In another embodiment, the pharmaceutical composition contains about 10 mM of L-histidine (in base form). In another embodiment, the concentration of histidine in the pharmaceutical composition is about 20 mM. In another embodiment, the pharmaceutical composition contains about 20 mM of L-histidine (in base form). Ranges intermediate to the above-recited histidine concentrations are also intended to be part of this invention. For example, ranges of values using a combination of any of the above-recited values as upper and/or lower limits are intended to be included.

In general, the buffer is used to maintain an acceptable pH level (which can affect antibody stability) in the liquid pharmaceutical composition. The liquid pharmaceutical composition typically is buffered to maintain a pH in the range of from about 4 to about 8; from about 4.5 to about 7; from about 5.0 to 6.5, or from about 5.3 to about 6.3. Ranges intermediate to the above-recited pH's are also intended to be part of this invention. For example, ranges of values using a combination of any of the above-recited values as upper and/or lower limits are intended to be included. In one embodiment, the liquid pharmaceutical composition is buffered to maintain a pH of about 5.5. In another embodiment, the liquid pharmaceutical composition is buffered to maintain a pH of about 6.0.

As noted above, the compositions of the present invention optionally may further comprise a pharmaceutically acceptable tonicity agent in addition to a chelating agent. As used herein, the terms "tonicity agent" or "tonicifier" refers to an excipient that can adjust the osmotic pressure of a liquid antibody formulation. In certain embodiments, the tonicity agent can adjust the osmotic pressure of a liquid antibody formulation to isotonic so that the antibody formulation is physiologically compatible with the cells of the body tissue of the subject. In still other embodiments, the "tonicity agent" may contribute to an improvement in stability of any of the anti-M-CSF antibodies described herein. An "isotonic" formulation is one that has essentially the same osmotic pressure as human blood. Isotonic formulations generally have an osmotic pressure from about 250 to 350 mOsm. The term "hypotonic" describes a formulation with an osmotic pressure below that of human blood. Correspondingly, the term "hypertonic" is used to describe a formulation with an osmotic pressure above that of human blood. Isotonicity can be measured using a vapor pressure or ice-freezing type osmometer, for example.

The tonicity agent used to prepare the compositions of the present invention can exist in different forms. When the tonicity agent is referred to, it is intended that all of these different forms are encompassed by the name of the tonicity agent. For example, the tonicity agent can be in an enantiomeric (*e.g*., L- or D-enantiomer) or racemic form; isomers such as alpha or beta, including alpha, alpha; or beta, beta; or alpha, beta; or beta, alpha; a free acid or free base form; a hydrated form (*e.g*., monohydrate), or an anhydrous form.

In one embodiment, the tonicity agent is a saccharide. As used herein, the term "saccharide" refers to a class of molecules that are derivatives of polyhydric alcohols. Saccharides are commonly referred to as carbohydrates and may contain different amounts of sugar (saccharide) units, *e.g*., monosaccharides, disaccharides and polysaccharides. Saccharides that are suitable for use as a tonicity agent in the present invention, include, but are not limited to, saccharides selected from the group consisting of fructose, glucose, mannose, sorbose, xylose, lactose, maltose, sucrose, dextran, pullulan, dextrin, cyclodextrins, soluble starch, hydroxyethyl starch, water-soluble glucans, and mixtures thereof.

In another embodiment, the tonicity agent is a polyol. As used herein, the term "polyol" refers an excipient with multiple hydroxyl groups, and includes sugars (reducing and nonreducing sugars), sugar alcohols and sugar acids. In one embodiment, the polyol has a molecular weight that is less than about 600 kD (*e.g*., in the range from about 120 to about 400 kD). A "reducing sugar" is one which contains a hemiacetal group that can reduce metal ions or react covalently with lysine and other amino groups in proteins and a "nonreducing sugar" is one which does not have these properties of a reducing sugar. Polyols that are suitable for use as a tonicity agent in the present invention, include, but are not limited to, polyols selected from the group consisting of mannitol, trehalose, sorbitol, erythritol, isomalt, lactitol, maltitol, xylitol, glycerol, lactitol, propylene glycol, polyethylene glycol, inositol, and mixtures thereof. In one embodiment, the tonicity agent is a non-reducing sugar selected from the group consisting of trehalose, sucrose, and mixtures thereof.

In one embodiment, the tonicity agent is mannitol. In another embodiment, the tonicity agent is D-mannitol. In another embodiment, the tonicity agent is trehalose. In another embodiment, the tonicity agent is α-trehalose dihydrate. In another embodiment, the tonicity agent is sucrose.

In one embodiment, concentration of the tonicity agent in the liquid pharmaceutical composition ranges from about 1 millimolar to about 600 millimolar, from about 1 millimolar to about 400 millimolar, from 1 millimolar to about 300 millimolar, or from 200 millimolar to about 275 millimolar. In one another embodiment, the tonicity agent is mannitol and is present in the liquid pharmaceutical composition at a concentration of about 247 millimolar. In another embodiment, the tonicity agent is trehalose and is present in the liquid pharmaceutical composition at a concentration of about 222 millimolar. In another embodiment, the tonicity agent is trehalose and is present in the liquid pharmaceutical composition at a concentration of about 238 millimolar. In another embodiment, the tonicity agent is sucrose is present in the liquid pharmaceutical composition at a concentration of about 263 millimolar.

In one embodiment, concentration of the tonicity agent in the liquid pharmaceutical composition ranges from about 1 mg/ml to about 300 mg/ml, from about 1 mg/ml to about 200 mg/ml, or from about 50 mg/ml to about 150 mg/ml. In another embodiment, the tonicity agent is mannitol and is present in the liquid pharmaceutical composition at a concentration of about 45 mg/ml millimolar. In another embodiment, the tonicity agent is trehalose and is present in the liquid pharmaceutical composition at a concentration of about 84 mg/ml. In another embodiment, the tonicity agent is trehalose and is present in the liquid pharmaceutical composition at concentration of about 90 mg/ml. In another embodiment, the tonicity agent is sucrose and is present in the liquid pharmaceutical composition at a concentration of about 90 mg/ml.

In one embodiment, the tonicity agent is a salt, such as sodium chloride. In one embodiment, when the tonicity agent is a salt, the concentration of the salt in the liquid pharmaceutical composition ranges from about 1 mg/ml to about 20 mg/ml. In another embodiment, the tonicity agent is sodium chloride and the concentration of the sodium chloride in the liquid pharmaceutical composition is about 8.18 mg/ml.

Ranges intermediate to the above-recited tonicity agent concentrations are also intended to be part of this invention. For example, ranges of values using a combination of any of the above-recited values as upper and/or lower limits are intended to be included.

As noted above, the compositions of the present invention optionally may runner comprise a pharmaceutically acceptable surfactant in addition to a chelating agent. As used herein, the term "surfactant" refers to an excipient that can alter the surface tension of a liquid antibody formulation. In certain embodiments, the surfactant reduces the surface tension of a liquid antibody formulation. In still other embodiments, the "surfactant" may contribute to an improvement in stability of any of the anti-M-CSF antibodies described herein. For example, the surfactant may reduce aggregation of the formulated antibody and/or minimize the formation of particulates in the formulation and/or reduces adsorption. The surfactant may also improve stability of the antibody during and after a freeze/thaw cycle.

Suitable surfactants include polysorbate surfactants, poloxamers (*e.g*., poloxamer 18 and 407), triton surfactants such as Triton X-100®, polysorbate surfactants such as Tween 20® and Tween 80®, sodium dodecyl sulfate, sodium laurel sulfate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauroamidopropyl-betaine, cocamidopropyl-betaine, linoleamidopropyl-betaine, myristamidopropyl-betaine, palmidopropyl-betaine, isostearamidopropyl-betaine, myristamidopropyl-dimethylamine, palmidopropyl-dimethylamine, isostearamidopropyl-dimethylamine, sodium methyl cocoyl-taurate, disodium methyl oleyl-taurate, dihydroxypropyl PEG 5 linoleammonium chloride, polyethylene glycol, polypropylene glycol, and mixtures thereof.

In one embodiment, the surfactant is a polysorbate surfactant comprising at least one excipient that is selected from the group consisting of polysorbate 20, polysorbate 21, polysorbate 40, polysorbate 60, polysorbate 61, polysorbate 65, polysorbate 80, polysorbate 81, polysorbate 85, and mixtures thereof. In another embodiment, the liquid pharmaceutical composition comprises polysorbate 80.

The concentration of the surfactant when present in the liquid pharmaceutical composition generally ranges from about 0.01 mg/ml to about 10 mg/ml, from about 0.05 mg/ml to about 5.0 mg/ml, from about 0.1 mg/ml to about 1.0 mg/ml, or from about 0.2 mg/ml to about 0.7 mg/ml. In another embodiment, the concentration of the surfactant ranges from about 0.05 millimolar to about 1.0 millimolar. In another embodiment, the surfactant is present in an amount that is about 0.2 mg/ml. In another embodiment, the surfactant is present in an amount that is about 0.5 mg/ml. In one embodiment, the liquid pharmaceutical composition contains about 0.2 mg/ml polysorbate 80. In another embodiment, the liquid pharmaceutical composition contains about 0.4 mg/ml polysorbate 80. In another embodiment, the liquid pharmaceutical composition contains about 0.5 mg/ml polysorbate 80.

Ranges intermediate to the above-recited surfactant concentrations are also intended to be part of this invention. For example, ranges of values using a combination of any of the above-recited values as upper and/or lower limits are intended to be included.

As noted above, the compositions of the present invention optionally may further comprise a pharmaceutically acceptable antioxidant in addition to a chelating agent. Suitable antioxidants include, but are not limited to, methionine, sodium thiosulfate, catalase, and platinum. For example, the liquid pharmaceutical composition may contain methionine in a concentration that ranges from 1 mM to about 100 mM, and in particular, is about 27 mM.

In one embodiment, the present invention encompasses compositions comprising at least one antibody comprising an amino acid sequence that is at least 90% identical to a heavy chain amino acid sequence shown in SEQ ID NO: 2, and further comprising an amino acid sequence that is at least 90% identical to a light chain amino acid sequence shown in SEQ ID NO: 4, wherein the antibody binds to human M-CSF; and a chelating agent.

In another embodiment, the present invention encompasses compositions comprising at least one antibody comprising an amino acid sequence that is at least 90% identical to a heavy chain amino acid sequence shown in SEQ ID NO: 2 without the signal sequence, and further comprising an amino acid sequence that is at least 90% identical to a light chain amino acid sequence shown in SEQ ID NO: 4 without the signal sequence, wherein the antibody binds to human M-CSF; and a chelating agent.

In one embodiment, the present invention encompasses compositions comprising at least one antibody comprising an amino acid sequence that is at least 95% identical to a heavy chain amino acid sequence shown in SEQ ID NO: 2, and further comprising an amino acid sequence that is at least 95% identical to a light chain amino acid sequence shown in SEQ ID NO: 4, wherein the antibody binds to human M-CSF and has a purity of at least about 95%; and a chelating agent.

In one embodiment, the present invention encompasses compositions comprising at least one antibody comprising an amino acid sequence that is at least 99% identical to a heavy chain amino acid sequence shown in SEQ ID NO: 2, and further comprising an amino acid sequence that is at least 99% identical to a light chain amino acid sequence shown in SEQ ID NO: 4, wherein the antibody binds to human M-CSF and has a purity of at least about 95%; and a chelating agent.

In one embodiment, the present invention encompasses compositions comprising at least one antibody comprising an amino acid sequence that is at least 90% identical to a heavy chain amino acid sequence shown in SEQ ID NO: 2, and further comprising an amino acid sequence that is at least 90% identical to a light chain amino acid sequence shown in SEQ ID NO: 4, wherein the antibody binds to human M-CSF and the antibody has a purity of at least about 90%, 95% or 100%; and a chelating agent.

In one embodiment, the present invention encompasses a liquid pharmaceutical composition comprising at least one antibody comprising an amino acid sequence that is at least 95% identical to a heavy chain amino acid sequence shown in SEQ ID NO: 2, and further comprising an amino acid sequence that is at least 95% identical to a light chain amino acid sequence shown in SEQ ID NO: 4; and a chelating agent, wherein the antibody binds to human M-CSF, and the composition has an antibody concentration of from about 1.0 mg/ml to about 100 mg/m

In one embodiment, the present invention encompasses a liquid pharmaceutical composition comprising at least one human monoclonal IgG2 anti-M-CSF antibody having the heavy and light chain amino acid sequences of antibody 8.10.3F; and a chelating agent, wherein the antibody binds to human M-CSF, and the composition has an antibody concentration of from about 1.0 mg/ml to about 100 mg/ml.

In one embodiment, the present invention encompasses a liquid pharmaceutical composition comprising at least one antibody comprising an amino acid sequence that is at least 95% identical to a heavy chain amino acid sequence shown in SEQ ID NO: 2, and further comprising an amino acid sequence that is at least 95% identical to a light chain amino acid sequence shown in SEQ ID NO: 4; and a chelating agent, wherein the antibody binds to human M-CSF, and the composition contains a concentration of antibody that is at least about 5 mg/ml, at least about 10 mg/ml, at least about 15 mg/ml or at least about 20 mg/ml.

In one embodiment, the present invention encompasses a liquid pharmaceutical composition comprising at least one antibody comprising an amino acid sequence that is at least 95% identical to a heavy chain amino acid sequence shown in SEQ ID NO: 2, and further comprising an amino acid sequence that is at least 95% identical to a light chain amino acid sequence shown in SEQ ID NO: 4; and a chelating agent, wherein the antibody binds to human M-CSF, and the composition contains a concentration of antibody that is at least about 60 mg/ml, at least about 70 mg/ml, at least about 80 mg/ml, or at least about 90 mg/ml.

In one embodiment, the present invention encompasses a liquid pharmaceutical composition comprising at least one antibody comprising an amino acid sequence that is at least 95% identical to a heavy chain amino acid sequence shown in SEQ ID NO: 2, and further comprising an amino acid sequence that is at least 95% identical to a light chain amino acid sequence shown in SEQ ID NO: 4; and a chelating agent, wherein the antibody binds to human M-CSF, and the composition contains a concentration of antibody that is about 10 mg/ml.

In one embodiment, the present invention encompasses a liquid pharmaceutical composition comprising at least one antibody comprising an amino acid sequence that is at least 95% identical to a heavy chain amino acid sequence shown in SEQ ID NO: 2, and further comprising an amino acid sequence that is at least 95% identical to a light chain amino acid sequence shown in SEQ ID NO: 4; and a chelating agent, wherein the antibody binds to human M-CSF, and the composition contains a concentration of antibody that is about 20 mg/ml.

In one embodiment, the present invention encompasses a liquid pharmaceutical composition comprising at least one antibody comprising an amino acid sequence that is at least 95% identical to a heavy chain amino acid sequence shown in SEQ ID NO: 2, and further comprising an amino acid sequence that is at least 95% identical to a light chain amino acid sequence shown in SEQ ID NO: 4; and from about 0.01 millimolar to about 0.5 millimolar of chelating agent, wherein the antibody binds to human M-CSF, and the composition has an antibody concentration of from about 1.0 mg/ml to about 100 mg/ml.

In one embodiment, the present invention encompasses a liquid pharmaceutical composition comprising at least one antibody comprising an amino acid sequence that is at least 95% identical to a heavy chain amino acid sequence shown in SEQ ID NO: 2, and further comprising an amino acid sequence that is at least 95% identical to a light chain amino acid sequence shown in SEQ ID NO: 4; and from about 0.01 millimolar to about 0.5 millimolar of chelating agent, wherein the antibody binds to human M-CSF, and the composition has an antibody concentration of from about 1.0 mg/ml to about 100 mg/ml.

In one embodiment, the present invention encompasses a liquid pharmaceutical composition comprising at least one antibody comprising an amino acid sequence that is at least 95% identical to a heavy chain amino acid sequence shown in SEQ ID NO: 2, and further comprising an amino acid sequence that is at least 95% identical to a light chain amino acid sequence shown in SEQ ID NO: 4; and about 0.05 millimolar of chelating agent, wherein the antibody binds to human M-CSF, and the composition has an antibody concentration of from about 1.0 mg/ml to about 100 mg/ml.

In one embodiment, the present invention encompasses a liquid pharmaceutical composition comprising at least one antibody comprising an amino acid sequence that is at least 95% identical to a heavy chain amino acid sequence shown in SEQ ID NO: 2, and further comprising an amino acid sequence that is at least 95% identical to a light chain amino acid sequence shown in SEQ ID NO: 4; and from about 0.01 millimolar to about 0.5 millimolar of EDTA, wherein the antibody binds to human M-CSF, and the composition has an antibody concentration of from about 1.0 mg/ml to about 100 mg/ml.

In one embodiment, the present invention encompasses a liquid pharmaceutical composition comprising at least one antibody comprising an amino acid sequence that is at least 95% identical to a heavy chain amino acid sequence shown in SEQ ID NO: 2, and further comprising an amino acid sequence that is at least 95% identical to a light chain amino acid sequence shown in SEQ ID NO: 4; and from about 1.0 millimolar to about 100 millimolar of histidine, wherein the antibody binds to human M-CSF, and the composition has an antibody concentration of from about 1.0 mg/ml to about 100 mg/ml.

In one embodiment, the present invention encompasses a liquid pharmaceutical composition comprising at least one antibody comprising an amino acid sequence that is at least 95% identical to a heavy chain amino acid sequence shown in SEQ ID NO: 2, and further comprising an amino acid sequence that is at least 95% identical to a light chain amino acid sequence shown in SEQ ID NO: 4; from about 0.01 millimolar to about 0.5 millimolar of EDTA; and from about 1 millimolar to about 50 millimolar of histidine, wherein the antibody binds to human M-CSF, and the composition has an antibody concentration of from about 1.0 mg/ml to about 100 mg/ml.

In one embodiment, the present invention encompasses a liquid pharmaceutical composition comprising at least one antibody comprising an amino acid sequence that is at least 95% identical to a heavy chain amino acid sequence shown in SEQ ID NO: 2, and further comprising an amino acid sequence that is at least 95% identical to a light chain amino acid sequence shown in SEQ ID NO: 4; from about 0.01 millimolar to about 0.5 millimolar of EDTA; from about 1 millimolar to about 50 millimolar of histidine; and from about 200 millimolar to about 300 millimolar of mannitol, wherein the antibody binds to human M-CSF, and the composition has an antibody concentration of from about 1.0 mg/ml to about 100 mg/ml.

In one embodiment, the present invention encompasses a liquid pharmaceutical composition comprising at least one antibody comprising an amino acid sequence that is at least 95% identical to a heavy chain amino acid sequence shown in SEQ ID NO: 2, and further comprising an amino acid sequence that is at least 95% identical to a light chain amino acid sequence shown in SEQ ID NO: 4; from about 0.01 millimolar to about 0.5 millimolar of EDTA; from about 0.1 millimolar to about 50 millimolar of histidine; and from about 200 millimolar to about 300 millimolar of trehalose, wherein the antibody binds to human M-CSF, and the composition has an antibody concentration of from about 1.0 mg/ml to about 100 mg/ml.

In one embodiment, the liquid pharmaceutical composition comprises from about 0.01 mg/ml to about 200 mg/ml of at least one monoclonal anti-M-CSF antibody; and from about 1 micromolar to about 50 millimolar of a chelating agent.

In another embodiment, the liquid pharmaceutical composition comprises from about 0.1 mg/ml to about 100 mg/ml of at least one monoclonal anti-M-CSF antibody; and from about 1 micromolar to about 5.0 millimolar of a chelating agent.

In another embodiment, the liquid pharmaceutical composition comprises from about 0.1 mg/ml to about 100 mg/ml of at least one monoclonal anti-M-CSF antibody; and about 0.05 millimolar of a chelating agent.

In another embodiment, the liquid pharmaceutical composition comprises from about 0.1 mg/ml to about 100 mg/ml of at least one monoclonal anti-M-CSF antibody; and from about 1 micromolar to about 50 millimolar of EDTA.

In another embodiment, the liquid pharmaceutical composition comprises from about 0.1 mg/ml to about 100 mg/ml of at least one monoclonal anti-M-CSF antibody; and from about 1. micromolar to about 10.0 millimolar of EDTA.

In another embodiment, the liquid pharmaceutical composition comprises from about 0.1 mg/ml to about 100 mg/ml of at least one monoclonal anti-M-CSF antibody; and from about 0.01 millimolar to about 1.0 millimolar of EDTA.

In another embodiment, the liquid pharmaceutical composition comprises from about 0.1 mg/ml to about 100 mg/ml of at least one monoclonal anti-M-CSF antibody; and about 0.05. millimolar of EDTA.

In another embodiment, the liquid pharmaceutical composition comprises from about 0.1 mg/ml to about 100 mg/ml of at least one monoclonal anti-M-CSF antibody; and from about 30 micromolar to about 5.0 millimolar of DTPA.

In another embodiment, the liquid pharmaceutical composition comprises from about 0.1 mg/ml to about 100 mg/ml of at least one monoclonal anti-M-CSF antibody; and from about 1 micromolar to about 5.0 millimolar of deferoxamine.

In one embodiment, the liquid pharmaceutical composition comprises from about 0.01 mg/ml to about 200 mg/ml of at least one monoclonal anti-M-CSF antibody; and from about 1 mM to about 100 mM of histidine.

In another embodiment, the liquid pharmaceutical composition comprises from about 0.1 mg/ml to about 200 mg/ml of at least one monoclonal anti-M-CSF antibody; from about 30 micromolar to about 5.0 millimolar of a chelating agent; and from about 1 mM to about 100 mM of histidine.

In another embodiment, the liquid pharmaceutical composition comprises from about 0.1 mg/ml to about 200 mg/ml of at least one monoclonal anti-M-CSF antibody; from about 1 micromolar to about 5.0 millimolar of a chelating agent; and from about 10 millimolar to about 400 millimolar of trehalose.

In another embodiment, the liquid pharmaceutical composition comprises from about 0.1 mg/ml to about 200 mg/ml of at least one monoclonal anti-M-CSF antibody; from about 1 micromolar to about 5.0 millimolar of a chelating agent; and from about 1 millimolar to about 400 millimolar of mannitol.

In another embodiment, the liquid pharmaceutical composition comprises from about 0.1 mg/ml to about 200 mg/ml of at least one monoclonal anti-M-CSF antibody; from about 1 micromolar to about 5.0 millimolar of a chelating agent; from about 10 millimolar to about 400 millimolar of trehalose; and from about 1 mM to about 100 mM of histidine.

In another embodiment, the liquid pharmaceutical composition comprises from about 0.1 mg/ml to about 200 mg/ml of at least one monoclonal anti-M-CSF antibody; from about 1 micromolar to about 5.0 millimolar of a chelating agent; from about 10 millimolar to about 400 millimolar of trehalose; from about 1 mM to about 100 mM of histidine; and from about 0.01 mg/ml to about 10 mg/ml of polysorbate 80.

In another embodiment, the liquid pharmaceutical composition comprises from about 0.1 mg/ml to about 200 mg/ml of at least one monoclonal anti-M-CSF antibody; from about 1 micromolar to about 5.0 millimolar of a chelating agent; from about 1 millimolar to about 400 millimolar of mannitol; from about 1 mM to about 100 mM of histidine; and from about 0.01 mg/ml to about 10 mg/ml of polysorbate 80.

In another embodiment, the liquid pharmaceutical composition comprises from about 0.1 mg/ml to about 200 mg/ml of at least one monoclonal anti-M-CSF antibody; from about 1 micromolar to about 5.0 millimolar of EDTA; from about 10 millimolar to about 400 millimolar of a tonicity agent; from about 1 mM to about 100 mM of a buffer; and from about 0.01 mg/ml to about 10 mg/ml of polysorbate 80.

In another embodiment, the liquid pharmaceutical composition comprises from about 0.1 mg/ml to about 200 mg/ml of at least one monoclonal anti-M-CSF antibody; from about 1 micromolar to about 5.0 millimolar of EDTA; from about 10 millimolar to about 400 millimolar of a tonicity agent; from about 1 mM to about 100 mM of histidine; and from about 0.01 mg/ml to about 10 mg/ml of polysorbate 80.

In another embodiment, the liquid pharmaceutical composition comprises from about 0.1 mg/ml to about 200 mg/ml of a monoclonal anti-M-CSF antibody; from about 1 micromolar to about 5.0 millimolar of EDTA; from about 10 millimolar to about 400 millimolar of trehalose; from about 1 mM to about 100 mM of histidine; and from about 0.01 mg/ml to about 10 mg/ml of polysorbate 80.

In another embodiment, the liquid pharmaceutical composition comprises from about 0.1 mg/ml to about 200 mg/ml of at least one monoclonal anti-M-CSF antibody; from about 1 micromolar to about 5.0 millimolar of EDTA; from about 10 millimolar to about 400 millimolar of mannitol; from about 1 mM to about 100 mM of histidine; and from about 0.01 mg/ml to about 10 mg/ml of polysorbate 80.

In certain aspects of the present invention, the liquid anti-M-CSF antibody compositions comprise from about 0.1 mg/ml to about 200 mg/ml of a monoclonal anti-M-CSF antibody; from about 1 mM to about 100 mM of histidine; from about 0.005 millimolar to about 10 millimolar of polysorbate 80; from about 1 micromolar to about 5.0 millimolar of EDTA; and from about 10 millimolar to about 400 millimolar of trehalose.

In certain aspects of the present invention, the liquid anti-M-CSF antibody compositions comprise from about 0.1 mg/ml to about 200 mg/ml of at least one monoclonal anti-M-CSF antibody; from about 1 mM to about 100 mM of histidine; from about 0.01 mg/ml to about 10 mg/ml of polysorbate 80; from about 1 micromolar to about 5.0 millimolar of EDTA; and from about 10 millimolar to about 400 millimolar of mannitol.

In other aspects of the present invention, the liquid anti-M-CSF antibody compositions comprise from about 1.0 mg/ml to about 100 mg/ml of at least one monoclonal anti-M-CSF antibody; from about 10 mM to about 50 mM of histidine; from about 0.05 mg/ml to about 1 mg/ml of polysorbate 80; from about 0.01 millimolar to about 1 millimolar of EDTA; and from about 100 millimolar to about 300 millimolar of trehalose.

In other aspects of the present invention, the liquid anti-M-CSF antibody compositions comprise from about 1.0 mg/ml to about 100 mg/ml of at least one monoclonal anti-M-CSF antibody; from about 10 mM to about 50 mM of histidine; from about 0.05 mg/ml to about 1 mg/ml of polysorbate 80; from about 0.01 millimolar to about 1 millimolar of EDTA; and from about 100 millimolar to about 300 millimolar of mannitol.

In other aspects of the present invention, the liquid anti-M-CSF antibody compositions comprise from about 10 mg/ml to about 50 mg/ml of at least one monoclonal anti-M-CSF antibody; from about 10 mM to about 30 mM of histidine; from about 0.05 millimolar to about 0.2 mg/ml; from about 0.01 millimolar to about 1 millimolar of EDTA; and from about 200 millimolar to about 250 millimolar of trehalose.

In other aspects of the present invention, the liquid anti-M-CSF antibody compositions comprise about 20 mg/ml of at least one monoclonal anti-M-CSF antibody; about 20 mM of histidine; about 0.15 millimolar of polysorbate 80; about 0.05 millimolar of EDTA; and about 222 millimolar of trehalose.

In other aspects of the present invention, the composition comprises at least one monoclonal anti-M-CSF antibody; histidine; polysorbate 80; EDTA; and mannitol.

In still other aspects of the present invention, the composition comprises monoclonal anti-M-CSF antibody; histidine; polysorbate 80; EDTA; and trehalose.

In another embodiment, the invention is directed to a liquid pharmaceutical composition comprising at least one anti-M-CSF antibody and DTPA.

In another embodiment, the invention is directed to a composition comprising at least one anti-M-CSF antibody, a chelating agent, and a buffer. In another embodiment, the invention is directed to a composition comprising at least one anti-M-CSF antibody, a chelating agent, and histidine. In another embodiment, the invention is directed to a composition comprising at least one anti-M-CSF antibody, EDTA, and histidine. In another embodiment, the invention is directed to a composition comprising at least one anti-M-CSF antibody, DTPA, and histidine.

In another embodiment, the invention is directed to a composition comprising at least one anti-M-CSF antibody, a chelating agent, and a buffer. In another embodiment, the invention is directed to a composition comprising at least one anti-M-CSF antibody, a chelating agent, and acetate.

In another embodiment, the invention is directed to a composition comprising at least one anti-M-CSF antibody, a chelating agent, and a tonicity agent. In another embodiment, the invention is directed to a liquid pharmaceutical composition comprising at least one anti-M-CSF antibody, a chelating agent, and mannitol. In another embodiment, the invention is directed to a composition comprising at least one anti-M-CSF antibody, a chelating agent, and trehalose. In another embodiment, the invention is directed to a composition comprising at least one anti-M-CSF antibody, EDTA, and trehalose. In another embodiment, the invention is directed to a composition comprising at least one anti-M-CSF antibody, EDTA, and mannitol. In another embodiment, the invention is directed to a composition comprising at least one anti-M-CSF antibody, EDTA, and sucrose. In another embodiment, the invention is directed to a composition comprising at least one anti-M-CSF antibody, DTPA, and trehalose. In another embodiment, the invention is directed to a composition comprising at least one anti-M-CSF antibody, DTPA, and mannitol. In another embodiment, the invention is directed to a composition comprising at least one anti-M-CSF antibody, a chelating agent, and a surfactant. In another embodiment, the invention is directed to a composition comprising at least one anti-M-CSF antibody, EDTA, and a surfactant. In another embodiment, the invention is directed to a composition comprising at least one anti-M-CSF antibody, DTPA, and a surfactant. In another embodiment, the invention is directed to a composition comprising at least one anti-M-CSF antibody, a chelating agent selected from the group consisting of EDTA and DTPA, and polysorbate 80. In another embodiment, the invention is directed to a composition comprising at least one anti-M-CSF antibody, a buffer, and a surfactant. In another embodiment, the invention is directed to a composition comprising at least one anti-M-CSF antibody, histidine, and a surfactant. In another embodiment, the invention is directed to a composition comprising at least one anti-M-CSF antibody, histidine, and polysorbate 80. In another embodiment, the invention is directed to a composition comprising at least one anti-M-CSF antibody, a chelating agent, a buffer, and a surfactant. In another embodiment, the invention is directed to a composition comprising at least one anti-M-CSF antibody, a chelating agent, a buffer, and a tonicity agent. In another embodiment, the invention is directed to a composition comprising at least one anti-M-CSF antibody, a chelating agent, a buffer, a surfactant, and a tonicity agent. In another embodiment, the invention is directed to a composition comprising at least one anti-M-CSF antibody and histidine. In another embodiment, the invention is directed to a liquid pharmaceutical composition comprising at least one anti-M-CSF antibody and histidine.

In certain embodiments, the anti-M-CSF antibody compositions comprise at least one anti-M-CSF antibody, histidine, and EDTA. In other embodiments, the anti-M-CSF antibody compositions comprise at least one anti-M-CSF antibody, histidine, and polysorbate 80. In other embodiments, the anti-M-CSF antibody compositions comprise at least one anti-M-CSF antibody, histidine, and mannitol. In other embodiments, the anti-M-CSF antibody compositions comprise at least one anti-M-CSF antibody, histidine, and trehalose. In other embodiments, the anti-M-CSF antibody compositions comprise at least one anti-M-CSF antibody, histidine, and sucrose. In other embodiments, the anti-M-CSF antibody compositions comprise at least one anti-M-CSF antibody, histidine, polysorbate 80, and EDTA. In other embodiments, the anti-M-CSF antibody compositions comprise at least one anti-M-CSF antibody, histidine, polysorbate. 80, mannitol, and EDTA. In other embodiments, the anti-M-CSF antibody compositions comprise at least one anti-M-CSF antibody, histidine, polysorbate 80, trehalose, and EDTA. In other embodiments, the anti-M-CSF antibody compositions comprise at least one anti-M-CSF antibody, histidine, polysorbate 80, sucrose, and EDTA.
In still further embodiments, the anti-M-CSF antibody compositions comprise at least one anti-M-CSF antibody, acetate, and EDTA. In other embodiments, the M-CSF antibody compositions comprise at least one anti-M-CSF antibody, polysorbate 80, and EDTA. In other embodiments, the anti-M-CSF antibody compositions comprise at least one anti-M-CSF antibody, mannitol, and EDTA. In other embodiments, the anti-M-CSF antibody compositions comprise at least one anti-M-CSF antibody, polysorbate 80, mannitol, and EDTA. In other embodiments, the anti-M-CSF antibody compositions comprise at least one anti-M-CSF antibody, acetate, polysorbate 80, mannitol, and EDTA. In other embodiments, the anti-M-CSF antibody compositions comprise at least one anti-M-CSF antibody, sucrose, and EDTA. In other embodiments, the anti-M-CSF antibody compositions comprise at least one anti-M-CSF antibody, polysorbate 80, sucrose, and EDTA. In other embodiments, the anti-M-CSF antibody compositions comprise at least one anti-M-CSF antibody, acetate, polysorbate 80, sucrose, and EDTA. In other embodiments, the anti-M-CSF antibody compositions comprise at least one anti-M-CSF antibody, trehalose, and EDTA. In other embodiments, the anti-M-CSF antibody compositions comprise at least one anti-M-CSF antibody, polysorbate 80, trehalose, and EDTA. In other embodiments, the anti-M-CSF antibody compositions comprise at least one anti-M-CSF antibody, acetate, polysorbate 80, trehalose, and EDTA.

In certain aspects of the present invention, the anti-M-CSF antibody compositions comprise from about 1 mg/ml to about 100 mg/ml of at least one monoclonal anti-M-CSF antibody; from about 1 mM to about 50 mM of histidine; from about 0.01 mg/ml to about 5 mg/ml of polysorbate 80; from about 0.001 mg/ml to about 0.5 mg/ml of EDTA; and from about 10 mg/ml to about 200 mg/ml of mannitol.

In certain aspects of the present invention, the anti-M-CSF antibody compositions comprise from about 1 mg/ml to about 100 mg/ml of at least one monoclonal anti-M-CSF antibody; from about 1 mM to about 50 mM of histidine; from about 0.01 mg/ml to about 5 mg/ml of polysorbate 80; from about 0.001 mg/ml to about 0.5 mg/ml of EDTA; and from about 10 mg/ml to about 200 mg/ml of trehalose.

In certain aspects of the present invention, the anti-M-CSF antibody compositions comprise from about 1 mg/ml to about 100 mg/ml of at least one monoclonal anti-M-CSF antibody; from about 1 mM to about 50 mM of histidine; from about 0.01 mg/ml to about 5 mg/ml of polysorbate 80; from about 0.001 mg/ml to about 0.5 mg/ml of EDTA; and from about 10 mg/ml to about 200 mg/ml of sucrose.

In other aspects of the present invention, the anti-M-CSF antibody compositions comprise about 10 mg/ml of antibody; about 10 mM of histidine; about 0.2 mg/ml of polysorbate 80; about 0.02 mg/ml of EDTA; and about 45 mg/ml of mannitol.

In other aspects of the present invention, the anti-M-CSF antibody compositions comprise about 75 mg/ml of antibody; about 20 mM of histidine; about 0.5 mg/ml of polysorbate 80; about 0.05 mg/ml of EDTA; and about 90 mg/ml of sucrose.

In one embodiment, the liquid pharmaceutical composition comprises from about 0.01 mg/ml to about 200 mg/ml of at least one monoclonal anti-M-CSF antibody; and from about 0.3 micromolar to about 50 millimolar of a chelating agent.

In another embodiment, the liquid pharmaceutical composition comprises from about 0.1 mg/ml to about 100 mg/ml of at least one monoclonal anti-M-CSF antibody; and from about 1 micromolar to about 1 millimolar of a chelating agent.

In another embodiment, the liquid pharmaceutical composition comprises from about 0.1 mg/ml to about 100 mg/ml of at least one monoclonal anti-M-CSF antibody; and about 0.02 millimolar of a chelating agent.

In another embodiment, the liquid pharmaceutical composition comprises from about 0.1 mg/ml to about 100 mg/ml of at least one monoclonal anti-M-CSF antibody; and about 0.05 millimolar of a chelating agent.

In another embodiment, the liquid pharmaceutical composition comprises from about 0.1 mg/ml to about 100 mg/ml of at least one monoclonal anti-M-CSF antibody; and from about 1 micromolar to about 50 millimolar of EDTA.

In another embodiment, the liquid pharmaceutical composition comprises from about 0.1 mg/ml to about 100 mg/ml of monoclonal anti-M-CSF antibody; and from about 1 micromolar to about 10.0 millimolar of EDTA.

In another embodiment, the liquid pharmaceutical composition comprises from about 0.1 mg/ml to about 100 mg/ml of at least one monoclonal anti-M-CSF antibody; and from about 0.01 millimolar to about 1.0 millimolar of EDTA.

In another embodiment, the composition comprises from about 0.1 mg/ml to about 100 mg/ml of at least one monoclonal anti-M-CSF antibody; and about 0.02 millimolar of EDTA.

In another embodiment, the composition comprises from about 0.1 mg/ml to about 100 mg/ml of at least one monoclonal anti-M-CSF antibody; and about 0.05 millimolar of EDTA.

In another embodiment, the liquid pharmaceutical composition comprises from about 0.1 mg/ml to about 100 mg/ml of at least one monoclonal anti-M-CSF antibody; and from about 30 micromolar to about 5.0 millimolar of DTPA.

In another embodiment, the liquid pharmaceutical composition comprises from about 0.1 mg/ml to about 100 mg/ml of at least one monoclonal anti-M-CSF antibody; and from about 1 micromolar to about 5.0 millimolar of deferoxamine.

In one embodiment, the liquid pharmaceutical composition comprises from about 0.01 mg/ml to about 200 mg/ml of at least one monoclonal anti-M-CSF antibody; and from about 1 mM to about 100 mM of histidine.

In another embodiment, the liquid pharmaceutical composition comprises from about 0.1 mg/ml to about 200 mg/ml of at least one monoclonal anti-M-CSF antibody; from about 1 micromolar to about 5.0 millimolar of a chelating agent; and from about 1 mM to about 100 mM of histidine.

In another embodiment, the liquid pharmaceutical composition comprises from about 0.1 mg/ml to about 200 mg/ml of at least one monoclonal anti-M-CSF antibody; from about 1 micromolar to about 1.0 millimolar of a chelating agent; and from about 10 millimolar to about 400 millimolar of trehalose.

In another embodiment, the liquid pharmaceutical composition comprises from about 0.1 mg/ml to about 200 mg/ml of at least one monoclonal anti-M-CSF antibody; from about 1 micromolar to about 1.0 millimolar of a chelating agent; and from about 1 millimolar to about 400 millimolar of mannitol.

In another embodiment, the liquid pharmaceutical composition comprises from about 0.1 mg/ml to about 200 mg/ml of at least one monoclonal anti-M-CSF antibody; from about 1 micromolar to about 1.0 millimolar of a chelating agent; from about 10 millimolar to about 400 millimolar of trehalose; and from about 1 mM to about 100 mM of histidine.

In another embodiment, the liquid pharmaceutical composition comprises from about 0.1 mg/ml to about 200 mg/ml of at least one monoclonal anti-M-CSF antibody; from about 1 micromolar to about. 1.0 millimolar of a chelating agent; from about 10 millimolar to about 400 millimolar of trehalose; from about 1 mM to about 100 mM of histidine; and from about 0.01 mg/ml to about 10 mg/ml of a surfactant.

In another embodiment, the composition comprises from about 0.1 mg/ml to about 200 mg/ml of at least one monoclonal anti-M-CSF antibody; from about 1 micromolar to about 1.0 millimolar of a chelating agent; from about 1 millimolar to about 400 millimolar of mannitol; from about 1 mM to about 100 mM of histidine; and from about 0.01 mg/ml to about 10 mg/ml of a surfactant.

In another embodiment, the liquid pharmaceutical composition comprises from about 0.1 mg/ml to about 200 mg/ml of at least one monoclonal anti-M-CSF antibody; from about 1 micromolar to about 1.0 millimolar of EDTA; from about 10 millimolar to about 400 millimolar of a tonicity agent; from about 1 mM to about 100 mM of a buffer; and from about 0.01 mg/ml to about 10 mg/ml of a surfactant.

In another embodiment, the composition comprises from about 0.1 mg/ml to about 200 mg/ml of at least one monoclonal anti-M-CSF antibody; from about 1 micromolar to about 1.0 millimolar of EDTA; from about 10 millimolar to about 400 millimolar of a tonicity agent; from about 1 mM to about 100 mM of histidine; and from about 0.01 mg/ml to about 10 mg/ml of a surfactant.

In another embodiment, the liquid pharmaceutical composition comprises from about 0.1 mg/ml to about 200 mg/ml of at least one monoclonal anti-M-CSF antibody; from about 1 micromolar to about 1.0 millimolar of EDTA; from about 10 millimolar to about 400 millimolar of trehalose; from about 1 mM to about 100 mM of histidine; and from about 0.1 mg/ml to about 1 mg/ml of a surfactant.

In another embodiment, the liquid pharmaceutical composition comprises from about 0.1 mg/ml to about 200 mg/ml of at least one monoclonal anti-M-CSF antibody; from about 1 micromolar to about 1.0 millimolar of EDTA; from about 10 millimolar to about 400 millimolar of mannitol; from about 1 mM to about 100 mM of histidine; and from about from about 0.1 mg/ml to about 1 mg/ml of a surfactant.

In other aspects of the present invention, the liquid anti-M-CSF antibody compositions comprise from about 0.1 mg/ml to about 200 mg/ml of at least one monoclonal anti-M-CSF antibody; from about 1 mM to about 100 mM of histidine; from about 0.005 millimolar to about 10 millimolar of polysorbate 80; from about 1 micromolar to about 1.0 millimolar of EDTA; and from about 10 millimolar to about 400 millimolar of trehalose.

In other aspects of the present invention, the liquid anti-M-CSF antibody compositions comprise from about 0.1 mg/ml to about 200 mg/ml of at least one monoclonal anti-M-CSF antibody; from about 1 mM to about 100 mM of histidine; from about 0.005 millimolar to about 10 millimolar of polysorbate 80; from about 1 micromolar to about 1.0 millimolar of EDTA; and from about 10 millimolar to about 400 millimolar of mannitol.

In other aspects of the present invention, the liquid anti-M-CSF antibody compositions comprise from about 1:0 mg/ml to about 100 mg/ml of at least one monoclonal anti-M-CSF antibody; from about 10 mM to about 50 mM of histidine; from about 0.01 millimolar to about 1.0 millimolar of polysorbate 80; from about 1 micromolar to about 1.0 millimolar of EDTA; and from about 100 millimolar to about 300 millimolar of trehalose.

In other aspects of the present invention, the liquid anti-M-CSF antibody compositions comprise from about 1.0 mg/ml to about 100 mg/ml of at least one monoclonal anti-M-CSF antibody; from about 10 mM to about 50 mM of histidine; from about 0.01 millimolar to about 1.0 millimolar of polysorbate 80; from about 1 micromolar to about 1.0 millimolar of EDTA; and from about 100 millimolar to about 300 millimolar of mannitol.

In other aspects of the present invention, the liquid anti-M-CSF antibody compositions comprise from about 10 mg/ml to about 50 mg/ml of at least one monoclonal anti-M-CSF antibody; from about 10 mM to about 30 mM of histidine; from about 0.05 millimolar to about 0.5 millimolar of polysorbate 80; from about 0.1 millimolar to about 1 millimolar of EDTA; and from about 200 millimolar to about 250 millimolar of trehalose.

In other aspects of the present invention, the liquid anti-M-CSF antibody compositions comprise from about 50 mg/ml to about 100 mg/ml of at least one monoclonal anti-M-CSF antibody; from about 10 mM to about 30 mM of histidine; from about 0.05 millimolar to about 1 millimolar of polysorbate 80; from about 0.01 millimolar to about 1 millimolar of EDTA; and from about 100 millimolar to about 400 millimolar of sucrose.

In other aspects of the present invention, the anti-M-CSF liquid antibody compositions comprise about 10 mg/ml of at least one monoclonal anti-M-CSF antibody; about 10 mM of histidine; about 0.2 mg/ml of polysorbate 80; about 0.02 mg/ml of EDTA; and about 45 mg/ml of mannitol.

In other aspects of the present invention, the anti-M-CSF antibody compositions comprise about 75 mg/ml of at least one monoclonal anti-M-CSF antibody; about 20 mM of histidine; about 0.5 mg/ml of polysorbate 80; about 0.05 millimolar of EDTA; and about 90 mg/ml of sucrose.

In another embodiment, the invention is directed to a liquid pharmaceutical composition comprising at least one anti-M-CSF antibody and a chelating agent, wherein the molar concentration of the antibody ranges from about 0.01 millimolar to about 2 millimolar and the molar concentration of the chelating agent ranges from about 0.001 millimolar to about 5 millimolar, and wherein the molar ratio of antibody to chelating agent ranges from about 0.002 to about 2000; from about 0.01 to about 500; from about 0.05 to about 100; from about 0.1 to about 50; from about 0.5 to about 10; from about 1 to about 5; from about 1 to about 3; or about 1.6.

In another embodiment, the invention is directed to a composition comprising at least one anti-M-CSF antibody and a chelating agent, wherein the molar concentration of the antibody ranges from about 0.01 millimolar to about 2 millimolar and the molar concentration of the chelating agent ranges from about 0.001 millimolar to about 5 millimolar, and wherein the molar ratio of antibody to chelating agent ranges from about 0.002 to about 2000; from about 0.01 to about 500; from about 0.05 to about 100; from about 0.1 to about 50; from about 0.5 to about 10; from about 1 to about 5; or about 3.8.

In another embodiment, the invention is directed to a stable liquid pharmaceutical composition comprising at least one monoclonal anti-M-CSF antibody 8.10.3F and a chelating agent, wherein the molar concentration of the antibody ranges from about 0.01 millimolar to about 2 millimolar and the molar concentration of the chelating agent ranges from about 0.001 millimolar to about 5 millimolar, and wherein the molar ratio of antibody to chelating agent ranges from about 0.002 to about 2000; from about 0.01 to about 500; from about 0.05 to about 100; from about 0.1 to about 50; from about 0.5 to about 10; from about 1 to about 5; from about 1 to about 3; or about 1.6.

In another embodiment, the invention is directed to a stable composition comprising at least one monoclonal anti-M-CSF antibody 8.10.3F and a chelating agent, wherein the molar concentration of the antibody ranges from about 0.01 millimolar to about 2 millimolar and the molar concentration of the chelating agent ranges from about 0.001 millimolar to about 5 millimolar, and wherein the molar ratio of antibody to chelating agent ranges from about 0.002 to about 2000; from about 0.01 to about 500; from about 0.05 to about 100; from about 0.1 to about 50; from about 0.5 to about 10; from about 1 to about 5; or about 3.8.

In another embodiment, the invention is directed to a stable composition comprising at least one monoclonal anti-M-CSF antibody 8.10.3F, a chelating agent, and histidine; wherein the molar concentration of the antibody ranges from about 0.01 millimolar to about 2 millimolar and the molar concentration of histidine ranges from about 1 millimolar to about 100 millimolar and the molar concentration of the chelating agent ranges from about 0.001 millimolar to about 5 millimolar, and wherein the molar ratio of antibody to chelating agent ranges from about 0.002 to about 2000; from about 0.01 to about 500; from about 0.05 to about 100; from about 0.1 to about 50; from about 0.5 to about 10; from about 1 to about 5; or about 3.8.

In another embodiment, the invention is directed to a stable composition comprising at least one monoclonal anti-M-CSF antibody 8.10.3F, a chelating agent, and histidine; wherein the molar concentration of the antibody ranges from about 0.01 millimolar to about 2 millimolar and the molar concentration of histidine ranges from about 5 millimolar to about 30 millimolar and the molar concentration of the chelating agent ranges from about 0.001 millimolar to about 5 millimolar, and wherein the molar ratio of antibody to chelating agent ranges from about 0.002 to about 2000; from about 0.01 to about 500; from about 0.05 to about 100; from about 0.1 to about. 50; from about 0.5 to about 10; from about 1 to about 5; or about 3.8.

In another embodiment, the invention is directed to a stable composition comprising at least one monoclonal anti-M-CSF antibody 8.10.3F, a chelating agent, and histidine; wherein the molar concentration of the antibody ranges from about 0.01 millimolar to about 2 millimolar and the molar concentration of histidine is about 20 millimolar and the molar concentration of the chelating agent ranges from about 0.001 millimolar to about 5 millimolar, and wherein the molar ratio of antibody to chelating agent ranges from about 0.002 to about 2000; from about 0.01 to about 500; from about 0.05 to about 100; from about 0.1 to about 50; from about 0.5 to about 10; from about 1 to about 5; or about 3.8.

### Methods of Producing Anti-M-CSF Antibodies and Antibody Producing Cell Lines:

Antibodies in accordance with the invention can be prepared through the utilization of a transgenic mouse that has a substantial portion of the human antibody producing genome inserted, but that is rendered deficient in the production of endogenous, murine, antibodies. Such mice, then, are capable of producing human immunoglobulin molecules and antibodies and are deficient in the production of murine immunoglobulin molecules and antibodies. Technologies utilized for achieving the same are discussed below.

It is possible to produce transgenic animals (*e.g*., mice) that are capable, upon immunization, of producing a full repertoire of human antibodies in the absence of endogenous immunoglobulin production. In particular, however, one embodiment of transgenic production of mice and antibodies therefrom is disclosed in U.S. Published Application No. 20050059113 to Bedian, et al. Through use of such technology, antibodies that bind to M-CSF and hybridomas producing such antibodies can be prepared.

Human antibodies avoid potential problems associated with antibodies that possess murine or rat variable and/or constant regions. The presence of such murine or rat derived proteins can lead to the rapid clearance of the antibodies or can lead to the generation of an immune response against the antibody by a subject that receives administration of such antibodies.

For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen (*e.g*., M-CSF) challenge. See, *e.g*., Jakobovits et al, Proc. Natl. Acad. Sci. USA, 90:2551 (1993); Jakobovits et al., Nature, 362:255-258 (1993); Bruggermann et al., Year in Immuno., 7:33 (1993); and Duchosal et al., Nature 355:258 (1992). Human antibodies can also be derived from phage-display libraries (Hoogenboom et al., J. Mol. Biol., 227:381 (1991); Marks et al., J. MoL Biol., 222:581-597 (1991); Vaughan et al., Nature Biotech 14:309 (1996)).

In some embodiments, human antibodies are produced by immunizing a non-human animal comprising in its genome some or all of human immunoglobulin heavy chain and light chain loci with an M-CSF antigen. In a preferred embodiment, the non-human animal is a XENOMOUSE™ animal (Abgenix Inc., Fremont, CA). Another non-human animal that may be used is a transgenic mouse produced by Medarex (Medarex, Inc., Princeton, NJ).

In some embodiments, human anti-M-CSF antibodies can be produced by immunizing a non-human transgenic animal, *e.g*., XENOMOUSE™ mice, whose genome comprises human immunoglobulin genes so that the recombinant mouse produces human antibodies. XENOMOUSE™ mice are engineered mouse strains that comprise large fragments of human immunoglobulin heavy chain and light chain loci and are deficient in mouse antibody production. XENOMOUSE™ mice produce an adult-like human repertoire of fully human antibodies and generate antigen-specific human antibodies. In some embodiments, the XENOMOUSE™ mice contain approximately 80% of the human antibody V gene repertoire through introduction of megabase sized, germline configuration yeast artificial chromosome (YAC) fragments of the human heavy chain loci and kappa light chain loci. In other embodiments, XENOMOUSE™ mice further contain approximately all of the lambda light chain locus. See, *e.g*., Green et al., Nature Genetics 7:13-21 (1994) and U.S. Patents 5,916,771, 5,939,598, 5,985,615, 5,998,209, 6,075,181, 6,091,001, 6,114,598, 6,130,364, 6,162,963 and 6,150,584. See also WO 91/10741, WO 94/02602, WO 96/34096, WO 96/33735, WO 98/16654, WO 98/24893, WO 98/50433, WO 99/45031, WO 99/53049, WO 00/09560, and WO 00/037504.

In some embodiments, the non-human animal comprising human immunoglobulin genes are animals that have a human immunoglobulin "minilocus". In the minilocus approach, an exogenous lg locus is mimicked through the inclusion of individual genes from the lg locus. Thus, one or more V_{H} genes, one or more D_{H} genes, one or more J_{H} genes, a mu constant domain, and a second constant domain (preferably a gamma constant domain) are formed into a construct for insertion into an animal. This approach is described, *inter alia,* in U.S. Patent Nos. 5,545,807, 5,545,806, 5,569,825, 5,625,126, 5,633,425, 5,661,016, 5,770,429, 5,789,650, 5,814,318, 5,591,669, 5,612,205, 5,721,367, 5,789,215, and 5,643,763.

Therefore, in some embodiments, human antibodies can be produced by immunizing a non-human animal comprising in its genome some or all of human immunoglobulin heavy chain and light chain loci with an M-CSF antigen.

In some embodiments, the M-CSF antigen is isolated and/or purified M-CSF. In a preferred embodiment, the M-CSF antigen is human M-CSF. In some embodiments, the M-CSF antigen is a fragment of M-CSF. In some embodiments, the M-CSF fragment comprises at least one epitope of M-CSF. In other embodiments, the M-CSF antigen is a cell that expresses or overexpresses M-CSF or an immunogenic fragment thereof on its surface. In still other embodiments, the M-CSF antigen is an M-CSF fusion protein. M-CSF can be purified from natural sources using known techniques. In addition, recombinant M-CSF protein is commercially available.

In a preferred embodiment, the non-human animal is a XENOMOUSE™ animal (Abgenix Inc., Fremont, CA). Another non-human animal that may be used is a transgenic mouse produced by Medarex (Medarex, Inc., Princeton, NJ).

Immunization of animals can be by any method known in the art. See, *e.g*., Harlow and Lane, Antibodies: A Laboratory Manual, New York: Cold Spring Harbor Press, 1990. Methods for immunizing non-human animals such as mice, rats, sheep, goats, pigs, cattle and horses are well known in the art. See, *e.g*., Harlow and Lane, *supra,* and U.S. Patent 5,994,619. In a preferred embodiment, the M-CSF antigen is administered with an adjuvant to stimulate the immune response. Exemplary adjuvants include complete or incomplete Freund's adjuvant, RIBI (muramyl dipeptides) or ISCOM (immunostimulating complexes). Such adjuvants may protect the polypeptide from rapid dispersal by sequestering it in a local deposit, or they may contain substances that stimulate the host to secrete factors that are chemotactic for macrophages and other components of the immune system. Preferably, if a polypeptide is being administered, the immunization schedule can involve two or more administrations of the polypeptide, spread out over several weeks.

After immunization of an animal with an M-CSF antigen, antibodies and/or antibody-producing cells can be obtained from the animal. In some embodiments, anti-M-CSF antibody-containing serum is obtained from the animal by bleeding or sacrificing the animal. The serum may be used as it is obtained from the animal, an immunoglobulin fraction may be obtained from the serum, or the anti-M-CSF antibodies may be purified from the serum.

In some embodiments, antibody-producing immortalized cell lines are prepared from cells isolated from the immunized animal. After immunization, the animal is sacrificed and lymph node and/or splenic B cells are immortalized. Methods of immortalizing cells include, but are not limited to, transfecting them with oncogenes, infecting them with an oncogenic virus, cultivating them under conditions that select for immortalized cells, subjecting them to carcinogenic or mutating compounds, fusing them with an immortalized cell, *e.g*., a myeloma cell, and inactivating a tumor suppressor gene. See, *e.g*., Harlow and Lane, *supra.* In a preferred embodiment, the immunized animal is a non-human animal that expresses human immunoglobulin genes and the splenic B cells are fused to a myeloma cell line from the same species as the non-human animal. In a more preferred embodiment, the immunized animal is a XENOMOUSE™ animal and the myeloma cell line is a non-secretory mouse myeloma. In an even more preferred embodiment, the myeloma cell line is P3-X63-AG8-653. If fusion with myeloma cells is used, the myeloma cells preferably do not secrete immunoglobulin polypeptides (a non-secretory cell line). Immortalized cells are screened using M-CSF, a portion thereof, or a cell expressing M-CSF. In a preferred embodiment, the initial screening is performed using an enzyme-linked immunoassay (ELISA) or a radioimmunoassay. An example of ELISA screening is provided in WO 00/37504.

Anti-M-CSF antibody-producing cells, *e.g*., hybridoma, are selected, cloned and further screened for desirable characteristics, including robust growth, high antibody production and desirable antibody characteristics, as discussed further below. Hybridomas can be expanded *in vivo* in syngeneic animals, in animals that lack an immune system, *e.g*., nude mice, or in cell culture *in vitro.* Methods of selecting, cloning and expanding hybridomas are well known to those of ordinary skill in the art.

As will be appreciated, antibodies in accordance with the present invention can be recombinantly expressed in cell lines other than hybridoma cell lines. Nucleic acid sequences encoding the cDNAs or genomic clones for the particular antibodies can be used for transformation of a suitable mammalian or nonmammalian host cells.

The present invention also encompasses nucleic acid molecules encoding anti-M-CSF antibodies. In some embodiments, different nucleic acid molecules encode a heavy chain and a light chain of an anti-M-CSF immunoglobulin. In other embodiments, the same nucleic acid molecule encodes a heavy chain and a light chain of an anti-M-CSF immunoglobulin. In one embodiment, the nucleic acid encodes an anti-M-CSF antibody of the invention.

A nucleic acid molecule encoding the heavy or entire light chain of an anti-M-CSF antibody or portions thereof can be isolated from any source that produces such antibody. In various embodiments, the nucleic acid molecules are isolated from a B cell isolated from an animal immunized with anti-M-CSF or from an immortalized cell derived from such a B cell that expresses an anti-M-CSF antibody. Methods of isolating mRNA encoding an antibody are well-known in the art. See, *e.g*., Sambrook, et al., Molecular Cloning 3rd Ed. Vol.3 (1989). The mRNA may be used to produce cDNA for use in the polymerase chain reaction (PCR) or cDNA cloning of antibody genes. In a preferred embodiment, the nucleic acid molecule is isolated from a hybridoma that has as one of its fusion partners a human immunoglobulin-producing cell from a non-human transgenic animal. In an even more preferred embodiment, the human immunoglobulin producing cell is isolated from a XENOMOUSE™ animal. In another embodiment, the human immunoglobulin-producing cell is from a non-human, non-mouse transgenic animal, as described above. In another embodiment, the nucleic acid is isolated from a non-human, non-transgenic animal. The nucleic acid molecules isolated from a non-human animal may be used, *e.g*., for humanized antibodies.

In some embodiments, a nucleic acid encoding a heavy chain of an anti-M-CSF antibody of the invention can comprise a polynucleotide sequence encoding a V_{H} domain of the invention joined in-frame to a polynucleotide sequence encoding a heavy chain constant domain from any source. Similarly, a nucleic acid molecule encoding a light chain of an anti-M-CSF antibody of the invention can comprise a polynucleotide sequence encoding a V_{L} domain of the invention joined in-frame to a polynucleotide sequence encoding a light chain constant domain from any source.

In a further aspect of the invention, nucleic acid molecules encoding the variable domain of the heavy (V_{H}) and light (V_{L}) chains are "converted" to full-length antibody genes. In one embodiment, nucleic acid molecules encoding the V_{H} or V_{L} domains are converted to full-length antibody genes by insertion into an expression vector already encoding heavy chain constant (C_{H}) or light chain (C_{L}) constant domains, respectively, such that the V_{H} segment is operatively linked to the C_{H} segment(s) within the vector, and the V_{L} segment is operatively linked to the C_{L} segment within the vector. In another embodiment, nucleic acid molecules encoding the V_{H} and/or V_{L} domains are converted into full-length antibody genes by linking, *e.g*., ligating, a nucleic acid molecule encoding a V_{H} and/or V_{L} domains to a nucleic acid molecule encoding a C_{H} and/or C_{L} domain using standard molecular biological techniques. Nucleic acid sequences of human heavy and light chain immunoglobulin constant domain genes are known in the art. See, *e.g*., Kabat et al., Sequences of Proteins of Immunological Interest, 5th Ed., NIH Publ. No. 91-3242, 1991. Nucleic acid molecules encoding the full-length heavy and/or light chains may then be expressed from a cell into which they have been introduced and the anti-M-CSF antibody isolated.

The present invention also provides vectors comprising nucleic acid molecules that encode the heavy chain of an anti-M-CSF antibody of the invention or an antigen-binding portion thereof. The invention also provides vectors comprising nucleic acid molecules that encode the light chain of such antibodies or antigen-binding portion thereof. The invention further provides vectors comprising nucleic acid molecules encoding fusion proteins, modified antibodies, antibody fragments, and probes thereof.

In some embodiments, the anti-M-CSF antibodies, or antigen-binding portions of the invention are expressed by inserting DNAs encoding partial or full-length light and heavy chains, obtained as described above, into expression vectors such that the genes are operatively linked to necessary expression control sequences such as transcriptional and translational control sequences. Expression vectors include plasmids, retroviruses, adenoviruses, adeno-associated viruses (AAV), plant viruses such as cauliflower mosaic virus, tobacco mosaic virus, cosmids, YACs, EBV derived episomes, and the like. The antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vectors. In a preferred embodiment, both genes are inserted into the same expression vector. The antibody genes are inserted into the expression vector by standard methods (*e.g*., ligation of complementary restriction sites on the antibody gene fragment and vector, or blunt end ligation if no restriction sites are present).

A convenient vector is one that encodes a functionally complete human C_{H} or C_{L} immunoglobulin sequence, with appropriate restriction sites engineered so that any V_{H} or V_{L} sequence can easily be inserted and expressed, as described above. In such vectors, splicing usually occurs between the splice donor site in the inserted J region and the splice acceptor site preceding the human C domain, and also at the splice regions that occur within the human C_{H} exons. Polyadenylation and transcription termination occur at native chromosomal sites downstream of the coding regions. The recombinant expression vector also can encode a signal peptide that facilitates secretion of the antibody chain from a host cell. The antibody chain gene may be cloned into the vector such that the signal peptide is linked in-frame to the amino terminus of the immunoglobulin chain. The signal peptide can be an immunoglobulin signal peptide or a heterologous signal peptide (i.e., a signal peptide from a non-immunoglobulin protein).

In addition to the antibody chain genes, the recombinant expression vectors of the invention carry regulatory sequences that control the expression of the antibody chain genes in a host cell. It will be appreciated by those skilled in the art that the design of the expression vector, including the selection of regulatory sequences may depend on such factors as the choice of the host cell to be transformed, the level of expression of protein desired, etc. Preferred regulatory sequences for mammalian host cell expression include viral elements that direct high levels of protein expression in mammalian cells, such as promoters and/or enhancers derived from retroviruses (such as retroviral LTRs), cytomegalovirus (CMV) (such as the CMV promoter/enhancer), Simian Virus 40 (SV40) (such as the SV40 promoter/enhancer), adenovirus, (*e.g*., the adenovirus major late promoter (AdMLP)), polyoma and strong mammalian promoters such as native immunoglobulin and actin promoters. For further description of viral regulatory elements, and sequences thereof, see *e.g*., U.S. Patent No. 5,168,062, U.S. Patent No. 4,510,245 and U.S. Patent No. 4,968,615. Methods for expressing antibodies in plants, including a description of promoters and vectors, as well as transformation of plants is known in the art. See, *e.g*., United States Patents 6,517,529, herein incorporated by reference. Methods of expressing polypeptides in bacterial cells or fungal cells, *e.g*., yeast cells, are also well known in the art.

In addition to the antibody chain genes and regulatory sequences, the recombinant expression vectors of the invention may carry additional sequences, such as sequences that regulate replication of the vector in host cells (*e.g*., origins of replication) and selectable marker genes. The selectable marker gene facilitates selection of host cells into which the vector has been introduced (see *e.g*., U.S. Patent Nos. 4,399,216, 4,634,665 and 5,179,017). For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in DHFR-host cells with methotrexate selection/amplification), the neomycin resistance gene (for G418 selection), and the glutamine synthetase gene.

Nucleic acid molecules encoding anti-M-CSF antibodies and vectors comprising these nucleic acid molecules can be used for transformation of a suitable mammalian, plant, bacterial or yeast host cell. Antibodies of the invention can be produced transgenically through the generation of a mammal or plant that is transgenic for the immunoglobulin heavy and light chain sequences of interest and production of the antibody in a recoverable form therefrom.

Transformation can be by any known method for introducing polynucleotides into a host cell, including, for example packaging the polynucleotide in a virus (or into a viral vector) and transducing a host cell with the virus (or vector) or by transfection procedures known in the art, as exemplified by U.S. Pat. Nos. 4,399,216, 4,912,040, 4,740,461, and 4,959,455. The transformation procedure used depends upon the host to be transformed. Methods for introduction of heterologous polynucleotides into mammalian cells are well known in the art and include, but are not limited to, dextran-mediated transfection, calcium phosphate precipitation, polybrene mediated transfection, protoplast fusion, electroporation, particle bombardment, encapsulation of the polynucleotide(s) in liposomes, peptide conjugates, dendrimers, and direct microinjection of the DNA into nuclei.

Mammalian cell lines available as hosts for expression are well known in the art and include many immortalized cell lines available from the American Type Culture Collection (ATCC), including but not limited to Chinese hamster ovary (CHO) cells, NS0 cells, SP2 cells, HeLa cells, baby hamster kidney (BHK) cells, monkey kidney cells (COS), human hepatocellular carcinoma cells (*e.g*., Hep G2), and a number of other cell lines. Non-mammalian cells, including but not limited to, bacterial (*e.g*., *E. coli* and *Streptomyces* species), yeast (*e.g*., *Schizosaccharomyces pombe, Saccharomyces cerevisiae* and *Pichia pastoris*), insect (*e.g*., Sf9 cells), and plants can also be used to express recombinant antibodies.

Production of recombinant antibodies in Chinese hamster ovary (CHO) cells is the most widely used mammalian expression system, particularly for production of antibodies. The most commonly used CHO expression system is based on the use of CHO cells deficient in the production of endogenous dihydrofolate reductase (DHFR) coupled with a DHFR gene amplification system. These DHFR- CHO cells are transfected with either a single plasmid containing both antibody genes and a functional DHFR gene or two plasmids with the DHFR gene contained on a separate plasmid from the antibody gene cassettes. In other embodiments, the DHFR gene is on the plasmid that encodes either the heavy or light chain.

Transfected cells are selected in increasing concentrations of the drug methotrexate. Survival on high concentrations of methotrexate (1 to 10 µM) is associated with gene amplification of the DHFR gene during integration into the host chromosome or integration into active regions of the chromosome. During the DHFR gene amplification step, the antibody genes are also coamplified and integrated into the host chromosome.

The expression methods are selected by determining which system generates the highest expression levels and produce antibodies with constitutive M-CSF binding properties. Further, expression of antibodies of the invention (or other moieties therefrom) from production cell lines can be enhanced using a number of known techniques. For example, the glutamine sythetase and DHFR gene expression systems are common approaches for enhancing expression under certain conditions. High expressing cell clones can be identified using conventional techniques, such as limited dilution cloning and Microdrop technology. The Glutamine Synthetase system is discussed in whole or part in connection with European Patent Nos. 0 216 846, 0 256 055, and 0 323 997 and European Patent Application No. 89303964.4.

In connection with the transgenic production in mammals, antibodies can also be produced in, and recovered from, the milk of goats, cows, or other mammals. See, *e.g*., U.S. Pat. Nos. 5,827,690, 5,756,687, 5,750,172, and 5,741,957.

When recombinant expression vectors encoding anti-M-CSF antibody genes are introduced into host cells, the antibodies are produced by culturing the host cells for a period of time sufficient to allow for expression of the antibody in the host cells or, more preferably, secretion of the antibody into the culture medium in which the host cells are grown. The antibodies may be present in, the culture medium, whole cells, in a cell lysate, or in a partially purified or substantially pure form. The antibodies expressed in cell lines as described above may be purified and/or isolated from the associated cellular material. Purification is performed in order to eliminate other cellular components or other contaminants, *e.g*. other cellular nucleic acids or proteins, by standard techniques, including alkaline/SDS treatment, column chromatography and others well known in the art. See Ausubel, F., et al., ed. Current Protocols in Molecular Biology, Greene Publishing and Wiley Interscience, New York (1987). In one embodiment, the antibodies can be recovered from the culture medium using protein purification methods, including the purification methods described in the Examples herein.

In the present invention, it is possible that anti-M-CSF antibodies expressed by different cell lines or in transgenic animals will have different glycosylation patterns from each other. However, all of the anti-M-CSF antibodies encoded by the nucleic acids and amino acids provided herein are considered part of the instant invention, regardless of their glycosylation pattern or modification or deletion thereof.

As used herein, the term "glycosylation" means the pattern of carbohydrate units that are covalently attached to an antibody. When it is said that the anti-M-CSF antibodies herein have a particular glycosylation pattern, it is meant that the majority of the referenced anti-M-CSF antibodies have that particular glycosylation pattern. In other aspects, when it is said that the anti-M-CSF antibodies herein have a particular glycosylation pattern, it is meant that greater than or equal to 75%, 90%, 95%, or 99% of the referenced anti-M-CSF antibodies have that particular glycosylation pattern. When the anti-M-CSF antibodies are glycosylated they may have any possible glycosylation pattern. Moreover, each heavy chain within one antibody may have the same glycosylation pattern or the two heavy chains may have differing glycosylation patterns.

The anti-M-CSF antibodies of the present invention also encompass glycosylation variants of anti-M-CSF antibody 8.10.3F (*e.g*., by insertion of a glycosylation site or deletion of any glycosylation site by deletion, insertion or substitution of suitable amino acid residues).

Glycosylation of polypeptides is typically either N-linked or O-linked. Glycosylation of antibody poplypeptides is typically N-linked and forms a biantennary structure. N-linked refers to the attachment of the carbohydrate moiety to the side chain of an asparagine residue. The tri-peptide sequences asparagine-X-serine and asparagine-X-threonine, where X is any amino acid except proline, are the recognition sequences for enzymatic attachment of the carbohydrate moiety to the asparagine side chain. Thus, the presence of either of these tri-peptide sequences in an antibody creates a potential glycosylation site.

The three distinct structures of biantennary glycans are designated "G0", "G1" and "G2" having zero, one, or two, respectively, terminal galactose residues on the nonreducing end of the glycan. See Jefferis et al., Biochem. J., 268, 529-537 (1990). In some cases, the glycan structure may also have a fucose residue linked to an N-acetylglucosamine, which is covalently bonded.to the asparagine amino acid (*e.g*., position 297) found in the antibody. When the fucose (F) is present, the biantennary glycan nomenclature is changed to "G0F", "G1F", or "G2F" depending upon the number of terminal galactose residues. See Teillaud, Expert Opin. Biol. Ther., 5(Suppl.1):S1327 (2005). Furthermore, when the antibody contains both of the two heavy chains, the glycan nomenclature is repeated for each of the two heavy chains.

For example, in one embodiment, the anti-M-CSF antibody 8.10.3F described herein has a glycosylation pattern of "G0F,G0F" as reported in Example 10. The "G0F,G0F" glycoform is a species in which both heavy chains have the G0 glycan attached and each G0 glycan has a fucose (F) residue linked to an N-acetylglucosamine, which is covalently bonded to an asparagine amino acid at residue 297 found in the heavy chains of antibody 8.10.3F.

For purposes of the present invention, the anti-M-CSF antibodies may be glycosylated or non-glycosylated. In certain embodiments, the anti-M-CSF antibodies have a glycosylation pattern that is selected from the group consisting of "GOF,GOF"; "G0F,G1F"; "G1F,G1F"; "G1F,G2F"; and mixtures thereof. In other embodiments, the anti-M-CSF antibodies have a glycosylation pattern that is "G0F,G0F". In still other embodiments, the anti-M-CSF antibodies are not glycosylated.

Site directed mutagenesis of the antibody CH2 domain to eliminate glycosylation is encompassed by the present invention in order to prevent changes in either the immunogenicity, pharmacokinetic, and/or effector functions resulting from non-human glycosylation.

### Routes of Administration and Dosages:

The compositions of this invention may be in liquid solutions (*e.g*., injectable and infusible solutions). The preferred form depends on the intended mode of administration and therapeutic application. Typical preferred compositions are in the form of injectable or infusible solutions, such as compositions similar to those used for passive immunization of humans. The preferred mode of administration is parenteral (*e.g*., intravenous, subcutaneous, intraperitoneal, intramuscular, and intrasternally) or by infusion techniques, in the form of sterile injectable aqueous, liquid or olagenous suspensions. As will be appreciated by the skilled artisan, the route and/or mode of administration will vary depending upon the desired results. In a preferred embodiment, the antibody is administered by intravenous infusion or injection. In another preferred embodiment, the antibody is administered by intramuscular or subcutaneous injection. Therapeutic compositions typically are sterile and stable under the conditions of manufacture and storage.

The composition can be formulated as a solution, microemulsion, dispersion, or liposome. Sterile injectable solutions can be prepared by incorporating the anti-M-CSF antibody in the required amount in an appropriate diluent with one or a combination of ingredients enumerated above, as required, followed by sterilization (*e.g*., filter sterilization). Generally, dispersions are prepared by incorporating the active compound into a sterile vehicle that contains a basic dispersion medium and the required other ingredients from those enumerated above. Such suspensions may be formulated according to the known art using those suitable dispersing of wetting agents and suspending agents or other acceptable agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed, including synthetic mono- or diglycerides. In addition, n-3 polyunsaturated fatty acids may find use in the preparation of injectables.

In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants.

Prolonged absorption of injectable compositions can be brought about by including in the composition an agent that delays absorption, for example, monostearate salts and gelatin or by formulating the composition into prolonged absorption forms such as, depots, liposomes, polymeric microspheres, polymeric gels, and implants.

Other methods for administration of the antibodies described herein include dermal patches that release the medications directly into a subject's skin. Such patches can contain the antibodies of the present invention in an optionally buffered, liquid solution, dissolved and/or dispersed in an adhesive, or dispersed in a polymer.

Still other methods for administration of the antibodies described herein include liquid ophthalmological drops for the eyes, incuding, without limitation, such ocular deliveries, as intravitreal, subtenon, and intraocular.

The antibody may be administered once, but more preferably is administered multiple times. For example, the antibody may be administered from once daily to once every six months or longer. The administering may be on a schedule such as three times daily, twice daily, once daily, once every two days, once every three days, once weekly, once every two weeks, once every month, once every two months, once every three months and once every six months.

The antibody may also be administered continuously via a minipump. The antibody may be administered at the site of a tumor or inflamed body part, into the tumor or inflamed body part or at a site distant from the site of the tumor or inflamed body part. The antibody may be administered once, at least twice or for at least the period of time until the condition is treated, palliated or cured. The antibody generally may be administered for as long as the tumor or inflammation is present provided that the antibody causes the tumor or cancer to stop growing or to decrease in weight or volume or until the inflamed body part experiences a reduction in inflammation. The antibody typically would be administered as part of a pharmaceutical composition as described *supra.*

The compositions of the invention may include a therapeutically effective amount or a prophylactically effective amount of an antibody or antigen-binding portion of the invention. In preparing the composition, the therapeutically effective amount of the anti-M-CSF antibody present in the composition can be determined, for example, by taking into account the desired dose volumes and mode(s) of administration, the nature and severity of the condition to be treated, and the age and size of the subject.

Exemplary, non-limiting dose ranges for administration of the pharmaceutical compositions of the present invention to a subject are from about 0.01 mg/kg to about 200 mg/kg (expressed in terms of milligrams (mg) of anti-M-CSF antibody administered per kilogram (kg) of subject weight), from about 0.01 mg/kg to about 100 mg/kg, from about 0.01 mg/kg to about 10 mg/kg, from about 0.1 mg/kg to about 10 mg/kg, or from about 0,1 mg/kg to about 3 mg/kg For purposes of the present invention, an average human subject weighs about 70 kg. In addition, the quantity of active component in a unit dose preparation may be varied or adjusted from 0.1 mg to 100 mg and from 0.5 mg to 100 mg, according to the particular application and the potency of the active component. Ranges intermediate to any of the dosages cited herein, *e.g*., about 0.01 mg/kg - 199 mg/kg, are also intended to be part of this invention. For example, ranges of values using a combination of any of the recited values as upper and/or lower limits are intended to be included.

Dosage regimens can also be adjusted to provide the optimum desired response (*e.g*., a therapeutic or prophylactic response) by administering several divided doses to a subject over time or the dose can be proportionally reduced or increased as indicated by the exigencies of the therapeutic situation. It is especially advantageous to formulate parenteral compositions in dosage unit form for ease of administration and uniformity of dosage.

Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the anti-M-CSF antibody or portion and the particular therapeutic or prophylactic effect to be achieved, and (b) the limitations inherent in the art of compounding such an antibody for the treatment of sensitivity in individuals.

The liquid compositions of the present invention can be prepared as unit dosage forms. For example, a unit dosage per vial may contain from 1 to 1000 milliliters (mls) of different concentrations of an anti-M-CSF antibody. In other embodiments, a unit dosage per vial may contain about 1 ml, 2 ml, 3 ml, 4 ml, 5 ml, 6 ml, 7 ml, 8 ml, 9 ml, 10 ml, 15 ml, 20 ml, 30 ml, 40 ml, 50 ml or 100 ml of different concentrations of an anti-M-CSF antibody. If necessary, these preparations can be adjusted to a desired concentration by adding a sterile diluent to each vial.

The liquid compositions of the present invention can also be prepared as unit dosage forms in sterile bags or containers, which are suitable for connection to an intravenous administration line or catheter.

### Stability Assessment:

The present invention comprises stable compositions comprising an anti-M-CSF antibody as described herein and a chelating agent. A stable composition is desirable to maintain or resist changes in, for example, product appearance and integrity (including physical or chemical degradation potentially leading to a reduction in biological activity). Various analytical techniques and indicators for measuring protein stability are reported in the literature and a number of these techniques and indicators are reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10: 29-90 (1993). In general, the liquid pharmaceutical compositions of the present invention exhibit improved stability when subjected to low storage temperatures over a period of time.

In one embodiment, the composition when stored at a temperature ranging from about 2°C to about 8°C (preferably, about 5°C) for at least about 12 months, preferably at least about 18 months and more preferably at least about 24 months, is more stable than an isotonic composition lacking the chelating agent that is stored under the same conditions for the same time.

In another embodiment, the composition when stored at a temperature from about 25°C to about 30°C for at least about 3 months, preferably at least 6 months, and more preferably at least about 12 months, is more stable than an isotonic composition lacking the chelating agent that is stored under the same conditions for the same time.

In another embodiment, the composition when stored at a temperature of about 40°C for at least about 1 months, preferably at least about 2 months, more preferably at least about 3 months, and yet more preferably at least about 26 weeks, is more stable than an isotonic composition lacking the chelating agent that is stored under the same conditions for the same time.

As used herein, the term "a freeze/thaw cycle" refers to techniques for using a liquid antibody sample after frozen storage, wherein the temperature of the sample is lowered to a temperature of 0°C or lower in order to freeze the liquid sample, and then subjecting the sample to a temperature which will restore its liquid state for a sufficient period of time to permit use of the sample, followed by and return to frozen storage, preferably at a temperature of 0°C or lower. As used herein, the term "frozen storage" refers to freezing and maintaining a previously liquid antibody sample at a temperature of 0°C or below, and preferably -20°C or lower.

In one embodiment, the composition when subjected to at least 1 freeze/thaw cycle, preferably at least 2 freeze/thaw cycles, more preferably at least 3 freeze/thaw cycles, still more preferably at least 4 freeze/thaw cycles, still more preferably at least 5 freeze/thaw cycles, and still more preferably at least 6 freeze/thaw cycles, remains stable.

In another embodiment, the composition satisfies two or more of the following conditions:
(a) the composition when stored at a temperature from about 2°C to about 8°C for at least about 12 months, preferably at least about 18 months and more preferably at least about 24 months, is more stable than an isotonic composition lacking the chelating agent that is stored under the same conditions for the same time;
(b) the composition when stored at a temperature from about 25°C to about 30°C for at least about 3 months, preferably at least 6 months, and more preferably at least about 12 months, is more stable than an isotonic composition lacking the chelating agent that is stored under the same conditions for the same time;
(c) the composition when stored at a temperature of about 40°C for at least about 1 months, preferably at least about 2 months, more preferably at least about 3 months, and yet more preferably at least about 26 weeks, is more stable than an isotonic composition lacking the chelating agent that is stored under the same conditions for the same time; or
(d) the composition when subjected to at least 1 freeze/thaw cycle, preferably at least 2 freeze/thaw cycles, more preferably at least 3 freeze/thaw cycles, still more preferably at least 4 freeze/thaw cycles, still more preferably at least 5 freeze/thaw cycles, and still more preferably at least 6 freeze/thaw cycles, remains stable.

In another embodiment, the composition satisfies three or more of the conditions - discussed immediately above.

For purposes of the present application, antibody aggregation, antibody fragmentation, and/or composition discoloration, for example, can be used as indicators of the stability of the composition. In general, the compositions of the present invention exhibit a lower level of at least one of antibody aggregation, antibody fragmentation and composition discoloration when subjected to one or more of the above-described storage or freeze/thaw conditions relative to isotonic compositions lacking the chelating agent that are subjected to the same conditions.

Protein aggregation in a liquid pharmaceutical composition can be measured by various methods known in the art. Such methods include gel filtration chromatography to separate proteins on the basis of their molecular weight and/or size. A "gel" is a matrix of water and a polymer, such as agarose or polymerized acrylamide. The present invention also encompasses the use of gel filtration HPLC (high performance liquid chromatography) (also known as Size-Exclusion HPLC, *i.e.,* SE-HPLC). Other recognized methods of measuring aggregation include cation exchange chromatography, which is the general liquid chromatographic technique of ion-exchange chromatography utilizing anion columns. The cations exchanged in the present invention are from the protein molecules. Since multivalent protein aggregates may have some multiple of the net charge of the antigen-binding monomer protein, the aggregates can be retained more strongly, and may be separated from the monomer molecules. A preferred cationic exchanger is a polyaspartic acid column. Thus, a monomeric protein can be readily distinguished from an aggregate. However, those of ordinary skill in the art will realize that aggregation assays of the invention are not limited to any particular type of chromatography column, so long as it is capable of separating the two forms of protein molecules.

Protein fragmentation in a composition of the present invention can be measured by various methods known in the art. Such methods include, for example, size exclusion chromatography, ultraviolet detection (*e.g*., at 214 nanometers), SDS-PAGE and/or matrix-assisted laser desorption ionization/time-of-flight mass spectrometry (MALDI/TOF MS). Protein fragmentation resulting in a charge alteration (*e.g*., occurring as a result of deamidation) can be evaluated, for example, by ion-exchange chromatography or isoelectric focusing (IEF).

Composition discoloration generally can be measured by visual observation of the composition itself. The present liquid pharmaceutical compositions comprising a chelating agent generally reduce composition discoloration (*e.g*., pink or yellow) relative to isotonic compositions that do not contain the chelating agent. For purposes of the present invention, the term "discoloration" refers to both changes in color (*e.g*., from clear and colorless to pink or yellow) and to changes in clarity (*e.g*., from clear and colorless to turbid, cloudy and/or having particulates). Composition discoloration generally can be measured using additional techniques such as by spectrophotometric (ultraviolet or visible wavelength detection, *e.g*., at 214 nanometers), fluorimetric detection and/or by visual comparison against a standard color scale of the compositions with and without the chelating agent. See PhEur 5.0, 2005 Monograph 2.2.2.

In one embodiment, antibody aggregation is determined after the composition is subjected to at least one of the following conditions:
(a) the composition is stored at a temperature ranging from about 2°C to about 8°C (preferably, about 5°C) for at least about 12 months, preferably at least about 18 months and more preferably at least about 24 months;
(b) the composition is stored at a temperature from about 25°C to about 30°C for at least about 3 months, preferably at least 6 months, and more preferably at least about 12 months;
(c) the composition is stored at a temperature of about 40°C for at least about 1 months, preferably at least about 2 months, more preferably at least about 3 months, and yet more preferably at least about 26 weeks; or
(d) the composition is subjected to at least 1 freeze/thaw cycle, preferably at least 2 freeze/thaw cycles, more preferably at least 3 freeze/thaw cycles, still more preferably at least 4 freeze/thaw cycles, still more preferably at least 5 freeze/thaw cycles, and still more preferably at
least 6 freeze/thaw cycles. Antibody aggregates are then chromatographically separated (*e.g*., using HPLC) and the extent of aggregation determined from the resulting chromatogram.

In one embodiment, the present invention also provides a composition comprising at least one antibody wherein the antibody comprises a heavy chain amino acid sequence comprising SEQ ID NO: 2 and a light chain amino acid sequence comprising SEQ ID NO: 4, wherein the antibody binds to human M-CSF; and a chelating agent, and the antibody is stable at a temperature of about 5°C for at least about 26 weeks.

In another embodiment, the present invention also provides a composition comprising at least one antibody wherein the antibody comprises a heavy chain amino acid sequence comprising SEQ ID NO: 2 and a light chain amino acid sequence comprising SEQ ID NO: 4, wherein the antibody binds to human M-CSF; and a chelating agent, and the antibody is stable at a temperature of about 25°C for at least about 26 weeks.

In another embodiment, the present invention also provides a composition comprising at least one antibody wherein the antibody comprises a heavy chain amino acid sequence comprising SEQ ID NO: 2 and a light chain amino acid sequence comprising SEQ ID NO: 4, wherein the antibody binds to human M-CSF; and a chelating agent, and the antibody is stable at a temperature of about 40°C for at least about 26 weeks.

The stable compositions of the present invention typically have an aggregate peak area on the chromatogram that is less than or equal to any of the following: about 8.0%, about 7.5%, about 7.0%, about 6.5%, about 6.0%, about 5.5%, about 5.0%, about 4.5%, about 4%, about 3.5%, about 3%, about 2.5%, about 2%, about 1.5%, about 1.0%, about 0.9%, or about 0.8% of the total peak area on the chromatogram. In one specific example of this technique for measuring aggregation, the composition is stored for 26 weeks at 40°C and chromatographic separation is then conducted using SE-HPLC with ultraviolet detection at 214 nanometers. This technique was used to measure antibody aggregation in Example 9 where, for example, Formulation 11 (containing a chelating agent) exhibited an aggregate peak area on the chromatogram of about 4.9% while Formulation 9 (isotonic, but lacking a chelating agent) exhibited an aggregate peak area on the chromatogram of about 11.6%.

In general, the difference between the aggregate chromatogram peak area for a stable composition of the present invention and the aggregate chromatogram peak area for an isotonic composition lacking the chelating agent that is subjected to the same conditions is at least about 8.0% or greater, is least about 7.5% or greater, is least about 7.0% or greater, is at least about 6.5% or greater, is at least about 6.0% or greater, is at least about 5.5% or greater, at least about 5.0% or greater, at least about 4.5% or greater, at least about 4% or greater, at least about 3.5% or greater, at least about 3.0% or greater, at least about 2.5% or greater, at least about 2.0% or greater, at least about 1.5% or greater, at least about 1.0% or greater, at least about 0.5% or greater, at least about 0.3% or greater, at least about 0.2% or greater, or at least about 0.1 % or greater. For example, the difference between Formulation 11 (aggregate peak area on the chromatogram of about 4.9%) and Formulation 9 (aggregate peak area on the chromatogram of about 11.6%) tested in Example 9 as discussed above is about 6.7%.

In another embodiment, antibody fragmentation is determined after the composition is subjected to at least one of the following conditions:
(a) the composition is stored at a temperature ranging from about 2°C to about 8°C, (preferably, about 5°C) for at least about 12 months, preferably at least about 18 months and more preferably at least about 24 months;
(b) the composition is stored at a temperature from about 25°C to about 30°C for at least about 3 months, preferably at least 6 months, and more preferably at least about 12 months;
(c) the composition is stored at a temperature of about 40°C for at least about 1 months, preferably at least about 2 months, more preferably at least about 3 months, and yet more preferably at least about 26 weeks; or
(d) the composition is subjected to at least 1 freeze/thaw cycle, preferably at least 2 freeze/thaw cycles, more preferably at least 3 freeze/thaw cycles, still more preferably at least 4 freeze/thaw cycles, still more preferably at least 5 freeze/thaw cycles, and still more preferably at least 6 freeze/thaw cycles.

Without wishing to be bound by theory it is believed that one of the anti-M-CSF antibody 8.10.3F fragments is the result of cleavage of the peptide bond between Asp99 and Pro100 within the heavy chain variable domain (V_{H}) amino acid sequence of SEQ ID NO: 2 for antibody 8.10.3F. In certain embodiments, the aforementioned antibody fragment results in a fragment that ranges in size (*i.e.,* weight) of from about 10.5kD to about 11.5kD, and in some embodiments, the band is about 11 kD, and in some embodiments, the band is about 10.8 kD. A reducing SDS-PAGE gel, run with appropriate size markers, can be used to visualize antibody fragment banding patterns and their sizes.

In general, the difference between the fragment band volume for a stable composition of the present invention and the fragment band volume for an isotonic composition lacking the chelating agent that is subjected to the same conditions is at least about 8.0% or greater, is least about 7.5% or greater, is least about 7.0% or greater, is at least about 6.5% or greater, is at least about 6.0% or greater, is at least about 5.5% or greater, at least about 5.0% or greater, at least about 4.5% or greater, at least about 4% or greater, at least about 3.5% or greater, at least about 3.0% or greater, at least about 2.5% or greater, at least about 2.0% or greater, at least about 1.5% or greater, at least about 1.0% or greater, at least about 0.5% or greater, at least about 0.3% or greater, at least about 0.2% or greater, or at least about 0.1 % or greater. For example, the difference between Formulation 11 (fragment band volume on the chromatogram of about 1.7%) and Formulation 9 (fragment band volume on the chromatogram of about 3.5%) tested in Example 9 as discussed above is about 1.8%.

In one embodiment, the present invention also provides a stable composition comprising at least one monoclonal anti-M-CSF antibody and a stabilizing amount of a chelating agent, wherein after the composition is stored for a period of about 26 weeks at a temperature of about 40°C, one or both of the following conditions are satisfied: the decrease between a fragment chromatogram peak area for the stable composition comprising at least one monoclonal anti-M-CSF antibody and the chelating agent, and a fragment chromatogram peak area for an isotonic composition lacking the chelating agent, is at least about 1%; or the decrease between a fragment chromatogram peak area for the stable composition comprising at least one monoclonal anti-M-CSF antibody and the chelating agent, and a fragment chromatogram peak area for an isotonic composition lacking the chelating agent, is at least about 0.5%.

In another embodiment, the present invention also provides a stable composition comprising at least one monoclonal anti-M-CSF antibody and a stabilizing amount of a chelating agent, wherein after the composition is stored for a period of about 26 weeks at a temperature of about 40°C, one or both of the following conditions are satisfied: the decrease between a fragment chromatogram peak area for the stable composition comprising at least one monoclonal anti-M-CSF antibody and the chelating agent, and a fragment chromatogram peak area for an isotonic composition lacking the chelating agent, is at least about 3.5%; or the decrease between a fragment chromatogram peak area for the stable composition comprising at least one monoclonal anti-M-CSF antibody and the chelating agent, and a fragment chromatogram peak area for an isotonic composition lacking the chelating agent, is at least about 1.7%.

In another embodiment, the present invention also provides a method for stabilizing at least one monoclonal anti-M-CSF antibody comprising the method of forming a composition comprising the antibodies and a stabilizing amount of a chelating agent, wherein after the composition is stored for a period of about 26 weeks at a temperature of about 40°C, one or both of the following conditions are satisfied: the decrease between an aggregate chromatogram peak area for the stable composition comprising monoclonal anti-M-CSF antibodies and the chelating agent, and an aggregate chromatogram peak area for an isotonic composition lacking the chelating agent, is at least about 1%; or the decrease between an aggregate chromatogram peak area for the stable composition comprising monoclonal anti-M-CSF antibodies and the chelating agent, and an aggregate chromatogram peak area for an isotonic composition lacking the chelating agent, is at least about 0.5%.

In another embodiment, the present invention also provides a method for stabilizing at least one monoclonal anti-M-CSF antibody comprising the method of forming a composition comprising the antibodies and a stabilizing amount of a chelating agent, wherein after the composition is stored for a period of about 26 weeks at a temperature of about 40°C, one or both of the following conditions are satisfied: the decrease between an aggregate chromatogram peak area for the stable composition comprising monoclonal anti-M-CSF antibodies and the chelating agent, and an aggregate chromatogram peak area for an isotonic composition lacking the chelating agent, is at least about 11.6%; and/or the decrease between an aggregate chromatogram peak area for the stable composition comprising monoclonal anti-M-CSF antibodies and the chelating agent, and a aggregate chromatogram peak area for an isotonic composition lacking the chelating agent, is at least about 4.9%.

In another embodiment, the present invention also provides methods for stabilizing at least one monoclonal anti-M-CSF antibody in a composition comprising the method: forming a composition comprising the antibody and at least one stabilizing chelating agent compound, wherein the composition comprises a sufficient amount of the stabilizing chelating agent to stabilize the composition during storage for a period of about 26 weeks at a temperature of about 40°C; and wherein at the end of the storage period at least one of the following conditions are satisfied: the amount of aggregated antibodies is less than or equal to about 3.5% by peak area of a chromatogram of the antibodies after chromatographic separation; or the amount of antibody fragment formed by antibody hydrolysis having a molecular weight ranging from about 10.5 kD to about 11.5 kD is less than or equal to about 1.7% by peak area of a chromatogram of the antibodies after chromatographic separation.

In another embodiment, the present invention also provides methods for stabilizing at least one monoclonal anti-M-CSF antibody 8.10.3F in a composition comprising the method: forming a composition comprising the antibody and at least one stabilizing chelating agent compound, wherein the composition comprises a sufficient amount of the stabilizing chelating agent to stabilize the composition during storage for a period of about 26 weeks at a temperature of about 40°C; and wherein at the end of the storage period at least one of the following conditions are satisfied: the amount of aggregated antibodies is less than or equal to about 3.5% by peak area of a chromatogram of the antibodies after chromatographic separation; or the amount of antibody fragment formed by antibody hydrolysis having a molecular weight ranging from about 10.5 kD to about 11.5 kD is less than or equal to about 1.7% by peak area of a chromatogram of the antibodies after chromatographic separation.

Accordingly, the present invention provides a method for stabilizing at least one anti-M-CSF antibody by combining the antibody in a liquid composition with a chelating agent in an amount, which reduces chemical or physical instability of the antibody.

Likewise, the present invention provides a method for analyzing the stability of at least one anti-M-CSF antibody comprising identifying an antibody fragment formed by antibody hydrolysis having a molecular weight ranging from about 10.5 kD to about 11.5 kD polypeptide fragment in a composition comprising the anti-M-CSF antibody by separating species by organic SE-HPLC, and identifying the presence of the polypeptide fragment in the composition by using ultraviolet detection at 214 nanometers.

Also provided is a method for detecting the presence of a polypeptide fragment having a molecular weight of between about 10 kD and about 12 kD in a composition comprising at least one anti-M-CSF antibody antibody, the method comprising separating species by organic SE-HPLC, and identifying the presence of the polypeptide fragment in the composition by using ultraviolet detection at 214 nanometers or at other suitable wavelengths, *e.g*., 280 nm.

### Methods of Treatment:

Any of the types of antibodies described herein may be used therapeutically. In a preferred embodiment, the anti-M-CSF antibody is a human antibody. In another preferred embodiment, the M-CSF is human M-CSF and the subject is a human subject. In yet another preferred embodiment, the anti-M-CSF antibody is a human IgG2 antibody. Alternatively, the subject may be a mammal that expresses an M-CSF protein that the anti-M-CSF antibody cross-reacts with. The antibody may be administered to a non-human mammal expressing M-CSF with which the antibody cross-reacts (*i.e*., a primate) for veterinary purposes or as an animal model of human disease. Such animal models may be useful for evaluating the therapeutic efficacy of antibodies of this invention.

In one embodiment, the present invention provides a method for the treatment of an M-CSF-mediated disorder in a subject, comprising administering to the subject a therapeutically effective amount of a liquid pharmaceutical composition comprising: at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF; and a pharmaceutically acceptable excipient.

In one embodiment, the present invention provides a method for the treatment of an inflammatory disease in a subject, comprising administering to the subject a therapeutically effective amount of a liquid pharmaceutical composition comprising: at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF; and a pharmaceutically acceptable excipient comprising a chelating agent alone or in combination with other excipients chosen from a buffer, antioxidant, a tonicity agent, or a surfactant, and mixtures thereof. In further embodiments, the aforementioned subject is one that is in need of the treatment of an inflammatory disease. In other embodiments, the methods and compositions of the present invention encompass the treatment of the inflammatory diseases selected from the group consisting of pain, fever, inflammation, atherosclerosis, sepsis, asthma, autoimmune diseases, osteoporosis, rheumatoid arthritis, and osteoarthritis.

In another embodiment, the present invention provides a method for the treatment of a neoplasia disorder in a subject, comprising administering to the subject a therapeutically effective amount of a liquid pharmaceutical composition comprising: at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF; and a pharmaceutically acceptable excipient comprising a chelating agent alone or in combination with other excipients chosen from a buffer, an antioxidant, a tonicity agent, or a surfactant, and mixtures thereof. In further embodiments, the aforementioned subject is one that is in need of the treatment of a neoplasia disorder.

Both of the terms, "neoplasia" and "neoplasia disorder", refer to a "neoplasm" or tumor, which may be benign, premalignant, metastatic, or malignant. Also encompassed by the present invention are benign, premalignant, metastatic, or malignant neoplasias. Also encompassed by the present invention are benign, premalignant, metastatic, or malignant tumors. Thus, all of benign, premalignant, metastatic, or malignant neoplasia or tumors are encompassed by the present invention and may be referred to interchangeably, as neoplasia, neoplasms or neoplasia-related conditions. Tumors are generally known in the art to be a mass of neoplasia or "neoplastic" cells. Although, it is to be understood that even one neoplastic cell is considered, for purposes of the present invention to be a neoplasm or alternatively, neoplasia.

Neoplasia disorders that may be treated by an anti-M-CSF antibody of the invention can involve any tissue or organ, and include, but are not limited to bone, brain, lung, squamous cell, bladder, gastric, pancreatic, breast, head, neck, liver, renal, ovarian, prostate, colorectal, esophageal, gynecological (*e.g*., cervical and ovarian), nasopharynx, or thyroid cancers. Also encompassed by the term neoplasia disorders, are bone metastases, melanomas, lymphomas, leukemias, and multiple myelomas. In particular, the anti-M-CSF antibody formulations of the present invention are useful to treat cancers of the breast, prostate, colon and lung.

In other embodiments, the methods and compositions of the present invention encompass the prevention and treatment of the neoplasia disorders selected from the group consisting of acral lentiginous melanoma, actinic keratoses, adenocarcinoma, adenoid cycstic carcinoma, adenomas, familial adenomatous polyposis, familial polyps, colon polyps, polyps, adenosarcoma, adenosquamous carcinoma, adrenocortical carcinoma, AIDS-related lymphoma, anal cancer, astrocytic tumors, bartholin gland carcinoma, basal cell carcinoma, bile duct cancer, bladder cancer, brain stem glioma, brain tumors, breast cancer, bronchial gland carcinomas, capillary carcinoma, carcinoids, carcinoma, carcinoma of the fallopian tubes, carcinoma of the endometrium, carcinosarcoma, cavernous, central nervous system lymphoma, cerebral astrocytoma, cholangiocarcinoma, chondosarcoma, choriod plexus papilloma/carcinoma, clear cell carcinoma, skin cancer, brain cancer, colon cancer, colorectal cancer, cutaneous T-cell lymphoma, cystadenoma, endodermal sinus tumor, endometrial hyperplasia, endometrial stromal sarcoma, endometrioid adenocarcinoma, ependymal, epitheloid, esophageal cancer, Ewing's sarcoma, extragonadal germ cell tumor, fibrolamellar; focal nodular hyperplasia, gallbladder cancer, gastrinoma, germ cell tumors, gestational trophoblastic tumor, glioblastoma, glioma, glucagonoma, hemangiblastomas, hemangioendothelioma, hemangiomas, hepatic adenoma, hepatic adenomatosis, hepatocellular carcinoma, Hodgkin's lymphoma, hypopharyngeal cancer, hypothalamic and visual pathway glioma, insulinoma, intaepithelial neoplasia, interepithelial squamous cell neoplasia, intraocular melanoma, invasive squamous cell carcinoma, large cell carcinoma, islet cell carcinoma, Kaposi's sarcoma, kidney cancer, laryngeal cancer, leiomyosarcoma, lentigo maligna melanomas, leukemia-related conditions, lip and oral cavity cancer, liver cancer, lung cancer, lymphoma, malignant mesothelial tumors, malignant thymoma, medulloblastoma, medulloepithelioma, melanoma, meningeal, merkel cell carcinoma, mesothelial, metastatic carcinoma, mucoepidermoid carcinoma, multiple myeloma/plasma cell neoplasm, mycosis fungoides, myelodysplastic syndrome, myeloproliferative conditions, nasal cavity and paranasal sinus cancer, nasopharyngeal cancer, neuroblastoma, neuroepithelial adenocarcinoma nodular melanoma, neoplasms of the central nervous system (*e.g*., primary CNS lymphoma, spinal axis tumors, brain stem gliomas or pituitary adenomas), non-Hodgkin's lymphoma, oat cell carcinoma, oligodendroglial, oral cancer, oropharyngeal cancer, osteosarcoma, pancreatic polypeptide, ovarian cancer, ovarian germ cell tumor, pancreatic cancer, papillary serous adenocarcinoma, pineal cell, pituitary tumors, plasmacytoma, pseudosarcoma, pulmonary blastoma, parathyroid cancer, penile cancer, pheochromocytoma, pineal and supratentorial primitive neuroectodermal tumors, pituitary tumor, plasma cell neoplasm, pleuropulmonary blastoma, prostate cancer, rectal cancer, renal cell carcinoma, retinoblastoma, rhabdomyosarcoma, sarcoma, serous carcinoma, small cell carcinoma, small intestine cancer, soft tissue carcinomas, somatostatin-secreting tumor, squamous carcinoma, squamous cell carcinoma, submesothelial, superficial spreading melanoma, supratentorial primitive neuroectodermal tumors, thyroid cancer, undifferentiatied carcinoma, urethral cancer, uterine cancer, uveal melanoma, verrucous carcinoma, vaginal cancer, vipoma, vulvar cancer, Waldenstrom's macroglobulinemia, well differentiated carcinoma, and Wilm's tumor.

In a more preferred embodiment, the anti-M-CSF antibody is administered to a subject with breast cancer, prostate cancer, lung cancer or colon cancer. In an even more preferred embodiment, the method causes the cancer to stop proliferating abnormally, or not to increase in weight or volume or to decrease in weight or volume.

The compositions of the present invention may be used in combination with agents useful for treating a cancer in a mammal such as chemotherapeutic agents. In some embodiments, the chemotherapeutic agent is selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, tamoxifen, anti-hormones, *e.g*., anti-androgens, and anti-angiogenesis agents.

In addition, a composition of a human anti-M-CSF monoclonal antibody of the invention can also be used with signal transduction inhibitors, such as agents that can inhibit EGF-R (epidermal growth factor receptor) responses, such as EGF-R antibodies, EGF antibodies, and molecules that are EGF-R inhibitors; VEGF (vascular endothelial growth factor) inhibitors, such as VEGF receptors and molecules that can inhibit VEGF; and erbB2 receptor inhibitors, such as organic molecules or antibodies that bind to the erbB2 receptor, for example, HERCEPTIN^{™} (Genentech, Inc.). EGFR-inhibiting agents include, but are not limited to, the monoclonal antibodies C225 and anti-EGFR 22 Mab (ImClone Systems Incorporated), ABX-EGF (Abgenix/Cell Genesys), EMD-7200 (Merck KgaA), EMD-5590 (Merck KgaA), MDX-447/H-477 (Medarex Inc. and Merck KgaA), and the compounds ZD-1834, ZD-1838 and ZD-1839 (AstraZeneca), PKI-166 (Novartis), PKI-166/CGP-75166 (Novartis), PTK 787 (Novartis), CP 701 (Cephalon), leflunomide (Pharmacia/Sugen), CI-1033 (Warner Lambert Parke Davis), CI-1033/PD 183,805 (Warner Lambert Parke Davis), CL-387,785 (Wyeth-Ayerst), BBR-1611 (Boehringer Mannheim GmbH/Roche), Naamidine A (Bristol Myers Squibb), RC-3940-II (Pharmacia), BIBX-1382 (Boehringer Ingelheim), OLX-103 (Merck & Co.), VRCTC-310 (Ventech Research), EGF fusion toxin (Seragen Inc.), DAB-389 (Seragen/Lilgand), ZM-252808 (Imperial Cancer Research Fund), RG-50864 (INSERM), LFM-A12 (Parker Hughes Cancer Center), WHIP97 (Parker Hughes Cancer Center), GW-282974 (Glaxo), KT-8391 (Kyowa Hakko) and EGF-R Vaccine (York Medical/Centro de Immunologia Molecular (CIM)). These and other EGF-R-inhibiting agents can be used in the present invention.

VEGF inhibitors, for example SU-5416 and SU-6668 (Sugen, Inc.), AVASTIN™ (Genentech), SH-268 (Schering), and NX-1838 (NeXstar) can also be combined with the compound of the present invention. Anti-inflammatory agents can be used in conjunction with an anti-M-CSF antibody formulation of the present invention. For the treatment of inflammatory diseases such as rheumatoid arthritis, the human anti-M-CSF antibodies of the invention may be combined with agents such as TNF-α inhibitors such as TNF drugs (such as REMICADE™, CDP-870 and HUMIRA™) and TNF receptor immunoglobulin molecules (such as ENBREL™), CTLA-4lg, anti-CD20 antibodies (*e.g*., rituxamab), IL-6 antibodies, IL-6 receptor antibodies (*e.g*., tocilizumab), IL-1 inhibitors, IL-1 receptor antagonists or soluble IL-1ra (*e.g*. Kineret or ICE inhibitors), Cox-2 inhibitors (such as celecoxib, rofecoxib, valdecoxib and etoricoxib), metalloprotease inhibitors (preferably MMP-13 selective inhibitors), p2X7 inhibitors, α2δ ligands (such as NEURONTIN™ AND PREGABALIN™), low dose methotrexate, sulfasalazine, Mesalamine leflunomide, hydroxychloroquine, d-penicillamine, auranofin or parenteral or oral gold.

The compositions of the invention can also be used in combination with existing therapeutic agents for the treatment of osteoarthritis. Suitable agents to be used in combination include standard non-steroidal anti-inflammatory agents (hereinafter NSAID's) such as piroxicam, diclofenac, propionic acids such as naproxen, flurbiprofen, fenoprofen, ketoprofen and ibuprofen, fenamates such as mefenamic acid, indomethacin, sulindac, apazone, pyrazolones such as phenylbutazone, salicylates such as aspirin, Cox-2 inhibitors such as celecoxib, valdecoxib, rofecoxib and etoricoxib, analgesics and intraarticular therapies such as corticosteroids and hyaluronic acids such as hyalgan and synvisc.

The human anti-M-CSF antibody compositions of the present invention may also be used in combination with cardiovascular agents such as calcium channel blockers, lipid lowering agents such as statins (*e.g*., atorvastain calcium), fibrates, beta-blockers, ACE inhibitors, Angiotensin-2 receptor antagonists, and platelet aggregation inhibitors.

The compositions of the present invention may also be used in combination with CNS agents such as antidepressants (such as sertraline), anti-Parkinsonian drugs (such as deprenyl, L-dopa, REQUIP™, MIRAPEX™, MAOB inhibitors such as selegine and rasagiline, comP inhibitors such as Tasmar, A-2 inhibitors, dopamine reuptake inhibitors, NMDA antagonists, nicotine agonists, dopamine agonists and inhibitors of neuronal nitric oxide synthase), and anti-Alzheimer's drugs such as donepezil, Tacrine, α2δ Ligands (such NEUROTIN™ and PREGABALIN™) inhibitors, Cox-2 inhibitors, propentofylline or metryfonate.

The anti-M-CSF antibody compositions of the present invention may also be used in combination with osteoporosis agents such as roloxifene, droloxifene, lasofoxifene or fosomax and immunosuppressant agents such as FK-506 and rapamycin.

### Articles of Manufacture

In another embodiment of the invention, an article of manufacture is provided comprising a container, which holds the composition comprising at least one of the monoclonal anti-M-CSF antibodies of the present invention in combination with a pharmaceutically acceptable chelating agent, and optionally provides instructions for its use. Suitable containers include, for example, bottles, bags, vials and syringes. The container may be formed from a variety of materials such as glass or plastic. An exemplary container is a 3-20 cc single use glass vial. Alternatively, for a multidose formulation, the container may be 3-100 cc glass vial. The container holds the formulation and the label on, or associated with, the container may indicate directions for use. The article of manufacture may further include other materials desirable from a commercial and user standpoint, including other buffers, diluents, filters, needles, syringes, and package inserts with instructions for use, contraindications, and/or lists of potential side-effects.

The present invention also provides a kit for preparing a composition of an antibody comprising a first container comprising monoclonal anti-M-CSF antibody 8.10.3F, and a second container comprising a pharmaceutically acceptable chelating agent.

The following examples describe embodiments of the invention. Other embodiments within the scope of the claims herein will be apparent to one skilled in the art from consideration of the specification or practice of the invention as disclosed herein. It is intended that the specification, together with the examples, be considered exemplary only, with the scope and spirit of the invention being indicated by the claims, which follow the examples. In the examples, all percentages are given on a weight basis unless otherwise indicated. The skilled artisan will appreciate that the weight quantities and/or weight-to-volume ratios recited in the examples can be converted to moles and/or molarities using the art-recognized molecular weights of the recited ingredients. Weight quantities exemplified herein (*e.g*., grams) are for the volumes (*e.g*., of buffer solutions, antibody formulation, etc.) recited. The skilled artisan will appreciate that the weight quantities can be proportionally adjusted when different formulation volumes are desired.

### EXAMPLE 1

This Example shows the generation of hybridoma cell lines that produce anti-M-CSF antibodies as described in U.S. Published Application No. 20050059113 to Bedian, et al.

### Immunization and hybridoma generation

Eight to ten week old XENOMOUSE™ mice were immunized intraperitoneally or in their hind footpads with human M-CSF (10 µg/dose/mouse). This dose was repeated five to seven times over a three to eight week period. Four days before fusion, the mice were given a final injection of human M-CSF in phosphate buffered saline (PBS). The spleen and lymph node lymphocytes from immunized mice were fused with the non-secretory myeloma P3-X63-Ag8.653 cell line, and the fused cells were subjected to HAT selection. See Galfre, G. and Milstein, C., "Preparation of monoclonal antibodies: strategies and procedures." Methods Enzymol. 73:3-46 (1981). A panel of hybridomas all secreting M-CSF specific human IgG2 and IgG4 antibodies was recovered. Antibodies also were generated using XENOMAX^{™} technology as described in Babcook, J.S. et al., Proc. Natl. Acad. Sci. USA 93:7843-48, 1996. Nine cell lines engineered to produce antibodies of the invention were selected for further study and designated 252, 88, 100, 3.8.3, 2.7.3, 1.120.1, 9.14.4, 8.10.3 and 9.7.2. The hybridomas were deposited under terms in accordance with the Budapest Treaty with the American Type Culture Collection (ATCC), 10801 University Blvd., Manassas, VA 20110-2209 on August 8, 2003. The hybridomas were assigned the following accession numbers:

| | |
|---|---|
| Hybridoma 3.8.3 (LN 15891) | PTA-5390 |
| Hybridoma 2.7.3 (LN 15892) | PTA-5391 |
| Hybridoma 1.120.1 (LN 15893) | PTA-5392 |
| Hybridoma 9.7.2 (LN 15894) | PTA-5393 |
| Hybridoma 9.14.4 (LN 15895) | PTA-5394 |
| Hybridoma 8.10.3 (LN 15896) | PTA-5395 |
| Hybridoma 88-gamma (UC 25489) | PTA-5396 |
| Hybridoma 88-kappa (UC 25490) | PTA-5397 |
| Hybridoma 100-gamma (UC 25491) | PTA-5398 |
| Hybridoma 100-kappa (UC 25492) | PTA-5399 |
| Hybridoma 252-gamma (UC 25493) | PTA-5400 |
| Hybridoma 252-kappa (UC 25494) | PTA-5401 |

### EXAMPLE 2

This Example shows the generation of a recombinant mammalian cell line that produces anti-M-CSF antibodies.

DNA encoding the heavy and light chains of monoclonal antibodies 8.10.3 was cloned from the respective hybridoma cell line 8.10.3 and the DNA sequences were determined by methods known to one skilled in the art. The DNA from the hybridoma cell line 8.10.3 was mutated at specific framework regions in the variable domain to obtain 8.10.3F. From nucleic acid sequence and predicted amino acid sequence of the antibody 8.10.3F, the identity of the gene usage for each antibody chain was determined by ("VBASE"). Table 2 sets forth the gene utilization of antibody 8.10.3F in accordance with the present invention:

**Table 2: Heavy and Light Chain Human Gene Utilization and Sequences**

| Antibody | Heavy Chain | | | | Light Chain | | |
|---|---|---|---|---|---|---|---|
| | SEQ ID NO: | V_{H} | D_{H} | J_{H} | SEQ ID NO: | V_{κ} | J_{κ} |
| 8.10.3F | **1** (nucleic acid) | 3-48 | 1-26 | 4b | **3** (nucleic acid) | A27 | 4 |
| | **2** (amino acid) | | | | **4** (amino acid) | | |

Antibody 8.10.3F DNA sequence inserts were obtained from the hybridoma cell line and subcloned into expression vectors. The expression vectors were then transfected into a mouse myeloma (NSO) host cell line to generate a primary transfectant cell line producing anti-M-CSF antibodies having the heavy and light chain sequences of 8.10.3F. Finally, samples of the 8.10.3F antibody producing NS0 cell line were frozen and stored in liquid nitrogen.

### EXAMPLE 3

This Example shows the production of anti-M-CSF 8.10.3F antibodies from the NS0 cell line generated according to Example 2.

A vial of 8.10.3F subcloned NS0 cells was removed from liquid nitrogen storage as described in Example 2. The frozen cells were thawed rapidly to 37°C until the last ice crystal disappeared. The entire contents (1 milliliter) of the thawed vial were then pipetted into a 75 cm² T-Flask. Fourteen milliliters of prewarmed (36.5°C ± 1.0°C) CD Hybridoma growth medium (available from Invitrogen, Carlsbad, CA) containing 10% Low IgG containing fetal bovine serum (available from Invitrogen, Carlsbad, CA) was slowly pipetted into the T-Flask.

The flask was planted at a target viable cell density of from about 2.0 x 10⁵ to about 5.0 x 10⁵cells/ml. The flask was then placed in an incubator having a carbon dioxide level of 9% and a temperature of 36.5°C and the cells were grown for about 3 days. At the end of this period, targeted cell number was on the order of 1.0 to 3.0x10⁶ cells/ml.

After the cells were grown for about 3 days, they were split so that a target cell density of 2.5 x 10⁵ +/- 0.5 x 10⁵ was achieved and then disposable shake flasks (*i.e*., seed flasks) were seeded based on cell density. Each shake flask contained CD Hybridoma growth media containing 10% Low IgG containing fetal bovine serum. The flasks were then shaken at 100 +/-10 rpm at 36.5°C ± 1.0°C for about 3 days. Cell density in each flask at the end of this period was 1.0 to 3.0x10⁶ cells/ml and greater than 80% of the cells were viable.

After the cells were grown for about 3 days, the broth was harvested. Clarified broth was obtained after centrifugation for 15 minutes at 7000 rpm and subsequent filtration with a sterile 0.22 µm 4 inch Opticap™ Millipore™ filter into a sterile TC-Tech™ bag.

### EXAMPLE 4

This Example shows the purification of anti-M-CSF antibodies from Example 3.

The clarified broth was then purified with three chromatographic steps comprising a Protein A affinity column and two ion exchange columns. A low pH inactivation and a viral filtration were also done to clear any potential viruses in the process. The product is concentrated and diafiltered into the formulation buffer to make the anti-M-CSF antibody composition.

The Protein A column (Amersham Pharmacia) was prepped by washing with 3 column volumes of 8 M urea, followed by an equilibration wash with 20 mM Tris (pH 8). The final filtrate from Example 3 was spiked with 2% v/v of 1 M Tris pH 8.3 and 0.02% NaN₃ before being loaded onto the Protein A column via gravity-drip mode. After load was complete, the resin was washed with 5 column volumes of 20 mM Tris (pH 8), followed by 5 column volumes of the elution buffer (0.1 M Glycine, pH 3.0). Any precipitation was noted, and then a 10% v/v spike of 1 M Tris pH 8.3 was added to the eluted antibody. The eluted protein was then dialyzed into 100 fold the volume amount of eluted material of dialysis buffer (*e.g*., 140 mM sodium chloride/20mM sodium acetate, pH 5.5). Following dialysis, the antibody was sterile filtered with a 0.22 µm filter and stored until further use.

### EXAMPLE 5

A study was conducted to evaluate the effect of EDTA and histidine on discoloration, aggregation, and fragmentation in liquid compositions comprising monoclonal anti-M-CSF antibody 8.10.3F. Discoloration and aggregation in such liquid compositions are generally undesirable from a product aesthetic perspective, a product integrity perspective, or both.

### Preparation of the Formulation

The pharmaceutical formulations of the invention were made according to the following protocol. Materials which were used in preparation of the formulations include: glacial acetic acid 99.9% (Molecular Weight (MW) 60.05); concentrated sodium hydroxide 18.94N (50% w/w; MW 40.0); concentrated hydrochloric acid 37.8% (12.44N; MW 36.46); histidine (MW 155.16); sodium chloride (MW 58.44); mannitol (MW 182.17); polysorbate 80 (crillet® 4 HP); sodium acetate trihydrate (MW 136.08); sodium citrate dihydrate (MW 294.1); disodium ethylenediaminetetraacetic acid dihydrate (MW 292.2); succinic acid (MW 118.1); antibody 8.10.3F bulk solution (about 10 mg/ml in sodium acetate, pH 5.5, prepared according to Examples 2-4); and water for injection (Milli-Q water). These solutions were prepared and then sterile filtered into 1 L Nalgene bottles and stored at 5°C.

The antibody formulations that were evaluated are listed in Table 3 below. To prepare each formulation, first the formulation buffers were made either with buffer only or with buffer and additional excipients such as tonicity agents (except addition of surfactant such as polysorbate 80) (reported in Table 3), followed by adjustment of pH to desired level. The buffer solutions were then filtered through sterilizing filter (0.22 micron pore size) into a sterilized receptacle. An antibody bulk solution from the purification process described in Example 4 was obtained at about 10-15 mg/mL in 20 mM sodium acetate buffer pH 5.5 and 140 mM sodium chloride. Buffer exchanges of this bulk solution into the above identified formulation solutions were carried out with Amicon® Centrifugal concentrators (*e.g*. with 30kD cut-off membrane) on an Eppendorf 5810R centrifuge run at about 4500xg. At least 8 volume exchanges were made for each formulation in respective buffers. Approximately 2 to 5 milliliters of formulations 1 through 13 were prepared. Antibody concentrations were determined by Ultraviolet-Visible spectrometry (UV-Vis) method using an extinction coefficient of 1.43 (mg/ml)⁻¹ cm⁻¹ at 280 nm. The final volume of the antibody solution was adjusted by appropriate dilution to achieve desired antibody concentration. A 20 mg/ml polysorbate 80 (PS80) solution was prepared by dilution and dissolution of polysorbate 80 by the appropriate formulation buffer prepared as described above. For formulations 9 to 13, addition of required quantity of 20 g/L polysorbate 80 solution was made to achieve 0.2 g/L polysorbate 80 in the antibody formulation. The formulations with all of its ingredients included, was then sterilized by filtration through sterile 0.22 micron membrane filter.

For the formulation number 11 (*i.e*., histidine, mannitol, polysorbate 80, and EDTA), a 1 molar (M) hydrochloric acid solution was first prepared by appropriate dilution from concentrated hydrochloric acid with water for injections. Individual solutions were then prepared by dissolving the following pre-weighed ingredients in about 90% of the water for injections: 45 grams per liter (g/L) of mannitol, 1.55 g/L of histidine, 0.02 g/L of disodium ethylenediaminetetraacetic acid dihydrate. After addition of all of the excipients except polysorbate 80, dissolution was achieved, and the pH of the solution was adjusted to pH 6 with 1 M hydrochloric acid solution which was prepared as described above. After the addition of the hydrochloric acid solution, the final quantity of the water was added. The buffer solution was then filtered through a sterilization filter (0.22 micron pore size) into a sterilized receptacle.

A 20g/L polysorbate 80 solution was prepared by appropriate dilution of polysorbate 80 by formulation buffer (45 g/L of mannitol, 1.55 g/L of histidine, 0.02 g/L of disodium ethylenediaminetetraacetic acid dihydrate, pH 6).

The filtered formulations were then filled into vials. The vials were washed and autoclaved, as were the 13 mm Daikyo 777-1 serum stoppers. A fill-volume of 0.25 to 1 ml was used in 2 ml Type 1 glass vials. The vials were closed with Daikyo 777-1 Flurotec® coated stoppers, crimp sealed, and placed in stability chambers.

### Formulation appearance analysis:

Each formulation was visually evaluated at initial (*i.e*., time zero) and therafter at desired sampling intervals (weeks) for particulate formation, color change, and turbidity change. Visual observations were reported in Table 3. The appearance assays were via visual inspection performed in a light box equipped with white and black backgrounds. Antibody concentrations were determined by ultraviolet-visible spectrometry (UV-Vis) methods using an extinction coefficient of 1.34 (mg/ml)-1.cm-1 at 280 nm.

**Table 3: Description of Anti-M-CSF 8.10.3F Antibody Formulations, Appearance and Concentration.**

| **Formulation No.** | **Formulation Description** | **pH** | **Visual Evaluation** | **8.10.3F Antibody concentration, (mg/ml)** |
|---|---|---|---|---|
| 1 | 20 mM sodium acetate | 4.0 | clear and colorless | 8.2 |
| 2 | 5 mM sodium acetate, 5 mM sodium citrate, 5 mM histidine, 5 mM succinic acid | 5.0 | clear and colorless | 11.9 |
| 3 | 5 mM sodium acetate, 5 mM sodium citrate, 5 mM histidine, 5 mM succinic acid | 5.5 | clear and colorless | 8.2 |
| 4 | 20 mM histidine | 6.0 | clear and colorless | 8.2 |
| 5 | 20 mM sodium citrate | 5.5 | clear and colorless | 8.1 |
| 6 | 20 mM sodium acetate | 5.5 | clear and colorless | 8.1 |
| 7 | 20 mM sodium succinate | 5.5 | clear and colorless | 8.4 |
| 8 | 20 mM disodium EDTA dihydrate | 5.5 | clear and colorless | 8.1 |
| 9 | 20 mM sodium acetate, 140 mM NaCl, 0.2 mg/ml polysorbate 80 | 5.5 | clear and colorless | 8.4 |
| 10 | 10mM sodium acetate, 45 mg/ml Mannitol, 0.02 mg/ml EDTA, 0.2 mg/ml polysorbate 80 | 5.5 | clear and colorless | 9.0 |
| 11 | 10 mM histidine, 45 mg/ml mannitol, 0.02 mg/ml disodium EDTA dihydrate, 0.2 mg/ml polysorbate 80 . | 6.0 | clear and colorless | 8.4 |
| 12 | 10 mM sodium citrate, 45 mg/ml mannitol, 0.02 mg/ml disodium EDTA dihydrate, 0.2 mg/ml polysorbate 80 | 5.5 | clear and colorless | 8.4 |
| 13 | 10 mM sodium succinate, 45 mg/ml mannitol, 0.02 mg/ml disodium EDTA dihydrate, 0.2 mg/ml polysorbate 80 | 5.5 | clear and colorless | 8.6 |

The results in Table 3 indicate that all tested antibody 8.3.10F formulations had no significant discoloration, no significant turbidity, and no significant particulate formation at the initial timepoint (*i.e.,* time equal zero).

### EXAMPLE 6

A study was conducted to evaluate the effect of various formulation compositions and pH on anti-M-CSF antibody 8.10.3F fragmentation.

### Fragmentation Analysis:

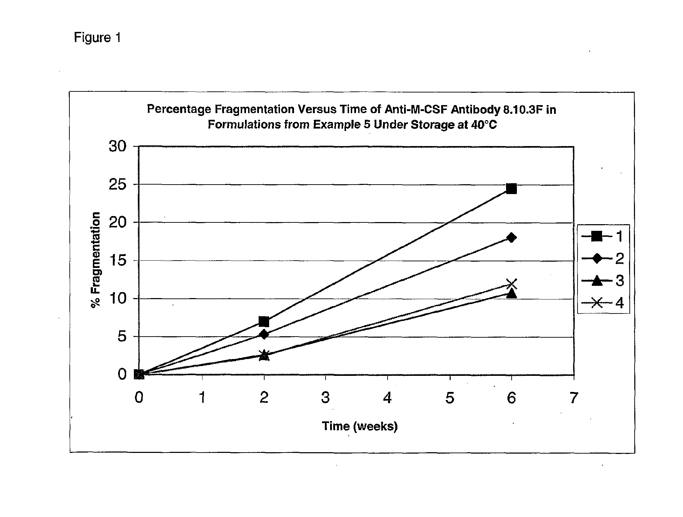

As noted above, the antibody formulations prepared according to Table 3 and Example 5 were stored at a temperature of 40ºC. At weeks, 0 (initial), 2, 4, and 6, the 40°C formulations were analyzed for fragmentation using reduced SDS-PAGE (rSDS-PAGE). The formulation vials were aseptically sampled at each time point and an aliquot from the vial was loaded onto NuPAGE 4-12% bis-Tris gels with colloidal blue (Coomassie) stain. Gel reduction was achieved by use of the NuPAGE® reducing agent. Percentage fragmentation (*i.e*., the presence of an 11 kilodalton (kD) polypeptide fragment and other fragments) in the reduced gels was estimated densitometrically by 100 % minus (% heavy chain + % light chain) and reported in Table 4. Figure 1 shows a line graph that shows the percent fragmentation (*i.e*., presence of polypeptides other than heavy chain (approx 50 kD) and light chain (approx 25 kD)) estimated from SDS-PAGE reduced gels. Reduced fragmentation was seen at pH ranges between 5.5 and 6.0. The gel data showed fragment bands with approximate molecular masses of 40 kD and 11 kD.

**Table 4: Percent Fragmentation for Formulations in Table 3 after Storage at 40°C:**

| **Formulation No.** | **Initial** | **2 weeks 40°C** | **4 weeks 40°C** | **6 weeks 40°C** |
|---|---|---|---|---|
| 1 | 0 | 7% | 20.1% | 24.5% |
| 2 | 0 | 5.3% | 13.1% | 18.1% |
| 3 | 0 | 2.6% | 6.6% | 10.8% |
| 4 | 0 | 2.5% | 10.9% | 12% |
| 5 | 0 | 2.4% | 4.7% | 2.2% |
| 6 | 0 | 0 | 2.2% | 2.6% |
| 7 | 0 | 0 | 6.2% | 5.5% |
| 8 | 0 | 2.7% | 8.8% | 9.4% |
| 9 | 0 | - | - | 4.1% |
| 10 | 0 | - | - | 2.9% |
| 11 | 0 | - | - | 5.0% |
| 12 | 0 | - | - | 4.6% |
| 13 | 0 | - | - | 3.7% |

Figure 1 shows the percent fragmentation (*i.e*., presence of polypeptides other than heavy chain (about 50 kD) and light chain (about 25 kD) for each of the sample formulations detailed in Table 4. Reduced levels of fragmentation were seen in formulations having pH ranges between 5.5 and 6.0. Reduced levels of fragmentation were also seen in formulations without acetate, but having a chelating agent.

### EXAMPLE 7

A study was conducted to evaluate the effect of various formulation compositions and pH on anti-M-CSF antibody 8.10.3F charged species generation. Percentage major isoelectric focusing (IEF) band estimated from IEF gels with antibody samples stored at 40°C over 6 weeks.

### Formation of Acidic and Basic Species:

Antibody formulations 1-4 prepared according to Table 3 and Example 5 were stored at a temperature of 40ºC. After storing for 6 weeks, each formulation was analyzed for the formation of acidic and basic species using Isoelectric Focusing (IEF). The Imaging Capillary Electrophoresis was conducted using a Convergent Biosciences iCE₂₈₀ analyzer for evaluation of charge heterogeneity. The Convergent iCE₂₈₀ is an imaging capillary isoelectric focusing (IEF) instrument, which allows the user to take an image of a separated sample contained within a capillary. IEF asays were conducted using pH 3-10.5 polyacrylamide gels and Coomassie blue stain. The sample protein components were separated based on their relative isoelectric points (pl).The major species was assigned based on the highest densitometric band intensity at a particular pl, in the initial samples. The change in percentage major species was followed as a function of storage duration. The loss in percentage major species from the initial value is a mesure of the extent of acidic and basic specied formation.

Formation of acidic and basic species was also monitored by Imaging Capillary Electrophoresis (iCE). ICE was conducted using a Convergent Biosciences iCE₂₈₀ analyzer for evaluation of charge heterogeneity. The Convergent iCE₂₈₀ is an imaging capillary isoelectric focusing instrument, which allows the user to take an image of a separated sample contained within a capillary. The samples were prepared in a mixture of electrophoretic ampholytes, methyl cellulose, calibration markers, and water. The samples were introduced into the iCE₂₈₀ and a high potential/voltage was applied. The sample protein components were separated based on their relative isoelectric points (pl). The relative amount of each separated component was observed by an imaging CCD camera. The data was then processed and reported as loss of the main peak (*i.e*., formation of acidic and basic species) using conventional chromatography integration software.

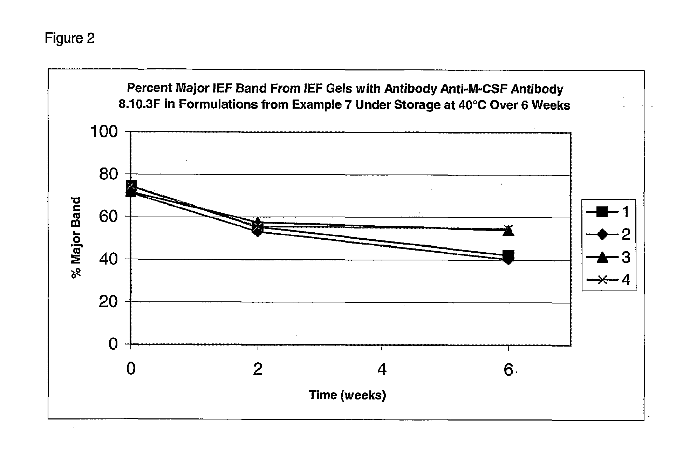

Figure 2 shows the percentage major IEF band estimated from IEF gels with formulations 1-4 stored at 40°C over 6 weeks. As seen in Figure 2, a lesser extent of decrease in the major IEF band at pH 5.5 and 6.0 suggested improved stability at pH ranging from 5.5 and 6.0 (*i.e*., formulation nos. 3 and 4).

### EXAMPLE 8

A study was conducted to evaluate the effect of EDTA on anti-M-CSF antibody 8.10.3F aggregation.

Specifically, sample formulation nos. 3, 5, 6, 7, and 8 were prepared with and without EDTA according to Table 3 and Example 5 and stored in several glass vials at 40°C for 0 (initial), 2, 4, and 6 weeks. The glass vials were then sampled aseptically to measure the level of antibody 8.11.3F aggregation in the formulations at the 0, 2, 4, and 6 week time points. In addition, formulation 11 (with EDTA) was also prepared according to Table 3 and stored in several glass vials at 40°C for 26 weeks.

### Aggregation Analysis:

After weeks 0, 2, 4, and 6, each formulation was analyzed for aggregation using size exclusion chromatography. The size exclusion chromatography (*i.e*., SE-HPLC) was carried out using a TSK gel G3000SWXL-G2000SWXL column, mobile phase 0.2 M sodium phosphate buffer at pH 7.0, a flow rate of 1 ml/min, and UV detection at 214 nm. Table 5 shows the percentage of eluted high molecular weight species (*i.e*., aggregates of anti-M-CSF antibody 8.11.3F) measured at the relevant times for each of the formulation treatments. Aggregation levels were calculated by integrating the areas under the chromatogram peaks for each formulation and reporting the integrated areas under the high molecular weight species peaks as a percentage of total peak area (see Table 5).

**Table 5: Percent Aggregation for Formulations 3, 5, 6, 7, and 8 after Storage at 40°C:**

| **Formulation No.** | **Initial** | **2 weeks 40°C** | **4 weeks 40°C** | **6 weeks 40°C** |
|---|---|---|---|---|
| 3 | 0.2% | 1.3% | 1.9% | 3.2% |
| 5 | 0.2% | 1.5% | 2.2% | 3.8% |
| 6 | 0.2% | 1.1% | 1.9% | 3% |
| 7 | 0.2% | 1.2% | - | 2.9% |
| 8 | 0.2% | 1.1% | 1.4% | 2.6% |

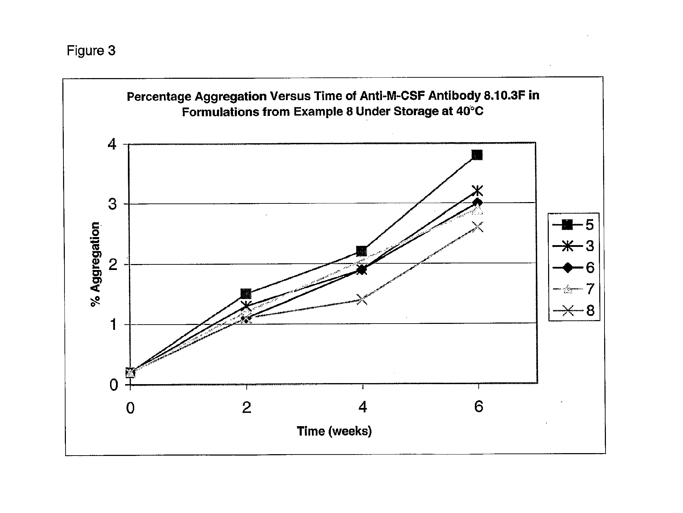

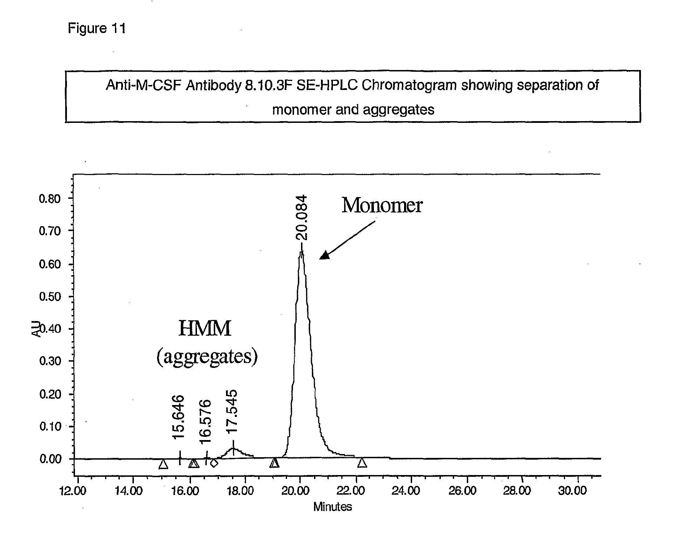

As can be seen in Table 5 and Figure 3, the EDTA containing formulation (*i.e*., formulation 8) showed lower levels of aggregation over time as compared to formulations without EDTA. Figure 11 shows a size exclusion chromatogram for monoclonal anti-M-CSF antibody 8.10.3F stored in formulation 11 for 26 weeks at 40°C.

### EXAMPLE 9

A study was conducted to evaluate the effect of EDTA on anti-M-CSF antibody 8.10.3F aggregation and fragmentation.

Specifically, sample formulation nos. 9, 10, 11, 12, and 13 were prepared with and without EDTA according to Table 3 and Example 5 and stored in several glass vials at 40°C for 0 (initial), 4, 6, 8, 12 and 26 weeks. The glass vials were then sampled aseptically to measure the level of antibody 8.11.3F aggregation in the formulations at the predetermined time points.

### Aggregation Analysis:

At weeks 0, 4, 6, 8, 12, and 26, each formulation was analyzed for aggregation using size exclusion chromatography. Size exclusion - high pressure liquid chromatography (SE-HPLC) was carried out using a TSK gel G3000SWXL-G2000SWXL column, mobile phase 0.2 M sodium phosphate buffer at pH 7.0, a flow rate of 1 ml/min, and UV detection at 214 nm. Table 6 shows the percentage of eluted high molecular weight species (*i.e*., aggregates of anti-M-CSF antibody 8.11.3F) measured at the relevant times for each of the formulation treatments. Aggregation levels were calculated by integrating the areas under the chromatogram peaks for each formulation and reporting the integrated areas under the high molecular weight species peaks as a percentage of total peak area (see Table 6).

**Table 6: Percent Aggregation for Formulations 9, 10, 11, 12, and 13 after Storage at 40°C:**

| **Formulation No.** | **Initial** | **4 weeks 40°C** | **6 weeks 40°C** | **8 weeks 40°C** | **12 weeks 40°C** | **26 weeks 40°C** |
|---|---|---|---|---|---|---|
| 9 | 0.2% | 2.0% | 2.6% | 3.9% | 5.8% | 11.6% |
| 10 | 0.1% | 0.9% | 1.4% | 1.6% | 2.7% | 4.7% |
| 11 | 0.2% | 1.0% | 1.4% | 1.8% | 2.6% | 4.9% |
| 12 | 0.2% | 1.8% | 2.3% | 2.9% | 3.9% | 7.0% |
| 13 | 0.2% | 1.4% | 1.9% | 2.4% | 3.5% | 6.2% |

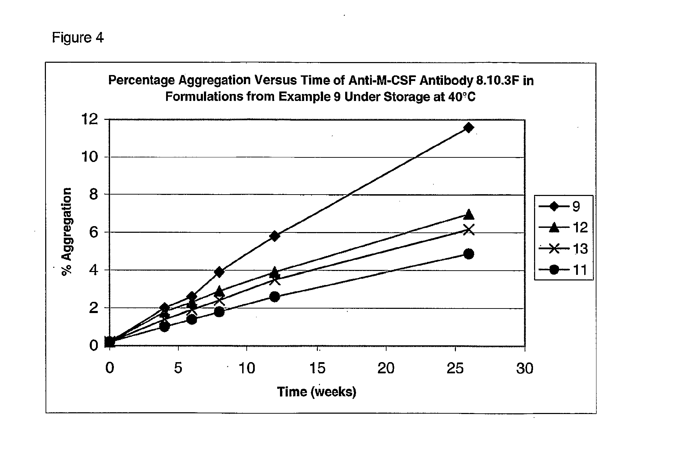

As can be seen in Table 6 and Figure 4, the EDTA containing formulations (*i.e*., formulations 10, 11, 12 and 13) showed lower levels of aggregation over time as compared to the formulation without EDTA (*i.e.*, formulation 9).

### Fragmentation Analysis:

At weeks 0, 4, 6, 8, 12, and 26, formulation nos. 9, 10, 11, 12, and 13 were also analyzed for fragmentation.

Organic size exclusion - high pressure liquid chromatography (SE-HPLC) was conducted on the samples at time points 0, 4, 6, 8, 12 and 26 weeks to determine the percent fragmentation for an approximately 11 kD fragment of the antibody. The samples were injected onto a TSK gel Super SW3000 size exclusion column, using an isocratic mobile phase of 40% acetonitrile + 0.1% TFA at a flow rate of 0.50 mL/min and UV detection at 214 nm. The percentage of eluted species was determined by integrating area under peaks.

**Table 7: Percent Fragmentation of a between 10.5 kD and a 11.kD Fragment for Formulations 9, 10, 11, 12, and 13 after Storage at 40°C:**

| **Formulation No.** | **Initial** | **8 weeks 40°C** | **12 weeks 40°C** | **26 weeks 40°C** |
|---|---|---|---|---|
| 9 | 0.1 % | 1.6% | 2.3% | 3.5% |
| 10 | 0.1% | 0.7% | 0.9% | 1.7% |
| 11 | 0.1% | 0.6% | 0.8% | 1.7% |
| 12 | 0.1% | 1.5% | 2.1% | 4.1% |
| 13 | 0.1% | 1.0% | 1.4% | 3.0% |

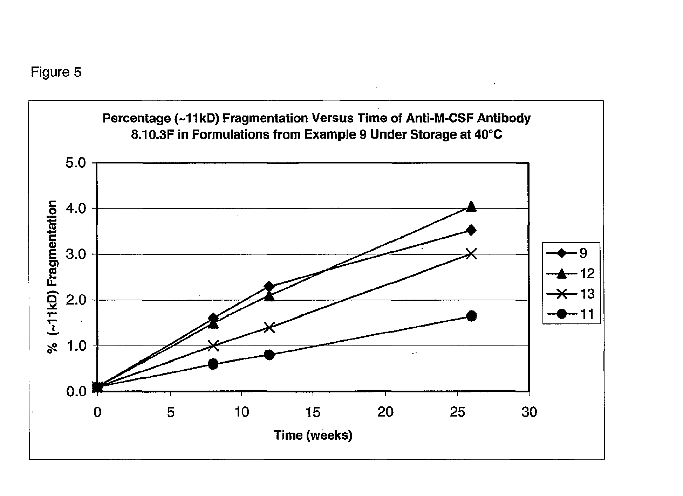

As can be seen in Table 7 and Figure 5, the EDTA containing formulations (*i.e.,* formulations 10, 11, 12 and 13) showed lower levels of fragmentation yielding an 11 kD fragment over time as compared to the formulation without EDTA (*i.e*., formulation 9). In addition, the histidine containing formulation (*i.e*., formulation 11) showed lower levels of fragmentation yielding an 11 kD fragment over time as compared to the formulations without histidine (*i.e*., formulations 9, 12 and 13).

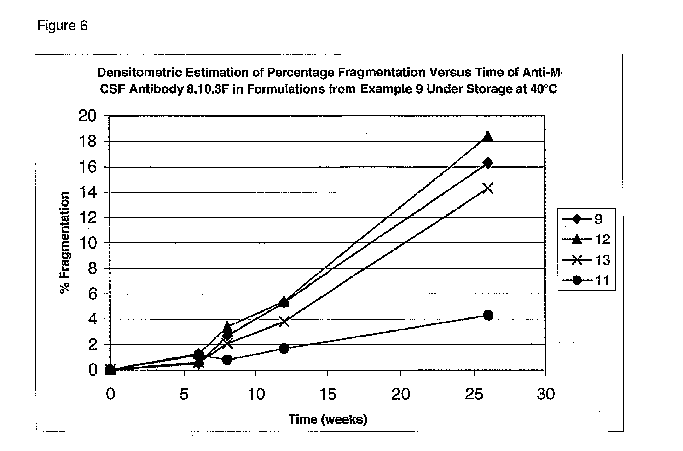

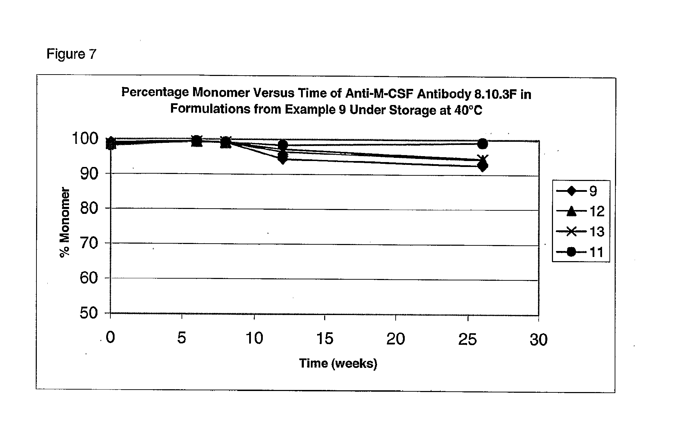

In addition, SDS-PAGE gels were also run with the samples at time points 0, 4, 6, 8, 12 and 26 weeks using NuPAGE 4-12% Bis-Tris gel, and colloidal blue (Coomassie) stain. For the reduced gels, reduction was achieved by NuPAGE® reducing agent. Percentage fragmentation in reduced gels was estimated densitometrically by 100 % minus (% heavy chain + % light chain). The gel data showed fragment bands with approximate molecular masses of 40 kD and 11 kD. Figure 6 shows the percentage fragmentation estimated from SDS-PAGE reduced gel data with the formulation samples. Figure 7 shows the percentage monomer of antibody estimated from SDS-PAGE non-reduced gel data with the formulation samples.

Figures 4-7 show improved anti-M-CSF antibody stability for formulation 11 (10 mM histidine, 45 mg/ml mannitol, 0.02 mg/ml disodium EDTA dihydrate, and 0.2 mg/ml polysorbate 80) for reduced aggregation (Fig. 4), reduced quantity of 11 kD fragmentation (Fig. 5), reduced fragmented species (Fig. 6), and retaining highest % intact antibody monomer (Fig. 7) as compared to formulations 9, 10, 12 and 13.

### EXAMPLE 10

A study was conducted to evaluate the effect of EDTA and histidine on anti-M-CSF antibody 8.10.3F fragmentation.

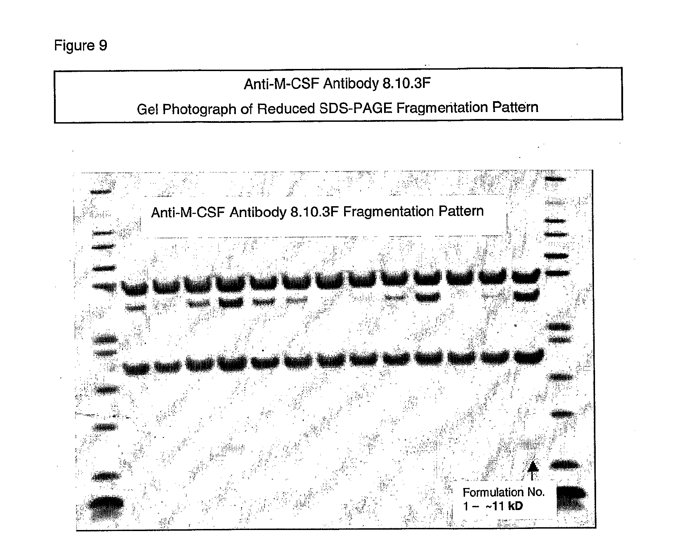

Several experimental formulations of antibody 8.10.3F generated truncated species (*i.e*., fragments) upon stressed conditions of 40°C for 6 weeks, as observed by the formation of bands at approximately 40kD and 11 kD appearing on reduced SDS-PAGE gel photograph as shown in Figure 9. The identity of the most abundant clip site was determined to be between residues Asp99 and Pro100 on the heavy chain of the molecule. Also observed were some minor truncation sites in the light chain, one between residues GIy213 and GIu214 and another between GIu214 and Cys215. The truncation level varies depending on the formulation and thus far has only been observed at higher temperatures (*e.g*., 40°C) *i.e*., under stressed conditions.

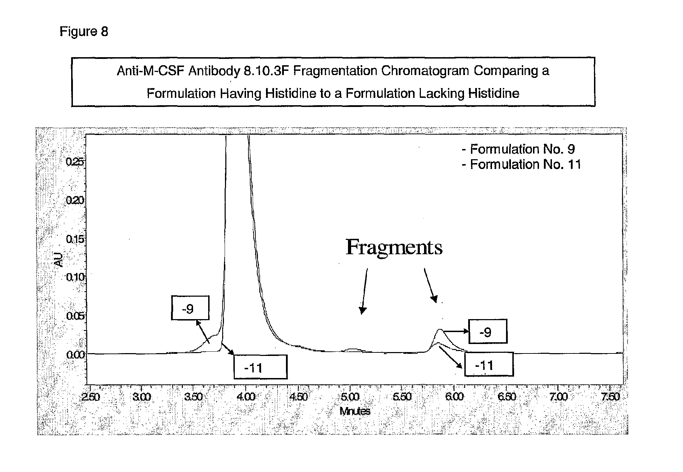

A sample of antibody 8.10.3F, which was formulated in sodium acetate and sodium chloride (formulation no. 9) and stored at 40°C for 26 weeks, was observed to have a higher presence of an 11 kD fragment than a formulation comprising histidine and EDTA (formulation no. 11) using organic SE-HPLC (see Figure 8).

This sample was then analyzed by organic size exclusion chromatography/mass spectrometry (SEC/MS) in order to determine the site of truncation. The sample was injected onto a size exclusion column (Phenomenex SEC3000, 4.6 x 250 mm) using an isocratic mobile phase of 40% acetonitrile + 0.1% TFA at a flow rate of 0.50 mL/min. The eluent of the column was split such that approximately half of the flow was directed into the source of an electrospray mass spectrometer (Micromass Q-Tof Micro™, Waters Inc.). Mass spectra of each of the chromatographic peaks were deconvoluted using the MaxEnt algorithm included in the operating software. The measured molecular masses were then compared to the theoretical molecular mass based on the predicted amino acid sequence of antibody 8.10.3F.

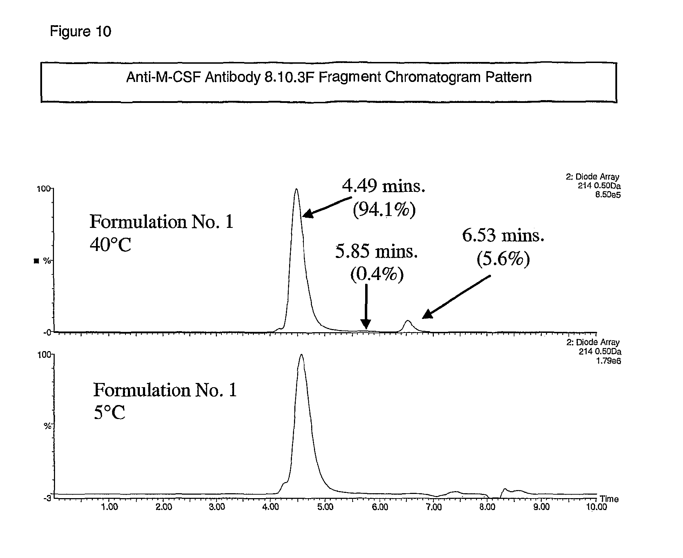

Organic SE-HPLC separation with 214 nm detection followed by mass spectrometric identification were performed for antibody 8.10.3F in formulation no. 1 stored at 40°C for 6 weeks compared to a control sample. Figure 10 shows the resulting chromatogram having the 40°C storage as the top graph and the 5°C control as the bottom graph. The chromatogram measured masses were tabulated and compared to the theoretical masses of the postulated species in Table 8.

**Table 8: The measured masses of the species observed in the sample compared with the theoretical masses of the postulated species. All of the measured molecular weights were within experimental error of the theoretical molecular weights of the postulated species.**

| **Peak RT (retention time) (minutes)** | **Measured MW (Da)** | **Identity** | **Theoretical MW (Da)** |
|---|---|---|---|
| 4.45 | 147,220 | 8.10.3F ("Parent") | 147,223* |
| 4.45 | 136,424 | Parent minus (Heavy Chain 1-99) | 136,431* |
| 4.45 | 125,634 | Parent minus clipped 2x (Heavy Chain 1-99) | 125,633* |
| 6.53 | 10,816 | Heavy chain 1-99 | 10,816 |

| | | | |
|---|---|---|---|
| * Consistent with "G0,G0" glycoform and des-Lys C-terminus on heavy chains. The "G0,G1"; "G1,G1"; and "G1,G2" glycoforms were also observed. The "G0, G0" glycoform is a species in which both heavy chains have the G0 glycan attached, as described in Jefferis *et al., Biochem.* J., 268, 529-537, (1990). The G1 and G2 glycans have one and two, respectively, galactose residues on the nonreducing end of the glycan. The antibodies were N-glycosylated at residue 297 of the heavy chain. | | | |

Under stressed conditions, antibody 8.10.3F can undergo cleavage and generate truncated species. The main cleavage site is consistent with cleavage of an an Asp-Pro bond in the heavy chain of 8.10.3F, which would generate a 10,816 Da (*i.e*., about 11 kD) species along with the corresponding parent species minus one and two of the truncation product.

### EXAMPLE 11

A study was conducted to evaluate the effect of various buffers on anti-M-CSF antibody 8.10.3F aggregation.

Specifically, sample formulation nos. 6, 3, 5 and 8 were prepared with according to Table 9 and Example 5 and stored in glass vials at 40°C for 6 weeks. The glass vials were then sampled aseptically to measure the level of antibody 8.11.3F aggregation in the formulations at the 6 week time point.

### Aggregation Analysis:

At the 6 week time point, each formulation was analyzed for aggregation using size exclusion chromatography. The size exclusion chromatography (*i.e*., SE-HPLC) was carried out using a TSK gel G3000SWXL-G2000SWXL column, mobile phase 0.2 M sodium phosphate buffer at pH 7.0, a flow rate of 1 ml/min, and UV detection at 214 nm. Table 9 shows the percentage of eluted high molecular weight species (*i.e*., aggregates of anti-M-CSF antibody 8.11.3F) measured at the relevant time for each of the formulation treatments. Aggregation levels were calculated by integrating the areas under the chromatogram peaks for each formulation and reporting the integrated areas under the high molecular weight species peaks that eluted prior to the antibody monomer (*i.e*., the intact unaggregated polypeptide) as a percentage of total peak area (see Table 9).

**Table 9: Percent Aggregation for Formulations 6, 3, 5 and 8 after Storage at 40°C:**

| **Formulation No.** | **8.10.3F Antibody concentration, (mg/ml)** | **Formulation Description** | **% Aggregation** |
|---|---|---|---|
| 6 | 8 ±2 | 20 mM acetate pH 5.5 | 3.0% |
| 3 | 8 ±2 | Combination, pH 5.5 (acetate 5 mM, citrate 5 mM, histidine 5 mM, succinate 5 mM) | 3.2% |
| 5 | 8 ±2 | 20 mM citrate, pH 5.5 | 3.8% |
| 8 | 8 ±2 | 20 mM EDTA, pH 5.5 | 2.6% |

As can be seen in Table 9, the EDTA containing formulation (*i.e*., formulation 8) showed reduced levels of aggregation as compared to the formulations without EDTA (*i.e*., formulations 6, 3 and 5).

### EXAMPLE 12

A study was conducted to evaluate the effect of various excipients on anti-M-CSF antibody 8.10.3F aggregation and fragmentation.

Specifically, sample formulation nos. 18, 19, 20, 29, 30 and 31 were prepared according to Table 10 and Example 5 and stored in glass vials at 40°C for 6 weeks. The glass vials were then sampled aseptically to measure the level of antibody 8.11.3F aggregation and fragmentation in the formulations at the 6 week time point.

### Aggregation Analysis:

At the 6 week time point, each formulation was analyzed for aggregation using size exclusion chromatography. The size exclusion chromatography (*i.e*., SE-HPLC) was carried out using a TSK gel G3000SWXL-G2000SWXL column, mobile phase 0.2 M sodium phosphate buffer at pH 7.0, a flow rate of 1 ml/min, and UV detection at 214 nm. Table 10 shows the percentage of eluted high molecular weight species (*i.e*., aggregates of anti-M-CSF antibody 8.11.3F) measured at the relevant time for each of the formulation treatments. Aggregation levels were calculated by integrating the areas under the chromatogram peaks for each formulation and reporting the integrated areas under the high molecular weight species peaks that eluted prior to the antibody monomer (*i.e*., the intact unaggregated polypeptide) as a percentage of total peak area (see Table 10).

### Fragmentation Analysis:

At the 6 week time point, each formulation was also analyzed for fragmentation using organic SE-HPLC. Organic SE-HPLC was conducted on the samples to determine the percent fragmentation for an 11 kD fragment of the total polypeptide. The samples were injected onto a TSK gel Super SW3000 size exclusion column, using an isocratic mobile phase of 40% acetonitrile + 0.1% TFA at a flow rate of 0.50 mL/min and UV detection at 214 nm. The percentage of eluted species was determined by integrating area under peaks and reported in Table 10.

**Table 10: Description of Anti-M-CSF 8.10.3F Antibody Formulations and Results after Storage at 40°C:**

| **Formulation No.** | **8.10.3F mg/ml** | **Buffer** | **Excipient** | **PS80** | **Na₂EDTA 2H₂O** | **% Aggregate** | **% Fragment** |
|---|---|---|---|---|---|---|---|
| 29 | 6±2 | Sodium acetate, 20 mM, pH 5.5 | Sodium chloride 140m M | 0.2 | - | 2.0% | 1.2% |
| 18 | 6±2 | Sodium acetate, 20 mM, pH 5.5 | Mannitol 45 mg/ml | 0.2 | - | 1.4% | 1.0% |
| 30 | 6±2 | Sodium acetate, 20 mM, pH 5.5 | Mannitol 45 mg/ml | 0.2 | 0.02 mg/ml | 1.3% | 0.6% |
| 19 | 6±2 | Sodium acetate, 20 mM, pH 5.5 | Sucrose 90 mg/mL | 0.2 | 0.02 mg/ml | 1.2% | 0.5% |
| 20 | 6±2 | Sodium acetate, 20 mM, pH 5.5 | Trehalose 90 mg/mL | 0.2 | 0.02 mg/ml | 1.1% | 0.4% |
| 31 | 6±2 | Histidine, 10mM, pH 6.0 | Mannitol 45 mg/ml | 0.2 | 0.02 mg/ml | 0.8% | 0.5% |

As can be seen in Table 10, the EDTA containing formulations (*i.e*., formulations 30, 31, 19, and 20) showed reduced levels of aggregation and fragmentation as compared to the formulations without EDTA (*i.e*., formulations 29 and 18).

### EXAMPLE13

A study was conducted to evaluate the effect of various excipients on anti-M-CSF antibody 8.10.3F fragmentation.

Specifically, sample formulation nos. 21-28 were prepared according to Table 11 and Example 5 and stored in glass vials at 40°C for 26 weeks. The glass vials were then sampled aseptically to measure the level of antibody 8.11.3F fragmentation in the formulations at the 26 week time point.

### Fragmentation Analysis:

At the 26 week time point, each formulation was also analyzed for fragmentation using organic SE-HPLC. Organic SE-HPLC was conducted on the samples to determine the percent fragmentation for an 11 kD fragment of the total polypeptide. The samples were injected onto a TSK gel Super SW3000 size exclusion column, using an isocratic mobile phase of 40% acetonitrile + 0.1% TFA at a flow rate of 0.50 mL/min and UV detection at 214 nm. The percentage of eluted species was determined by integrating area under peaks and reported in Table 11.

**Table 11: Description of Anti-M-CSF 8.10.3F Antibody Formulations and Results after Storage at 40°C:**

| **Formulation No.** | **8.10.3F mg/ml** | **Buffer** | **Excipients** | **PS80 mg/mL** | **Na₂EDTA** .**2H₂O mg/mL** | **% Fragmentation** |
|---|---|---|---|---|---|---|
| 21 | 50±8 | Sodium acetate, 20 mM, pH 5.5 | Sodium chloride 140 mM | 0.2 | - | 4.1% |
| 22 | 50±8 | Sodium acetate, 10 mM, pH 5.5 | Mannitol 45 mg/ml | 0.2 | 0.02 mg/ml | 2.3% |
| 23 | 50±8 | Sodium acetate, 10 mM, pH 5.5 | Sucrose 90 mg/ml | 0.2 | 0.02 mg/ml | 3.0% |
| 24 | 50±8 | Sodium acetate, 10 mM, pH 5.5 | Trehalose 90 mg/ml | 0.2 | 0.02 mg/ml | 2.2% |
| 25 | 50±8 | Histidine, 10 mM, pH 6.0 | Mannitol 45 mg/ml | 0.2 | 0.02 mg/ml | 2.1% |
| 26 | 50±8 | Histidine, 10 mM, pH 6.0 | Sucrose 90 mg/ml | 0.2 | 0.02 mg/ml | 1.9% |
| 27 | 50±8 | Histidine, 10 mM, pH 6.0 | Trehalose 90 mg/ml | 0.2 | 0.02 mg/ml | 1.8% |
| 28 | 50±8 | Histidine, 5 mM, pH 6.0 | Mannitol 45 mg/ml | 0.2 | 0.02 mg/ml | 1.4% |

As can be seen in Table 11, the EDTA containing formulations (*i.e*., formulations 22-28) showed reduced levels of fragmentation as compared to the formulation without EDTA (*i.e*., formulation 21). Likewise, as can be seen in Table 11, the histidine containing formulations (*i.e.,* formulations 25-28) showed reduced levels of fragmentation as compared to the formulations without histidine (*i.e*., formulations 21-24).

All references cited in this specification, including without limitation all papers, publications, patents, patent applications, presentations, texts, reports, manuscripts, brochures, books, internet postings, journal articles, periodicals, and the like, are hereby incorporated by reference into this specification in their entireties. The discussion of the references herein is intended merely to summarize the assertions made by their authors and no admission is made that any reference constitutes prior art. Applicants reserve the right to challenge the accuracy and pertinency of the cited references.

As various changes could be made in the above methods and compositions without departing from the scope of the invention, it is intended that all matter contained in the above description shall be interpreted as illustrative and not in a limiting sense. In addition, it should be understood that aspects of the various embodiments may be interchanged both in whole or in part.

SEQUENCES

SEQ ID NO: 1

atggagttggggctgtgctgggttttccttgttgctattttagaaggtgtccagtgtgaggtgcagctggtggagtctgggggaggcttggtac
agcctggggggtccctgagactctcctgtgcagcctctggattcaccttcagtagttttagtatgacctgggtccgccaggctccaggaaa
ggggctggagtgggtttcatacattagtagtagaagtagtaccatatcctacgcagactctgtgaagggccgattcaccatctccagaga
caatgccaagaactcactgtatctgcaaatgaacagcctgagagacgaggacacggctgtgtattactgtgcgagagatcctcttctag
cgggagctaccttctttgactactggggccagggaaccctggtcaccgtctcctcagcctccaccaagggcccatcggtcttccccctgg
cgccctgctccaggagcacctccgagagcacagcggccctgggctgcctggtcaaggactacttccccgaaccggtgacggtgtcgt
ggaactcaggcgctctgaccagcggcgtgcacaccttcccagctgtcctacagtcctcaggactctactccctcagcagcgtggtgacc
gtgccctccagcaacttcggcacccagacctacacctgcaacgtagatcacaagcccagcaacaccaaggtggacaagacagttg
agcgcaaatgttgtgtcgagtgcccaccgtgcccagcaccacctgtggcaggaccgtcagtcttcctcttccccccaaaacccaagga
caccctcatgatctcccggacccctgaggtcacgtgcgtggtggtggacgtgagccacgaagaccccgaggtccagttcaactggtac
gtggacggcgtggaggtgcataatgccaagacaaagccacgggaggagcagttcaacagcacgttccgtgtggtcagcgtcctcac
cgttgtgcaccaggactggctgaacggcaaggagtacaagtgcaaggtctccaacaaaggcctcccagcccccatcgagaaaacc
atctccaaaaccaaagggcagccccgagaaccacaggtgtacaccctgcccccatcccgggaggagatgaccaagaaccaggtc
agcctgacctgcctggtcaaaggcttctaccccagcgacatcgccgtggagtgggagagcaatgggcagccggagaacaactaca
agaccacacctcccatgctggactccgacggctccttcttcctctacagcaagctcaccgtggacaagagcaggtggcagcagggga
acgtcttctcatgctccgtgatgcatgaggctctgcacaaccactacacgcagaagagcctctccctgtctccgggtaaa

SEQ ID NO: 2

MELGLCWVFLVAILEGVQCEVQLVESGGGLVQPGGSLRLSCAASGFTFSSFSMTWVRQAPGK
GLEWVSYISSRSSTISYADSVKGRFTISRDNAKNSLYLQMNSLRDEDTAVYYCARDPLLAGATFF
DYWGQGTLVTVSSASTKGPSVFPLAPCSRSTSESTAALGCLVKDYFPEPVTVSWNSGALTSGV
HTFPAVLQSSGLYSLSSVVTVPSSNFGTQTYTCNVDHKPSNTKVDKTVERKCCVECPPCPAPP
VAGPSVFLFPPKPKDTLMISRTPEVTCVVVDVSHEDPEVQFNWYVDGVEVHNAKTKPREEQFN
STFRVVSVLTVVHQDWLNGKEYKCKVSNKGLPAPIEKTISKTKGQPREPQVYTLPPSREEMTKN
QVSLTCLVKGFYPSDIAVEWESNGQPENNYKTTPPMLDSDGSFFLYSKLTVDKSRWQQGNVFS
CSVMHEALHNHYTQKSLSLSPGK

SEQ ID NO: 3

atggaaaccccagcgcagcttctcttcctcctgctactctggctcccagataccaccggagaatttgtgttgacgcagtctccaggcaccc
tgtctttgtctccaggggaaagagccaccctctcctgcagggccagtcagagtgttagcagcagttacttagcctggtaccagcagaaa
cctggccaggctcccaggctcctcatctatggtgcatccagcagggccactggcatcccagacaggttcagtggcagtgggtctggga
cagacttcactctcaccatcagcagactggagcctgaagattttgcagtgtattactgtcagcagtatggtagctcacctctcactttcggc
ggagggaccaaggtggagatcaaacgaactgtggctgcaccatctgtcttcatcttcccgccatctgatgagcagttgaaatctggaact
gcctctgttgtgtgcctgctgaataacttctatcccagagaggccaaagtacagtggaaggtggataacgccctccaatcgggtaactcc
caggagagtgtcacagagcaggacagcaaggacagcacctacagcctcagcagcaccctgacgctgagcaaagcagactacga
gaaacacaaagtctacgcctgcgaagtcacccatcagggcctgagctcgcccgtcacaaagagcttcaacaggggagagtgt

SEQ ID NO: 4

METPAQLLFLLLLWLPDTTGEFVLTQSPGTLSLSPGERATLSCRASQSVSSSYLAWYQQKPGQA
PRLLIYGASSRATGIPDRFSGSGSGTDFTLTISRLEPEDFAVYYCQQYGSSPLTFGGGTKVEIKR
TVAAPSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC

Particular embodiments of the invention are set forth in the following numbered paragraphs:
1. A composition comprising
   at least one chelating agent; and
   at least one antibody comprising:
   an amino acid sequence that is at least 90% identical to a heavy chain amino acid sequence shown in SEQ ID NO: 2, and
   an amino acid sequence that is at least 90% identical to a light chain amino acid
   sequence shown in SEQ ID NO: 4,
   wherein the antibody binds to human M-CSF.
2. The composition according to paragraph 1, wherein the composition is a liquid composition, the antibody is a human IgG2 antibody, and the antibody does not comprise a signal sequence.
3. The composition according to paragraph 1, wherein the antibody comprises a heavy chain amino acid sequence with at least 99% sequence identity to SEQ ID NO: 2 and a light chain amino acid sequence with at least 99% sequence identity to SEQ ID NO: 4.
4. The composition according to paragraph 1, wherein the antibody comprises a heavy chain amino acid sequence comprising the variable region of SEQ ID NO: 2 and a light chain amino acid sequence comprising the variable region of SEQ ID NO: 4.
5. The composition according to paragraph 1, wherein the antibody comprises an isolated human monoclonal IgG2 anti-M-CSF antibody having the heavy and light chain amino acid sequences of antibody 8.10.3F.
6. The composition according to paragraph 1, wherein the composition comprises at least one chelating agent that is EDTA.
7. The composition according to paragraph 1, wherein the composition further comprises a buffer.
8. The composition according to paragraph 1, wherein the composition comprises at least one chelating agent that is EDTA, and further comprises at least one buffer that is histidine.
9. The composition according to paragraph 1, wherein the composition further comprises a buffer and a surfactant.
10. The composition according to paragraph 1, wherein the composition further comprises a buffer, a surfactant, and a tonicity agent.
11. The composition according to paragraph 1, wherein the composition comprises at least one chelating agent that is EDTA, and further comprises a buffer, a surfactant, and a tonicity agent.
12. The composition according to paragraph 1, wherein the composition comprises at least one chelating agent that is EDTA, and further comprises histidine, a surfactant, and a tonicity agent.
13. The composition according to paragraph 1, wherein the composition comprises at least one chelating agent that is EDTA, and further comprises histidine, polysorbate 80, and a tonicity agent.
14. The composition according to paragraph 1, wherein the composition comprises:
   from about 1 mg/ml to about 200 mg/ml of antibody;
   from about 0.01 millimolar to about 5.0 millimolar of a chelating agent;
   and from about 1 mM to about 100 mM of histidine.
15. The composition according to paragraph 1, wherein the composition comprises:
   from about 1 mg/ml to about 200 mg/ml of antibody;
   from about 0.01 millimolar to about 5.0 millimolar of EDTA;
   and from about 1 mM to about 100 mM of histidine.
16. The composition according to paragraph 1, wherein the composition comprises:
   from about 1 mg/ml to about 200 mg/ml of antibody;
   from about 0.01 millimolar to about 1.0 millimolar of a chelating agent;
   from about 1 mM to about 100 m M of a buffer;
   from about 0.01 mg/ml to about 10 mg/ml of a surfactant;
   and from about 100 millimolar to about 400 millimolar of a tonicity agent.
17. The composition according to paragraph 1, wherein the composition comprises:
   from about 1 mg/ml to about 200 mg/ml of antibody;
   from about 0.01 millimolar to about 1.0 millimolar of EDTA;
   from about 1 mM to about 100 m M of a buffer;
   from about 0.01 mg/ml to about 10 mg/ml of a surfactant;
   and from about 100 millimolar to about 300 millimolar of a tonicity agent.
18. The composition according to paragraph 2, wherein the composition comprises:
   from about 1 mg/ml to about 100 mg/ml of antibody;
   from about 0.01 millimolar to about 1.0 millimolar of a chelating agent;
   from about 1 mM to about 100 mM of histidine;
   from about 0.01 mg/ml to about 2 mg/ml of a surfactant;
   and from about 1 mg/ml to about to about 200 mg/ml of mannitol.
19. The composition according to paragraph 2, wherein the composition comprises:
   about 10 mg/ml of antibody;
   about 0.02 mg/ml of a chelating agent;
   about 10 mM histidine;
   about 0.2 mg/ml of polysorbate 80;
   and about 45 mg/ml of mannitol.
20. The composition according to paragraph 1, wherein the composition comprises:
   from about 50 mg/ml to about 100 mg/ml of antibody;
   from about 0.01 mg/ml to about 1 mg/ml of EDTA;
   from about 5 mM to about 50 mM histidine;
   from about 0.1 mg/ml to about 2 mg/ml of polysorbate 80;
   and from about 1 mg/ml to about 150 mg/ml of a tonicity agent.
21. A composition comprising at least one monoclonal anti-M-CSF antibody and a chelating agent, wherein the composition comprises an amount of the chelating agent sufficient to stabilize the composition when maintained at a temperature of about 400C for a period of at least about 26 weeks.
22. A composition comprising at least one anti-M-CSF antibody and a chelating agent, wherein the molar concentration of the antibody ranges from about 0.01 millimolar to about 2 millimolar and the molar concentration of the chelating agent ranges from about 0.001 millimolar to about 5 millimolar, and wherein the molar ratio of antibody to chelating agent ranges from about 0.002 to about 2000.
23. A process for preparing a composition comprising mixing at least one antibody comprising an amino acid sequence that is at least 90% identical to a light chain amino acid sequence shown in SEQ ID NO: 4, and an amino acid sequence that is at least 90% identical to a heavy chain amino acid sequence shown in SEQ ID NO: 2; with at least one chelating agent.
24. A method for the treatment of an M-CSF-mediated disorder in a subject, comprising administering to the subject a therapeutically effective amount of a liquid pharmaceutical composition comprising at least one pharmaceutically acceptable chelating agent; and at least one antibody comprising an amino acid sequence that is at least 90% identical to a light chain amino acid sequence shown in SEQ ID NO: 4, and further comprising an amino acid sequence that is at least 90% identical to a heavy chain amino acid sequence shown in SEQ ID NO: 2, wherein the antibody binds to human M-CSF.
25. The method according to paragraph 24, wherein the M-CSF-mediated disorder is selected from the group consisting of neoplasia disorders and inflammatory disorders.

## Claims

1. A composition comprising
at least one chelating agent; and
at least one antibody comprising:
an amino acid sequence that is at least 90% identical to a heavy chain amino acid sequence shown in SEQ ID NO: 2, and
an amino acid sequence that is at least 90% identical to a light chain amino acid sequence shown in SEQ ID NO: 4,
wherein the antibody binds to human M-CSF and
wherein the composition is a powder or a liquid composition.

2. The composition according to claim 1, wherein the antibody is a human IgG2 antibody, and the antibody does not comprise a signal sequence.

3. The composition according to claim 1, wherein the antibody comprises a heavy chain amino acid sequence with at least 99% sequence identity to SEQ ID NO: 2 and a light chain amino acid sequence with at least 99% sequence identity to SEQ ID NO: 4.

4. The composition according to claim 1, wherein the antibody comprises a heavy chain amino acid sequence comprising the variable region of SEQ ID NO: 2 and a light chain amino acid sequence comprising the variable region of SEQ ID NO: 4.

5. The composition according to claim 1, wherein the antibody comprises an isolated human monoclonal IgG2 anti-M-CSF antibody having the heavy and light chain amino acid sequences of antibody 8.10.3F.

6. The composition according to claim 1, wherein the chelating agent is EDTA.

7. The composition according to claim 1, wherein the composition further comprises a buffer, preferably wherein the buffer is histidine.

8. The composition according to claim 6, wherein the composition further comprises a buffer, a surfactant, and a tonicity agent, preferably wherein the buffer is histidine.

9. The composition according to claim 8, wherein the surfactant is polysorbate 80 and/or the tonicity agent is selected from mannitol, trehalose, and sucrose.

10. The composition according to claim 1, wherein the composition comprises:
from about 0.1 mg/ml to about 200 mg/ml of antibody;
from about 0.001 mM to about 5.0 mM of chelating agent;
from about 1 mM to about 100 mM of histidine;
from about 0.01 mg/ml to about 10 mg/ml polysorbate 80; and
from about 10 mM to about 400 mM of trehalose.

11. The composition according to claim 1, wherein the composition comprises:
from about 1.0 mg/ml to about 100 mg/ml of antibody;
from about 0.01 mM to about 1 mM EDTA;
from about 10 mM to about 50 mM of histidine;
from about 0.05 mg/ml to about 1 mg/ml polysorbate 80; and
from about 100 mM to about 300 mM of trehalose.

12. The composition according to claim 1, wherein the composition comprises:
about 50 mg/ml of antibody;
about 0.02 mg/ml of EDTA;
about 10 mM histidine;
about 0.2 mg/ml of polysorbate 80; and
about 90 mg/ml of trehalose.

13. The composition according to claim 1, wherein the composition comprises:
from about 1 mg/ml to about 100 mg/ml of antibody;
from about 0.001 mg/ml to about 0.5 mg/ml of EDTA;
from about 1 mM to about 50 mM of histidine;
from about 0.01 mg/ml to about 5 mg/ml polysorbate 80; and
from about 10 mg/ml to about 200 mg/ml of sucrose.

14. The composition according to claim 1, wherein the composition comprises:
from about 50 mg/ml to about 100 mg/ml of antibody;
from about 0.01 mM to about 1 mM of EDTA;
from about 10 mM to about 30 mM of histidine;
from about 0.05 mM to about 1 mM polysorbate 80; and
from about 100 mM to about 400 mM of sucrose.

15. The composition according to claim 1, wherein the composition comprises:
about 50 mg/ml of antibody;
about 0.02 mg/ml of EDTA;
about 10 mM histidine;
about 0.2 mg/ml of polysorbate 80; and
about 90 mg/ml of sucrose.

16. A composition comprising at least one anti-M-CSF antibody and a chelating agent, wherein the molar concentration of the antibody ranges from about 0.01 millimolar to about 2 millimolar and the molar concentration of the chelating agent ranges from about 0.001 millimolar to about 5 millimolar, and wherein the molar ratio of antibody to chelating agent ranges from about 0.002 to about 2000.

17. The composition according to any one of claims 1 to 16 wherein the composition is a powder composition.

18. The composition according to any one of claims 1 to 17 for use in the treatment of an M-CSF-mediated disorder.

19. The composition according to claim 18, wherein the M-CSF-mediated disorder is selected from the group consisting of neoplasia disorders and inflammatory disorders.
